(19) Europäisches Patentamt
European Patent Office
Office européen des brevets

(11) EP 3 543 232 A1

(12) EUROPEAN PATENT APPLICATION

(43) Date of publication:
**25.09.2019 Bulletin 2019/39**

(21) Application number: **18163772.9**

(22) Date of filing: **23.03.2018**

(51) Int Cl.:
***C07D 403/12*** (2006.01)
***C07D 401/14*** (2006.01)
***C07D 401/12*** (2006.01)
***A61P 35/00*** (2006.01)
***A61K 31/506*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Step Pharma S.A.S.**
**75272 Paris, Cedex 06 (FR)**

(72) Inventors:
- **QUDDUS, Abdul**
  **Nottingham, Nottinghamshire, NG1 1GR (GB)**
- **NOVAK, Andrew**
  **Nottingham, Nottinghamshire, NG1 1GR (GB)**
- **COUSIN, David**
  **Nottingham, Nottinghamshire, NG1 1GR (GB)**
- **BLACKHAM, Emma**
  **Nottingham, Nottinghamshire, NG1 1GR (GB)**
- **WRIGGLESWORTH, Joseph**
  **Nottingham, Nottinghamshire, NG1 1GR (GB)**
- **JONES, Geraint**
  **Nottingham, Nottinghamshire, NG1 1GR (GB)**
- **BIRCH, Louise**
  **Nottingham, Nottinghamshire, NG1 1GR (GB)**
- **DUFFY, Lorna**
  **Nottingham, Nottinghamshire, NG1 1GR (GB)**
- **GEORGE, Pascal**
  **75272 Paris, Cedex 06 (FR)**
- **AHMED, Saleh**
  **Nottingham, Nottinghamshire, NG1 1GR (GB)**

(74) Representative: **Kinch, Alison**
**Sagittarius IP**
**Marlow International**
**Parkway**
**Marlow SL7 1YL (GB)**

# (54) AMINOPYRIMIDINE DERIVATIVES AS CTPS1 INHIBITORS

(57) Compounds of formula (I):

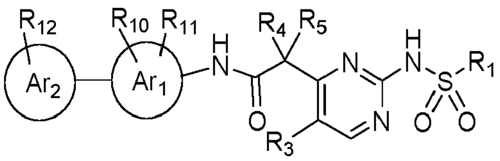

(I)

, compositions comprising such compounds and the use of such compounds as CTPS1 inhibitors, particularly in the treatment or prophylaxis of disorders associated with cell proliferation.

EP 3 543 232 A1

## Description

### Field of the invention

The invention relates to novel aminopyrimidine compounds, processes for the manufacture of such compounds, related intermediates, compositions comprising such compounds and the use of such compounds as cytidine triphosphate synthase 1 inhibitors, particularly in the treatment or prophylaxis of disorders associated with cell proliferation.

### Background of the invention

Nucleotides are a key building block for cellular metabolic processes such as deoxyribonucleic acid (DNA) and ribonucleic acid (RNA) synthesis. There are two classes of nucleotides, purines and pyrimidines and both classes are important for metabolic processes. Based on this, many therapies have been developed to target different aspects of nucleotide synthesis, with some inhibiting generation of purine nucleotides and some pyrimidine nucleotides.

The pyrimidine nucleotide cytidine 5' triphosphate (CTP) is a precursor required not just for the metabolism of DNA and RNA but also phospholipids and sialyation of proteins. CTP originates from two sources: a salvage pathway and a *de novo* synthesis pathway that depends on two enzymes, the CTP synthases (or synthetases) 1 and 2 (CTPS1 and CTPS2) (Evans and Guy 2004; Higgins, *et al.* 2007; Ostrander, *et al.* 1998).

CTPS1 and CTPS2 catalyse the conversion of uridine triphosphate (UTP) and glutamine into cytidine triphosphate (CTP):

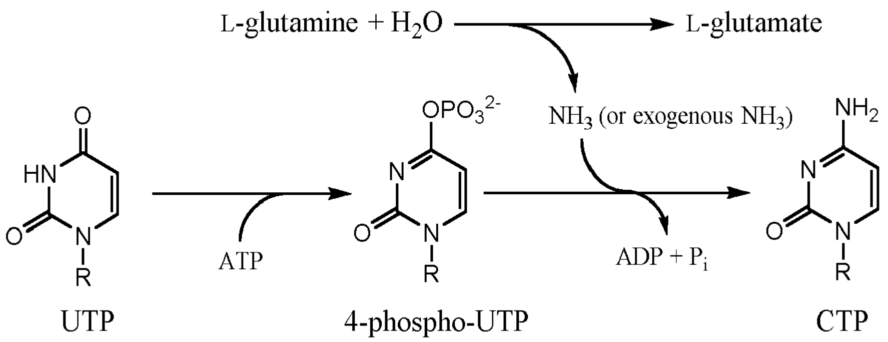


Both enzymes have two domains, an N-terminal synthetase domain and a C-terminal glutaminase domain (Kursula, *et al.* 2006). The synthetase domain transfers a phosphate from adenosine triphosphate (ATP) to the 4-position of UTP to create an activated intermediate, 4-phospho-UTP. The glutaminase domain generates ammonia from glutamine, via a covalent thioester intermediate with a conserved active site cysteine, generating glutamate. This ammonium is transferred from the glutaminase domain to the synthetase domain *via* a tunnel or can be derived from external ammonium. This ammonium is then used by the synthetase domain to generate CTP from the 4-phospho-UTP (Lieberman, 1956).

Although CTPS exists as two isoforms in humans and other eukaryotic organisms, CTPS1 and CTPS2, functional differences between the two isoforms are not yet fully elucidated (van Kuilenburg, *et al.* 2000).

The immune system provides protection from infections and has therefore evolved to rapidly respond to the wide variety of pathogens that the individual may be exposed to. This response can take many forms, but the expansion and differentiation of immune populations is a critical element and is hence closely linked to metabolic processes. Within this, CTP synthase activity appears to play an important role in DNA synthesis and the rapid expansion of lymphocytes following activation (Fairbanks, *et al.* 1995; van den Berg, *et al.* 1995).

Strong validation that CTPS1 is the critical enzyme in human lymphocyte proliferation came with the identification of a loss-of-function homozygous mutation (rs145092287) in this enzyme that causes a distinct and life-threatening immunodeficiency, characterized by an impaired capacity of activated T- and B-cells to proliferate in response to antigen receptor-mediated activation. Activated CTPS1-deficient cells were shown to have decreased levels of CTP. Normal T-cell proliferation was restored in CTPS1-deficient cells by expressing wild-type CTPS1 or by addition of exogenous CTP or its nucleoside precursor, cytidine. CTPS1 expression was found to be low in resting lymphocytes, but rapidly upregulated following activation of these cells. Expression of CTPS1 in other tissues was generally low. CTPS2 seems to be ubiquitously expressed in a range of cells and tissues but at low levels, and the failure of CTPS2, which is still intact in the patients, to compensate for the mutated CTPS1, supports CTPS1 being the critical enzyme for the immune populations affected in the patients (Martin, *et al.* 2014).

Overall, these findings suggest that CTPS1 is a critical enzyme necessary to meet the demands for the supply

of CTP required by several important immune populations.

Normally the immune response is tightly regulated to ensure protection from infection, whilst controlling any response targeting host tissues. In certain situations, the control of this process is not effective, leading to immune-mediated pathology. A wide range of human diseases are thought to be due to such inappropriate responses mediated by different elements of the immune system.

Given the role that cell populations, such as T and B lymphocytes, are thought to play in a wide range of autoimmune and other diseases, CTPS1 represents a target for a new class of immunosuppressive agents. Inhibition of CTPS1 therefore provides a novel approach to the inhibition of activated lymphocytes and selected other immune cell populations such as Natural Killer cells, Mucosal-Associated Invariant T (MAIT) and Invariant Natural Killer T cells, highlighted by the phenotype of the human mutation patients (Martin, *et al.* 2014).

As far as is known to date, no selective CTPS1 inhibitors have been developed. Several (presumed) non-selective CTPS inhibitors have been used for oncology indications up to phase I/II clinical trials, but were stopped due to toxicity and efficacy issues. More recently, the CTPS1 selective inhibitory peptide CTpep-3 has been identified. The inhibitory effects of CTpep-3 however, were seen in cell free assays but not in the cellular context. This was not unexpected though, since the peptide is unlikely to enter the cell and hence is not easily developable as a therapeutic (Sakamoto, *et al.* 2017).

In summary, the available information and data strongly suggest that inhibitors of CTPS1 will reduce the proliferation of a number of immune cell populations. Inhibitors of CTPS1 may therefore be expected to have utility for treatment or prophylaxis in a wide range of indications where the pathology is driven by these populations.

CTPS1 inhibitors represent a novel approach for inhibiting selected components of the immune system in various tissues, and the related pathologies or pathological conditions such as, in general terms, rejection of transplanted cells and tissues, Graft-related diseases or disorders, allergies and autoimmune diseases.

**Summary of the Invention**

The invention provides a compound of formula (I):

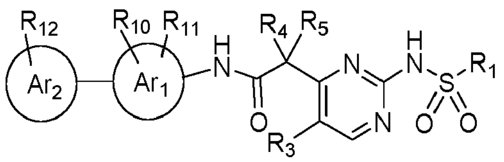

(I)

wherein

$R_1$ is $C_{1-5}$alkyl or $C_{0-2}$alkylene$C_{3-5}$cycloalkyl which cycloalkyl is optionally substituted by $CH_3$;

$R_3$ is H, halo or $CH_3$;

$R_4$ and $R_5$ are each independently H, $C_{1-6}$alkyl, $C_{1-3}$alkylene$OC_{1-3}$alkyl, or $R_4$ and $R_5$ together with the carbon atom to which they are attached form a $C_{3-6}$spirocycloalkyl;

Ar1 is a 6-membered aryl or heteroaryl;

Ar2 is a 6-membered aryl or heteroaryl and is attached to Ar1 in the para position relative to the amide;

$R_{10}$ is H, halo, $C_{1-2}$alkyl, $OC_{1-2}$alkyl, $OC_{1-2}$haloalkyl or CN;

$R_{11}$ is H, F, $CH_3$ or $OCH_3$; and

$R_{12}$ is attached to Ar2 in the ortho or meta position relative to Ar1 and $R_{12}$ is H, halo, $C_{1-4}$alkyl, $OC_{1-4}$alkyl, $OC_{0-2}$alkylene$C_{3-5}$cycloalkyl, CN, $C_{1-4}$haloalkyl, $OC_{1-4}$haloalkyl.

A compound of formula (I) may be provided in the form of a salt and/or solvate thereof and/or derivative thereof. Suitably, the compound of formula (I) may be provided in the form of a pharmaceutically acceptable salt and/or solvate thereof and/or derivative thereof. In particular, the compound of formula (I) may be provided in the form of a pharmaceutically acceptable salt and/or solvate, such as a pharmaceutically acceptable salt.

Also provided is a compound of formula (I), or a pharmaceutically acceptable salt and/or solvate thereof and/or derivative thereof, for use as a medicament, in particular for use in the inhibition of CTPS1 in a subject or the prophylaxis or treatment of associated diseases or disorders, such as those in which a reduction in T-cell and/or B-cell proliferation would be beneficial.

Further, there is provided a method for the inhibition of CTPS1 in a subject or the prophylaxis or treatment of associated diseases or disorders, such as those in which a reduction in T-cell and/or B-cell proliferation would be beneficial, by administering to a subject in need thereof a compound of formula (I) or a pharmaceutically acceptable salt and/or solvate thereof and/or derivative thereof.

Additionally provided is the use of a compound of formula (I), or a pharmaceutically acceptable salt and/or solvate thereof and/or derivative thereof, in the manufacture of a medicament for the inhibition of CTPS1 in a subject or the prophylaxis or treatment of associated diseases or disorders, such as those in which a reduction in T-cell and/or B-cell proliferation would be beneficial.

Suitably the disease or disorder is selected from: inflammatory skin diseases such as psoriasis or lichen planus; acute and/or chronic GVHD such as steroid resistant acute GVHD; acute lymphoproliferative syndrome (ALPS); systemic lupus erythematosus, lupus nephritis or cutaneous lupus; and transplantation.

Also provided are pharmaceutical compositions containing a compound of formula (I), or a pharmaceutically acceptable salt and/or solvate thereof and/or derivative thereof, and a pharmaceutically acceptable carrier or excipient.

Also provided are processes for preparing compounds of formula (I) and novel intermediates of use in the preparation of compounds of formula (I).

## Detailed description of the Invention

The invention provides a compound of formula (I):

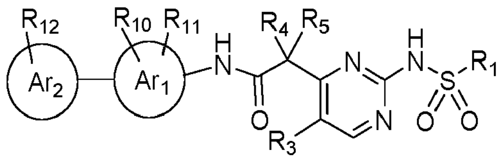

(I)

wherein

$R_1$ is $C_{1-5}$alkyl or $C_{0-2}$alkylene$C_{3-5}$cycloalkyl which cycloalkyl is optionally substituted by $CH_3$;

$R_3$ is H, halo or $CH_3$;

$R_4$ and $R_5$ are each independently H, $C_{1-6}$alkyl, $C_{1-3}$alkylene$OC_{1-3}$alkyl, or $R_4$ and $R_5$ together with the carbon atom to which they are attached form a $C_{3-6}$spirocycloalkyl;

Ar1 is a 6-membered aryl or heteroaryl;

Ar2 is a 6-membered aryl or heteroaryl and is attached to Ar1 in the para position relative to the amide;

$R_{10}$ is H, halo, $C_{1-2}$alkyl, $OC_{1-2}$alkyl, $OC_{1-2}$haloalkyl or CN;

$R_{11}$ is H, F, $CH_3$ or $OCH_3$; and

$R_{12}$ is attached to Ar2 in the ortho or meta position relative to Ar1 and $R_{12}$ is H, halo, $C_{1-4}$alkyl, $OC_{1-4}$alkyl, $OC_{0-2}$alkylene$C_{3-5}$cycloalkyl, CN, $C_{1-4}$haloalkyl, $OC_{1-4}$haloalkyl;

or a salt and/or solvate thereof and/or derivative thereof.

The term 'alkyl' as used herein, such as in $C_{1-3}$alkyl, $C_{1-4}$alkyl or $C_{1-5}$alkyl, whether alone or forming part of a larger group such as an Oalkyl group (e.g. $OC_{1-3}$alkyl, $OC_{1-4}$alkyl and $OC_{1-5}$alkyl), is a straight or a branched fully saturated hydrocarbon chain containing the specified number of carbon atoms. Examples of alkyl groups include the $C_{1-5}$alkyl groups methyl, ethyl, *n*-propyl, *iso*-propyl, *n*-butyl, *iso*-butyl, *sec*-butyl, *tert*-butyl and *n*-pentyl, *sec*-pentyl and 3-pentyl, in particular the $C_{1-3}$alkyl groups methyl, ethyl, *n*-propyl and *iso*-propyl. Reference to "propyl" includes *n*-propyl and *iso*-propyl, and reference to "butyl" includes *n*-butyl, isobutyl, *sec*-butyl and *tert*-butyl. Examples of Oalkyl groups

include the $OC_{1-4}$alkyl groups methoxy, ethoxy, propoxy (which includes *n*-propoxy and *iso*-propoxy) and butoxy (which includes *n*-butoxy, *iso*-butoxy, sec-butoxy and *tert*-butoxy).

The term 'alkylene' as used herein, such as in $C_{0-2}$alkylene$C_{3-5}$cycloalkyl or $C_{1-2}$alkylene$OC_{1-2}$alkyl is a bifunctional straight or a branched fully saturated hydrocarbon chain containing the specified number of carbon atoms. Examples of $C_{0-2}$alkylene groups are where the group is absent (i.e. $C_0$), methylene ($C_1$) and ethylene ($C_2$).

The term 'cycloalkyl' as used herein, such as in $C_{3-5}$cycloalkyl or $C_{3-6}$cycloalkyl, whether alone or forming part of a larger group such as $OC_{3-5}$cycloalkyl or $C_{0-2}$alkylene$C_{3-5}$cycloalkyl is a fully saturated hydrocarbon ring containing the specified number of carbon atoms. Examples of cycloalkyl groups include the $C_{3-6}$cycloalkyl groups cyclopropyl, cyclobutyl, cyclopentyl and cyclohexyl, in particular the $C_{3-5}$cycloalkyl groups cyclopropyl, cyclobutyl and cyclopentyl.

The term 'spirocycloalkyl' as used herein, such as in $C_{3-6}$spirocycloalkyl, is a fully saturated hydrocarbon ring containing the specified number of carbon atoms including the secondary carbon atom through which the spirocycloalkyl group is attached. Example of spirocycloalkyl groups include the $C_{3-6}$spirocycloalkyl groups cyclopropyl, cyclobutyl, cyclopentyl or cyclohexyl, in particular the $C_{3-5}$spirocycloalkyl groups cyclopropyl, cyclobutyl or cyclopentyl:

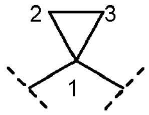
$C_3$ spirocycloalkyl

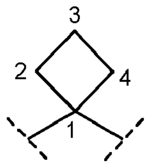
$C_4$ spirocycloalkyl

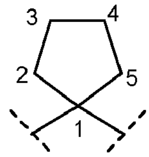
$C_5$ spirocycloalkyl

The term 'halo' or 'halogen' as used herein, refers to fluorine, chlorine, bromine or iodine. Particular examples of halo are fluorine and chlorine, especially fluorine.

The term 'haloalkyl' as used herein, such as in $C_{1-4}$haloalkyl, whether alone or forming part of a larger group such as an Ohaloalkyl group, such as in $OC_{1-4}$haloalkyl, is a straight or a branched fully saturated hydrocarbon chain containing the specified number of carbon atoms and at least one halogen atom, such as fluoro or chloro, especially fluoro. An example of haloalkyl is $CF_3$. Examples of Ohaloalkyl include $OCF_3$, $OCHF_2$ and $OCH_2CF_3$.

The term '6-membered aryl' as used herein refers to a phenyl ring.

The term '6-membered heteroaryl' as used herein refers to 6-membered aromatic rings containing at least one heteroatom (e.g. nitrogen). Exemplary 6-membered heteroaryls include one nitrogen atom (pyridinyl), two nitrogen atoms (pyridazinyl, pyrimidinyl or pyrazinyl) and three nitrogen atoms (triazinyl).

The phrase 'in the para position relative to the amide' as used herein, such as in relation to the position of Ar2, means that compounds with the following substructure are formed:

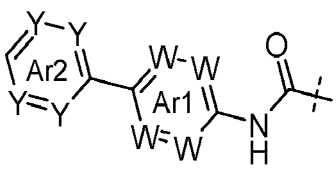

wherein W may be N, CH, $CR_{10}$ or $CR_{11}$, and Y may be N, CH or $CR_{12}$ as required by the definitions provided for compounds of formula (I).

The terms 'ortho' and 'meta' as used herein, such as when used in respect of defining the position of $R_{12}$ on Ar2 is with respect to Ar1:

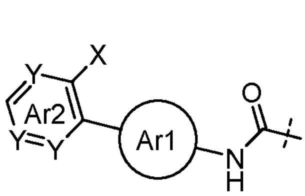
*ortho*

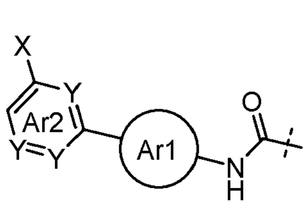
*meta*

In one embodiment of the invention $R_1$ is $C_{1-5}$alkyl. When $R_1$ is $C_{1-5}$alkyl, $R_1$ may be methyl, ethyl, propyl (n-propyl or isopropyl), butyl (n-butyl, isobutyl, sec-butyl or tert-butyl) or pentyl (e.g. n-pentyl, sec-pentyl or 3-pentyl).

In a second embodiment of the invention $R_1$ is $C_{0-2}$alkylene$C_{3-5}$cycloalkyl which cycloalkyl is optionally substituted by $CH_3$. In some embodiments, $R_1$ is $C_{0-2}$alkylene$C_{3-5}$cycloalkyl. In other embodiments, $R_1$ is $C_{0-2}$alkylene$C_{3-5}$cycloalkyl which cycloalkyl is substituted by $CH_3$. $R_1$ may be $C_{3-5}$cycloalkyl, which cycloalkyl is optionally substituted by $CH_3$. $R_1$ may be $C_1$alkylene$C_{3-5}$cycloalkyl, which cycloalkyl is optionally substituted by $CH_3$. $R_1$ may be $C_2$alkylene$C_{3-5}$cycloalkyl, which cycloalkyl is optionally substituted by $CH_3$. $R_1$ may be $C_{0-2}$alkylene$C_3$cycloalkyl, which cycloalkyl is optionally substituted by $CH_3$. $R_1$ may be $C_{0-2}$alkylene$C_4$cycloalkyl, which cycloalkyl is optionally substituted by $CH_3$. $R_1$ may be $C_{0-2}$alkylene$C_5$cycloalkyl, which cycloalkyl is optionally substituted by $CH_3$. Suitably, where $C_{0-2}$alkylene$C_{3-5}$cycloalkyl is optionally substituted by $CH_3$, the $CH_3$ is at the point of attachment of the $C_{3-5}$cycloalkyl to the $C_{0-2}$alkylene.

Suitably $R_1$ is cyclopropyl, cyclopropyl substituted by $CH_3$ at the point of attachment, cyclobutyl, $CH_3$ or $CH_2CH_3$. In particular $R_1$ is cyclopropyl, cyclobutyl, $CH_3$ or $CH_2CH_3$, especially cyclopropyl.

In one embodiment $R_3$ is H. In a second embodiment $R_3$ is halo, in particular chloro or fluoro, especially fluoro. In a third embodiment, $R_3$ is $CH_3$.

In one embodiment, $R_4$ and $R_5$ together with the carbon atom to which they are attached form a $C_{3-6}$spirocycloalkyl, such as spirocyclopropyl, spirocyclobutyl or spirocyclopentyl in particular spirocyclopropyl or spirocyclopentyl. In a second embodiment $R_4$ is $C_{1-6}$alkyl, in particular $C_{1-4}$alkyl such as methyl, ethyl, propyl (n-propyl or isopropyl) or butyl (n-butyl, isobutyl, sec-butyl or tert-butyl). In a third embodiment $R_4$ is $C_{1-3}$alkyleneO$C_{1-3}$alkyl, in particular $C_{1-2}$alkyleneO$C_{1-2}$alkyl such as $C_1$alkyleneO$C_1$alkyl, $C_2$alkyleneO$C_1$alkyl, $C_1$alkyleneO$C_2$alkyl or $C_2$alkyleneO$C_2$alkyl. In a fourth embodiment $R_4$ is H.

Suitably $R_4$ is H, $CH_3$, ethyl or $CH_2CH_2OCH_3$, in particular $CH_3$ or ethyl.

Suitably $R_4$ and $R_5$ together with the carbon atom to which they are attached form a spirocyclopropyl or spirocyclopentyl, in particular a spirocyclopentyl.

In one embodiment $R_5$ is $C_{1-6}$alkyl, in particular $C_{1-4}$alkyl such as methyl, ethyl, propyl (n-propyl or isopropyl) or butyl (n-butyl, isobutyl, sec-butyl or tert-butyl). In a second embodiment $R_5$ is $C_{1-3}$alkyleneO$C_{1-3}$alkyl, in particular $C_{1-2}$alkyleneO$C_{1-2}$alkyl such as $C_1$alkyleneO$C_1$alkyl, $C_2$alkyleneO$C_1$alkyl, $C_1$alkyleneO$C_2$alkyl or $C_2$alkyleneO$C_2$alkyl. In a third embodiment $R_5$ is H.

Suitably $R_5$ is H, $CH_3$ or ethyl.

Suitably $R_4$ is H, $CH_3$, ethyl or $CH_2CH_2OCH_3$ and $R_5$ is H, $CH_3$ or ethyl, in particular $R_4$ is $CH_3$, or ethyl and $R_5$ is H, methyl or ethyl. For example, $R_4$ and $R_5$ are H, $R_4$ and $R_5$ are methyl, $R_4$ and $R_5$ are ethyl or $R_4$ is $CH_2CH_2OCH_3$ and $R_5$ is H.

In one embodiment Ar1 is a 6-membered aryl, i.e. phenyl. In a second embodiment Ar1 is a 6-membered heteroaryl, in particular containing one nitrogen atom (pyridyl) or two nitrogen atoms (pyridazinyl, pyrimidinyl or pyrazinyl).

In particular Ar1 is phenyl or 2-pyridyl. The position numbering for Ar1 is in respect of the amide, with the carbon at the point of attachment designated position 1 and other numbers providing the relative location of the nitrogen atoms, for example:

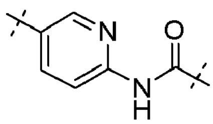

2-pyridyl

.

In one embodiment $R_{10}$ is H. In a second embodiment $R_{10}$ is halo, for example fluoro or chloro. In a third embodiment $R_{10}$ is $C_{1-2}$alkyl, such as $CH_3$ or ethyl. In a fourth embodiment $R_{10}$ is O$C_{1-2}$alkyl, such as $OCH_3$ or ethoxy. In a fifth embodiment $R_{10}$ is O$C_{1-2}$haloalkyl, such as $OCF_3$. In a sixth embodiment $R_{10}$ is CN.

Suitably $R_{10}$ is H, fluoro, chloro, $CH_3$, $OCH_3$, $OCF_3$ or CN, in particular H, fluoro, chloro, $OCH_3$, $OCF_3$ or CN especially H or fluoro.

In one embodiment $R_{11}$ is H. In a second embodiment $R_{11}$ is F. In a third embodiment, $R_{11}$ is $CH_3$. In a fourth embodiment $R_{11}$ is $OCH_3$.

In one embodiment Ar2 is a 6-membered aryl, i.e. phenyl. In a second embodiment Ar2 is a 6-membered heteroaryl, in particular containing one nitrogen atom (pyridyl) or two nitrogen atoms (pyridazinyl, pyrimidinyl or pyrazinyl).

The position numbering for Ar2 is in respect of the point of attachment to Ar1, for example:

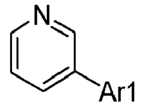

3-pyridyl

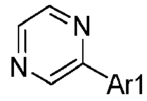

2,5-pyrazinyl .

In particular Ar2 is 3-pyridyl or 2,5-pyrazinyl, especially 2,5-pyrazinyl.

In one embodiment $R_{12}$ is H. In a second embodiment $R_{12}$ is halo, for example fluoro or chloro. In a third embodiment $R_{12}$ is $C_{1-4}$alkyl, such as methyl, ethyl, propyl (n-propyl or isopropyl) or butyl (n-butyl, isobutyl, sec-butyl or tert-butyl). In a fourth embodiment $R_{12}$ is $OC_{1-4}$alkyl, such as $OCH_3$, ethoxy, isopropoxy or n-propoxy. In a fifth embodiment $R_{12}$ is $OC_{0-2}$alkylene$C_{3-5}$cycloalkyl, such as $OC_{3-5}$cycloalkyl (e.g. cyclopropoxy or cyclobutoxy), $OC_1$alkylene$C_{3-5}$cycloalkyl or $OC_2$alkylene$C_{3-5}$cycloalkyl. In a sixth embodiment $R_{12}$ is CN. In a seventh embodiment $R_{12}$ is $C_{1-4}$haloalkyl, such as $CF_3$. In an eighth embodiment $R_{12}$ is $OC_{1-4}$haloalkyl, such as $OCF_3$, $OCHF_2$ or $OCH_2CF_3$.

$R_{12}$ is suitably H, F, Cl, $CH_3$, $OCH_3$, OEt, O*i*Pr, OCyclopropyl, CN, $CF_3$, $OCHF_2$ or $OCH_2CF_3$. In particular, $R_{12}$ is Cl, CN, $CF_3$, $OCHF_2$, $OCH_2CF_3$, $OCH_3$, OEt, O*i*Pr, OCyclopropyl, such as $CF_3$, $OCHF_2$, $OCH_2CF_3$, $OCH_3$, OEt, O*i*Pr, OCyclopropyl, e.g. OEt.

Suitably $R_{12}$ is in the meta position of Ar2. Alternatively, $R_{12}$ is in the ortho position of Ar2.

Throughout the specification Ar1 and Ar2 may be depicted as follows:

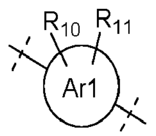 ; 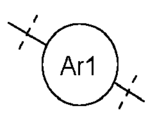 ; 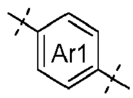 ; Ar1

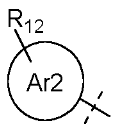 ; 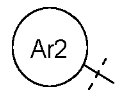 ; 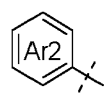 ; Ar2

All depictions with respect to Ar1 are equivalent and all depictions with respect to Ar2 are equivalent, unless the context requires otherwise, depictions of Ar1 and Ar2 should not be taken to exclude the presence of heteroatoms or substitutions.

The present invention provides the compounds described in any one of Examples P1 to P111.

The present invention provides the following compounds:

N-(4-(5-chloropyridin-3-yl)phenyl)-2-(2-(cyclopropanesulfonamido)pyrimidin-4-yl)butanamide;
1-(2-(cyclopropanesulfonamido)pyrimidin-4-yl)-N-(4-(6-ethoxypyrazin-2-yl)phenyl)cyclopentanecarboxamide;
2-(2-(cyclopropanesulfonamido)pyrimidin-4-yl)-N-(4-(6-methoxypyrazin-2-yl)phenyl)-2-methylpropanamide;
2-(2-(cyclopropanesulfonamido)pyrimidin-4-yl)-2-methyl-N-(4-(5-(trifluoromethyl)pyridin-3-yl)phenyl)propenamide;
2-methyl-N-(2-methyl-4-(6-methylpyrazin-2-yl)phenyl)-2-(2-(methylsulfonamido)pyrimidin-4-yl)propenamide;
2-(2-(cyclopropanesulfonamido)pyrimidin-4-yl)-N-(2-fluoro-4-(pyrazin-2-yl)phenyl)butanamide;
2-(2-(cyclopropanesulfonamido)pyrimidin-4-yl)-N-(4-(5-(trifluoromethyl)pyridin-3-yl)phenyl)butanamide;
2-(2-(cyclopropanesulfonamido)pyrimidin-4-yl)-N-(4-(6-(trifluoromethyl)pyrazin-2-yl)phenyl)acetamide;
2-(2-(cyclopropanesulfonamido)pyrimidin-4-yl)-N-(5-(6-isopropoxypyrazin-2-yl)pyridin-2-yl)-2-methylpropanamide;
2-(2-(cyclopropanesulfonamido)pyrimidin-4-yl)-N-(4-(6-ethoxypyrazin-2-yl)phenyl)-2-ethylbutanamide;
2-(2-(cyclopropanesulfonamido)pyrimidin-4-yl)-N-(2-fluoro-4-(6-(trifluoromethyl)pyrazin-2-yl)phenyl)acetamide;
2-(2-(cyclopropanesulfonamido)pyrimidin-4-yl)-N-(2-fluoro-4-(6-isopropoxypyrazin-2-yl)phenyl)acetamide;
2-(2-(cyclopropanesulfonamido)pyrimidin-4-yl)-N-(4-(5-(trifluoromethyl)pyridin-3-yl)phenyl)acetamide;
2-(2-(cyclopropanesulfonamido)pyrimidin-4-yl)-N-(4-(5-(2,2,2-trifluoroethoxy)pyridin-3-yl)phenyl)acetamide;
2-(2-(cyclopropanesulfonamido)-5-fluoropyrimidin-4-yl)-N-(4-(pyridin-3-yl)phenyl)acetamide;
2-(2-(cyclopropanesulfonamido)pyrimidin-4-yl)-N-(4-(pyridin-3-yl)phenyl)acetamide;
N-([1,1'-biphenyl]-4-yl)-2-(2-(cyclopropanesulfonamido)pyrimidin-4-yl)acetamide;
2-(2-(cyclopropanesulfonamido)pyrimidin-4-yl)-N-(4-(6-ethoxypyrazin-2-yl)phenyl)acetamide;
2-(2-(cyclopropanesulfonamido)pyrimidin-4-yl)-N-(4-(6-methoxypyrazin-2-yl)phenyl)acetamide;
2-(2-(cyclopropanesulfonamido)pyrimidin-4-yl)-N-(4-(6-(2,2,2-trifluoroethoxy)pyrazin-2-yl)phenyl)acetamide;

2-(2-(cyclopropanesulfonamido)pyrimidin-4-yl)-N-(4-(6-isopropoxypyrazin-2-yl)phenyl)acetamide;
2-(2-(cyclobutanesulfonamido)pyrimidin-4-yl)-N-(5-(6-ethoxypyrazin-2-yl)pyridin-2-yl)-2-methylpropanamide;
2-(2-(cyclobutanesulfonamido)pyrimidin-4-yl)-N-(2-fluoro-4-(6-isopropoxypyrazin-2-yl)phenyl)-2-methylpropanamide;
2-(2-(cyclobutanesulfonamido)pyrimidin-4-yl)-N-(4-(6-ethoxypyrazin-2-yl)-2-methylphenyl)-2-methylpropanamide;
2-(2-(cyclobutanesulfonamido)pyrimidin-4-yl)-N-(4-(6-methoxypyrazin-2-yl)phenyl)-2-methylpropanamide;
2-(2-(cyclobutanesulfonamido)pyrimidin-4-yl)-N-(4-(6-ethoxypyrazin-2-yl)phenyl)-2-methylpropanamide;
2-(2-(cyclopropanesulfonamido)pyrimidin-4-yl)-N-(5-(6-ethoxypyrazin-2-yl)-3-fluoropyridin-2-yl)-2-methylpropanamide;
2-(2-(cyclopropanesulfonamido)pyrimidin-4-yl)-N-(5'-ethoxy-[3,3'-bipyridin]-6-yl)-2-methylpropanamide;
N-([3,3'-bipyridin]-6-yl)-2-(2-(cyclopropanesulfonamido)pyrimidin-4-yl)-2-methylpropanamide;
2-(2-(cyclopropanesulfonamido)pyrimidin-4-yl)-2-methyl-N-(5-(6-(trifluoromethyl)pyrazin-2-yl)pyridin-2-yl)propenamide;
2-(2-(cyclopropanesulfonamido)pyrimidin-4-yl)-N-(5-(6-ethoxypyrazin-2-yl)pyridin-2-yl)-2-methylpropanamide;
2-(2-(cyclopropanesulfonamido)pyrimidin-4-yl)-N-(5-(6-cyclopropoxypyrazin-2-yl)pyridin-2-yl)-2-methylpropanamide;
N-(2-chloro-4-(6-ethoxypyrazin-2-yl)phenyl)-2-(2-(cyclopropanesulfonamido)pyrimidin-4-yl)-2-methylpropanamide;
N-(2-cyano-4-(6-ethoxypyrazin-2-yl)phenyl)-2-(2-(cyclopropanesulfonamido)pyrimidin-4-yl)-2-methylpropanamide;
2-(2-(cyclopropanesulfonamido)pyrimidin-4-yl)-N-(2-fluoro-4-(5-isopropoxypyridin-3-yl)phenyl)-2-methylpropanamide;
2-(2-(cyclopropanesulfonamido)pyrimidin-4-yl)-N-(2-fluoro-4-(pyridin-3-yl)phenyl)-2-methylpropanamide;
2-(2-(cyclopropanesulfonamido)pyrimidin-4-yl)-N-(2-fluoro-4-(6-(trifluoromethyl)pyrazin-2-yl)phenyl)-2-methylpropanamide;
2-(2-(cyclopropanesulfonamido)pyrimidin-4-yl)-N-(4-(6-ethoxypyrazin-2-yl)-2-fluorophenyl)-2-methylpropanamide;
2-(2-(cyclopropanesulfonamido)pyrimidin-4-yl)-N-(2-fluoro-4-(6-isopropoxypyrazin-2-yl)phenyl)-2-methylpropanamide;
2-(2-(cyclopropanesulfonamido)pyrimidin-4-yl)-N-(4-(6-ethoxypyrazin-2-yl)-2-fluoro-5-methylphenyl)-2-methylpropanamide;
2-(2-(cyclopropanesulfonamido)pyrimidin-4-yl)-N-(4-(6-ethoxypyrazin-2-yl)-2,6-difluorophenyl)-2-methylpropanamide;
2-(2-(cyclopropanesulfonamido)pyrimidin-4-yl)-N-(2-fluoro-4-(pyrazin-2-yl)phenyl)-2-methylpropanamide;
2-(2-(cyclopropanesulfonamido)pyrimidin-4-yl)-2-methyl-N-(2-methyl-4-(6-(trifluoromethyl)pyrazin-2-yl)phenyl)propenamide;
2-(2-(cyclopropanesulfonamido)pyrimidin-4-yl)-N-(4-(6-ethoxypyrazin-2-yl)-2,3-dimethylphenyl)-2-methylpropanamide;
2-(2-(cyclopropanesulfonamido)pyrimidin-4-yl)-N-(4-(6-ethoxypyrazin-2-yl)-5-fluoro-2-methylphenyl)-2-methylpropanamide;
2-(2-(cyclopropanesulfonamido)pyrimidin-4-yl)-N-(4-(6-ethoxypyrazin-2-yl)-2,5-dimethylphenyl)-2-methylpropanamide;
2-(2-(cyclopropanesulfonamido)pyrimidin-4-yl)-N-(4-(6-ethoxypyrazin-2-yl)-2-(trifluoromethoxy)phenyl)-2-methylpropanamide;
2-(2-(cyclopropanesulfonamido)pyrimidin-4-yl)-N-(4-(6-ethoxypyrazin-2-yl)-5-fluoro-2-methoxyphenyl)-2-methylpropanamide;
2-(2-(cyclopropanesulfonamido)pyrimidin-4-yl)-N-(4-(6-ethoxypyrazin-2-yl)-2-methoxyphenyl)-2-methylpropanamide;
2-(2-(cyclopropanesulfonamido)pyrimidin-4-yl)-2-methyl-N-(4-(pyrimidin-5-yl)phenyl)propenamide;
N-(4-(5-chloropyridin-3-yl)phenyl)-2-(2-(cyclopropanesulfonamido)pyrimidin-4-yl)-2-methylpropanamide;
N-(4-(5-cyanopyridin-3-yl)phenyl)-2-(2-(cyclopropanesulfonamido)pyrimidin-4-yl)-2-methylpropanamide;
2-(2-(cyclopropanesulfonamido)pyrimidin-4-yl)-N-(4-(5-fluoropyridin-3-yl)phenyl)-2-methylpropanamide;
2-(2-(cyclopropanesulfonamido)pyrimidin-4-yl)-2-methyl-N-(4-(5-methylpyridin-3-yl)phenyl)propenamide;
2-(2-(cyclopropanesulfonamido)pyrimidin-4-yl)-N-(4-(5-(difluoromethoxy)pyridin-3-yl)phenyl)-2-methylpropanamide;
2-(2-(cyclopropanesulfonamido)pyrimidin-4-yl)-N-(4-(5-methoxypyridin-3-yl)phenyl)-2-methylpropanamide;
2-(2-(cyclopropanesulfonamido)pyrimidin-4-yl)-N-(4-(5-ethoxypyridin-3-yl)phenyl)-2-methylpropanamide;
2-(2-(cyclopropanesulfonamido)pyrimidin-4-yl)-N-(4-(5-isopropoxypyridin-3-yl)phenyl)-2-methylpropanamide;
2-(2-(cyclopropanesulfonamido)pyrimidin-4-yl)-2-methyl-N-(4-(pyridin-3-yl)phenyl)propenamide;
2-(2-(cyclopropanesulfonamido)pyrimidin-4-yl)-2-methyl-N-(3'-(trifluoromethyl)-[1,1'-biphenyl]-4-yl)propenamide;

N-(3'-chloro-[1,1'-biphenyl]-4-yl)-2-(2-(cyclopropanesulfonamido)pyrimidin-4-yl)-2-methylpropanamide;
N-(3'-cyano-[1,1'-biphenyl]-4-yl)-2-(2-(cyclopropanesulfonamido)pyrimidin-4-yl)-2-methylpropanamide;
2-(2-(cyclopropanesulfonamido)pyrimidin-4-yl)-N-(3'-ethoxy-[1,1'-biphenyl]-4-yl)-2-methylpropanamide;
2-(2-(cyclopropanesulfonamido)pyrimidin-4-yl)-2-methyl-N-(4-(6-(trifluoromethyl)pyrazin-2-yl)phenyl)propenamide;
2-(2-(cyclopropanesulfonamido)pyrimidin-4-yl)-N-(4-(6-ethoxypyrazin-2-yl)phenyl)-2-methylpropanamide;
2-(2-(cyclopropanesulfonamido)pyrimidin-4-yl)-N-(4-(6-cyclopropoxypyrazin-2-yl)phenyl)-2-methylpropanamide;
2-(2-(cyclopropanesulfonamido)pyrimidin-4-yl)-N-(4-(6-isopropoxypyrazin-2-yl)phenyl)-2-methylpropanamide;
2-(2-(cyclopropanesulfonamido)-5-fluoropyrimidin-4-yl)-N-(4-(6-ethoxypyrazin-2-yl)phenyl)-2-methylpropanamide;
N-(4-(6-ethoxypyrazin-2-yl)phenyl)-2-methyl-2-(2-((1-methylcyclopropane)-1-sulfonamido)pyrimidin-4-yl)propenamide;
2-(2-(cyclopropanesulfonamido)-5-methylpyrimidin-4-yl)-N-(4-(6-ethoxypyrazin-2-yl)phenyl)-2-methylpropanamide;
2-(2-(cyclopropanesulfonamido)pyrimidin-4-yl)-2-methyl-N-(4-(pyrazin-2-yl)phenyl)propenamide;
N-(4-(6-ethoxypyrazin-2-yl)-2-fluorophenyl)-2-(2-(ethylsulfonamido)pyrimidin-4-yl)-2-methylpropanamide;
2-(2-(ethylsulfonamido)pyrimidin-4-yl)-2-methyl-N-(4-(6-(trifluoromethyl)pyrazin-2-yl)phenyl)propenamide;
N-(4-(6-ethoxypyrazin-2-yl)phenyl)-2-(2-(ethylsulfonamido)pyrimidin-4-yl)-2-methylpropanamide;
N-(5-(6-ethoxypyrazin-2-yl)-3-fluoropyridin-2-yl)-2-methyl-2-(2-(methylsulfonamido)pyrimidin-4-yl)propenamide;
N-(5-(6-ethoxypyrazin-2-yl)pyridin-2-yl)-2-methyl-2-(2-(methylsulfonamido)pyrimidin-4-yl)propenamide;
N-(2-fluoro-4-(5-isopropoxypyridin-3-yl)phenyl)-2-methyl-2-(2-(methylsulfonamido)pyrimidin-4-yl)propenamide;
N-(2-fluoro-4-(6-isopropoxypyrazin-2-yl)phenyl)-2-methyl-2-(2-(methylsulfonamido)pyrimidin-4-yl)propenamide;
2-methyl-N-(2-methyl-4-(6-(trifluoromethyl)pyrazin-2-yl)phenyl)-2-(2-(methylsulfonamido)pyrimidin-4-yl)propenamide;
2-methyl-2-(2-(methylsulfonamido)pyrimidin-4-yl)-N-(4-(6-(trifluoromethyl)pyrazin-2-yl)phenyl)propenamide;
N-(4-(6-ethoxypyrazin-2-yl)phenyl)-2-methyl-2-(2-(methylsulfonamido)pyrimidin-4-yl)propenamide;
2-(2-((1,1-dimethylethyl)sulfonamido)pyrimidin-4-yl)-N-(4-(6-ethoxypyrazin-2-yl)phenyl)-2-methylpropanamide;
1-(2-(cyclopropanesulfonamido)pyrimidin-4-yl)-N-(4-(6-ethoxypyrazin-2-yl)phenyl)cyclopropanecarboxamide;
2-(2-(cyclopropanesulfonamido)pyrimidin-4-yl)-N-(5'-(trifluoromethyl)-[3,3'-bipyridin]-6-yl)butanamide;
2-(2-(cyclopropanesulfonamido)pyrimidin-4-yl)-N-(5'-(2,2,2-trifluoroethoxy)-[3,3'-bipyridin]-6-yl)butanamide;
N-([3,3'-bipyridin]-6-yl)-2-(2-(cyclopropanesulfonamido)pyrimidin-4-yl)butanamide;
2-(2-(cyclopropanesulfonamido)pyrimidin-4-yl)-N-(5-(6-(trifluoromethyl)pyrazin-2-yl)pyridin-2-yl)butanamide;
2-(2-(cyclopropanesulfonamido)pyrimidin-4-yl)-N-(5-(6-ethoxypyrazin-2-yl)pyridin-2-yl)butanamide;
2-(2-(cyclopropanesulfonamido)pyrimidin-4-yl)-N-(5-(6-isopropoxypyrazin-2-yl)pyridin-2-yl)butanamide;
N-(4-(5-chloropyridin-3-yl)-2-fluorophenyl)-2-(2-(cyclopropanesulfonamido)pyrimidin-4-yl)butanamide;
2-(2-(cyclopropanesulfonamido)pyrimidin-4-yl)-N-(2-fluoro-4-(5-(2,2,2-trifluoroethoxy)pyridin-3-yl)phenyl)butanamide;
2-(2-(cyclopropanesulfonamido)pyrimidin-4-yl)-N-(2-fluoro-4-(5-isopropoxypyridin-3-yl)phenyl)butanamide;
2-(2-(cyclopropanesulfonamido)pyrimidin-4-yl)-N-(2-fluoro-4-(pyridin-3-yl)phenyl)butanamide;
2-(2-(cyclopropanesulfonamido)pyrimidin-4-yl)-N-(2-fluoro-4-(6-(trifluoromethyl)pyrazin-2-yl)phenyl)butanamide;
2-(2-(cyclopropanesulfonamido)pyrimidin-4-yl)-N-(2-fluoro-4-(6-methoxypyrazin-2-yl)phenyl)butanamide;
2-(2-(cyclopropanesulfonamido)pyrimidin-4-yl)-N-(4-(6-ethoxypyrazin-2-yl)-2-fluorophenyl)butanamide;
2-(2-(cyclopropanesulfonamido)pyrimidin-4-yl)-N-(2-fluoro-4-(6-isopropoxypyrazin-2-yl)phenyl)butanamide;
2-(2-(cyclopropanesulfonamido)pyrimidin-4-yl)-N-(2-fluoro-4-(6-(2,2,2-trifluoroethoxy)pyrazin-2-yl)phenyl)butanamide;
N-(4-(5-cyanopyridin-3-yl)phenyl)-2-(2-(cyclopropanesulfonamido)pyrimidin-4-yl)butanamide;
2-(2-(cyclopropanesulfonamido)pyrimidin-4-yl)-N-(4-(5-(2,2,2-trifluoroethoxy)pyridin-3-yl)phenyl)butanamide;
2-(2-(cyclopropanesulfonamido)pyrimidin-4-yl)-N-(4-(5-isopropoxypyridin-3-yl)phenyl)butanamide;
2-(2-(cyclopropanesulfonamido)pyrimidin-4-yl)-N-(4-(pyridin-3-yl)phenyl)butanamide;
2-(2-(cyclopropanesulfonamido)pyrimidin-4-yl)-N-(4-(6-(trifluoromethyl)pyrazin-2-yl)phenyl)butanamide;
N-(4-(6-chloropyrazin-2-yl)phenyl)-2-(2-(cyclopropanesulfonamido)pyrimidin-4-yl)butanamide;
2-(2-(cyclopropanesulfonamido)pyrimidin-4-yl)-N-(4-(6-ethoxypyrazin-2-yl)phenyl)butanamide;
2-(2-(cyclopropanesulfonamido)pyrimidin-4-yl)-N-(4-(6-methoxypyrazin-2-yl)phenyl)butanamide;
2-(2-(cyclopropanesulfonamido)pyrimidin-4-yl)-N-(4-(6-isopropoxypyrazin-2-yl)phenyl)butanamide;
2-(2-(cyclopropanesulfonamido)pyrimidin-4-yl)-N-(4-(6-(2,2,2-trifluoroethoxy)pyrazin-2-yl)phenyl)butanamide;
2-(2-(cyclopropanesulfonamido)pyrimidin-4-yl)-N-(4-(pyrazin-2-yl)phenyl)butanamide;
2-(2-(cyclopropanesulfonamido)pyrimidin-4-yl)-N-(4-(6-ethoxypyrazin-2-yl)phenyl)-4-methoxybutanamide; and
2-(2-(cyclopropanesulfonamido)pyrimidin-4-yl)-N-(4-(pyridin-3-yl)phenyl)propenamide.

The compounds of the invention may be provided in the form of a pharmaceutically acceptable salt and/or solvate thereof and/or derivative thereof. In particular, the compound of formula (I) may be provided in the form of a pharmaceutically acceptable salt and/or solvate, such as a pharmaceutically acceptable salt.

Compounds of the invention of particular interest are those demonstrating an $IC_{50}$ of 1uM or lower, especially 100nM or lower, in respect of CTPS1 enzyme, using the methods of the examples (or comparable methods).

It will be appreciated that for use in medicine the salts of the compounds of formula (I) should be pharmaceutically acceptable. Non-pharmaceutically acceptable salts of the compounds of formula (I) may be of use in other contexts. Suitable pharmaceutically acceptable salts will be apparent to those skilled in the art. Pharmaceutically acceptable salts include those described by Berge et al. (1977). Such pharmaceutically acceptable salts include acid and base addition salts. Pharmaceutically acceptable acid additional salts may be formed with inorganic acids e.g. hydrochloric, hydrobromic, sulphuric, nitric or phosphoric acid and organic acids e.g. succinic, maleic, acetic, fumaric, citric, tartaric, benzoic, p-toluenesulfonic, methanesulfonic or naphthalenesulfonic acid. Other salts e.g. oxalates or formates, may be used, for example in the isolation of compounds of formula (I) and are included within the scope of this invention.

Certain of the compounds of formula (I) may form acid or base addition salts with one or more equivalents of the acid or base. The present invention includes within its scope all possible stoichiometric and non-stoichiometric forms.

The compounds of formula (I) may be prepared in crystalline or non-crystalline form and, if crystalline, may optionally be solvated, e.g. as the hydrate. This invention includes within its scope stoichiometric solvates (e.g. hydrates) as well as compounds containing variable amounts of solvent (e.g. water).

It will be understood that the invention includes pharmaceutically acceptable derivatives of compounds of formula (I) and that these are included within the scope of the invention.

As used herein "pharmaceutically acceptable derivative" includes any pharmaceutically acceptable prodrug such as an ester or salt of such ester of a compound of formula (I) which, upon administration to the recipient is capable of providing (directly or indirectly) a compound of formula (I) or an active metabolite or residue thereof.

It is to be understood that the present invention encompasses all isomers of formula (I) and their pharmaceutically acceptable derivatives, including all geometric, tautomeric and optical forms, and mixtures thereof (e.g. racemic mixtures). Where additional chiral centres are present in compounds of formula (I), the present invention includes within its scope all possible diastereoisomers, including mixtures thereof. The different isomeric forms may be separated or resolved one from the other by conventional methods, or any given isomer may be obtained by conventional synthetic methods or by stereospecific or asymmetric syntheses.

The present disclosure includes all isotopic forms of the compounds of the invention provided herein, whether in a form (i) wherein all atoms of a given atomic number have a mass number (or mixture of mass numbers) which predominates in nature (referred to herein as the "natural isotopic form") or (ii) wherein one or more atoms are replaced by atoms having the same atomic number, but a mass number different from the mass number of atoms which predominates in nature (referred to herein as an "unnatural variant isotopic form"). It is understood that an atom may naturally exist as a mixture of mass numbers. The term "unnatural variant isotopic form" also includes embodiments in which the proportion of an atom of given atomic number having a mass number found less commonly in nature (referred to herein as an "uncommon isotope") has been increased relative to that which is naturally occurring e.g. to the level of >20%, >50%, >75%, >90%, >95% or > 99% by number of the atoms of that atomic number (the latter embodiment referred to as an "isotopically enriched variant form"). The term "unnatural variant isotopic form" also includes embodiments in which the proportion of an uncommon isotope has been reduced relative to that which is naturally occurring. Isotopic forms may include radioactive forms (i.e. they incorporate radioisotopes) and non-radioactive forms. Radioactive forms will typically be isotopically enriched variant forms.

An unnatural variant isotopic form of a compound may thus contain one or more artificial or uncommon isotopes such as deuterium ($^2H$ or D), carbon-11 ($^{11}C$), carbon-13 ($^{13}C$), carbon-14 ($^{14}C$), nitrogen-13 ($^{13}N$), nitrogen-15 ($^{15}N$), oxygen-15 ($^{15}O$), oxygen-17 ($^{17}O$), oxygen-18 ($^{18}O$), phosphorus-32 ($^{32}P$), sulphur-35 ($^{35}S$), chlorine-36 ($^{36}Cl$), chlorine-37 ($^{37}Cl$), fluorine-18 ($^{18}F$) iodine-123 ($^{121}I$), iodine-125 ($^{125}I$) in one or more atoms or may contain an increased proportion of said isotopes as compared with the proportion that predominates in nature in one or more atoms.

Unnatural variant isotopic forms comprising radioisotopes may, for example, be used for drug and/or substrate tissue distribution studies. The radioactive isotopes tritium, i.e. $^3H$, and carbon-14, i.e. $^{14}C$, are particularly useful for this purpose in view of their ease of incorporation and ready means of detection. Unnatural variant isotopic forms which incorporate deuterium i.e $^2H$ or D may afford certain therapeutic advantages resulting from greater metabolic stability, for example, increased *in vivo* half-life or reduced dosage requirements, and hence may be preferred in some circumstances. Further, unnatural variant isotopic forms may be prepared which incorporate positron emitting isotopes, such as $^{11}C$, $^{18}F$, $^{15}O$ and $^{13}N$, and would be useful in Positron Emission Topography (PET) studies for examining substrate receptor occupancy.

In one embodiment, the compounds of the invention are provided in a natural isotopic form.

In one embodiment, the compounds of the invention are provided in an unnatural variant isotopic form. In a specific embodiment, the unnatural variant isotopic form is a form in which deuterium (i.e. $^2H$ or D) is incorporated where

hydrogen is specified in the chemical structure in one or more atoms of a compound of the invention. In one embodiment, the atoms of the compounds of the invention are in an isotopic form which is not radioactive. In one embodiment, one or more atoms of the compounds of the invention are in an isotopic form which is radioactive. Suitably radioactive isotopes are stable isotopes. Suitably the unnatural variant isotopic form is a pharmaceutically acceptable form.

In one embodiment, a compound of the invention is provided whereby a single atom of the compound exists in an unnatural variant isotopic form. In another embodiment, a compound of the invention is provided whereby two or more atoms exist in an unnatural variant isotopic form.

Unnatural isotopic variant forms can generally be prepared by conventional techniques known to those skilled in the art or by processes described herein e.g. processes analogous to those described in the accompanying Examples for preparing natural isotopic forms. Thus, unnatural isotopic variant forms could be prepared by using appropriate isotopically variant (or labelled) reagents in place of the normal reagents employed in the Examples. Since the compounds of formula (I) are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions.

In general, the compounds of formula (I) may be made according to the organic synthesis techniques known to those skilled in this field, as well as by the representative methods set forth below, those in the Examples, and modifications thereof.

**General Routes:**

Generic routes by which compound examples of the invention may be conveniently prepared are summarised below.

## Scheme 1

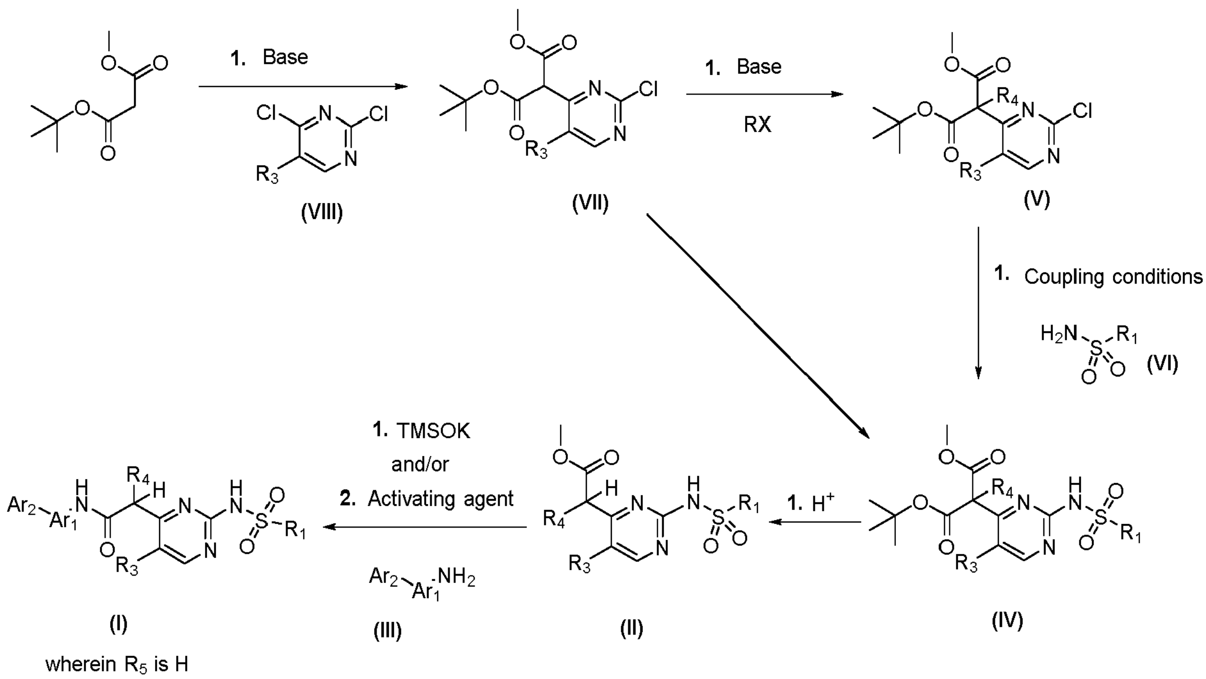


In general and as illustrated in Scheme 1, the compounds of formula (I) where $R_1$, $R_3$, Ar1 and Ar2 are defined above and where $R_4$ = H or Et, may be prepared in four or five steps starting from a 2,4-dichloropyrimidine derivative of general formula **(VIII).** The derivative **(VIII)** can be reacted with an unsymmetrical malonate ester derivative to displace the more reactive chloride and form intermediate compounds of formula **(VII).** Such reactions may be carried out in the presence of a strong base such as sodium hydride and in a polar solvent such as DMF. If mono alkylation is desired then treatment of intermediate **(VII)** with an inorganic base, such as sodium hydroxide, in the presence of an alkylating agent, such as iodoethane, yields compounds of the general formula **(V).** If a desmethyl ($R_4$ = H) linker is desired, compounds of general formula **(VII)** can be taken directly to compounds of general formula **(IV)** (see below).

Palladium catalysed amination of 2-chloropyrimidine derivative **(VII)** and **(V)** can be undertaken using a catalyst

such as [*t*-BuXPhos Pd(allyl)]Otf and substituted sulfonamide nucleophile **(VI),** in the presence of an inorganic base, for example potassium carbonate to form intermediate derivative **(IV).** This compound can then be deprotected *via* a decarboxylation, initiated by the use of a strong acid such as TFA to yield intermediate derivative **(II).** Such reactions are carried out in DCM at temperatures of 0 °C to room temperature.

Compounds of general formula **(I)** can be prepared by conversion of intermediate **(II)** by a one or two step process. Firstly, saponification using an agent such as TMSOK gives the intermediate carboxylic acid derivative followed by reaction with an activating agent, to generate a reactive, electrophilic carboxylic acid derivative, followed by subsequent reaction with an amine of formula **(III),** or a suitably protected derivative thereof. 2,4,6-Tripropyl-1,3,5,2,4,6-trioxatriphosphorinane-2,4,6-trioxide (T3P) is a reagent suitable for the activation of the carboxylate group. An alternative approach involves activation of the ester moiety directly using trimethylaluminium (usually a 2.0M solution in toluene or heptane) and addition of amine **(III).** These reactions are typically heated to 80 - 100 °C for a few hours in a solvent such as toluene.

## Scheme 2

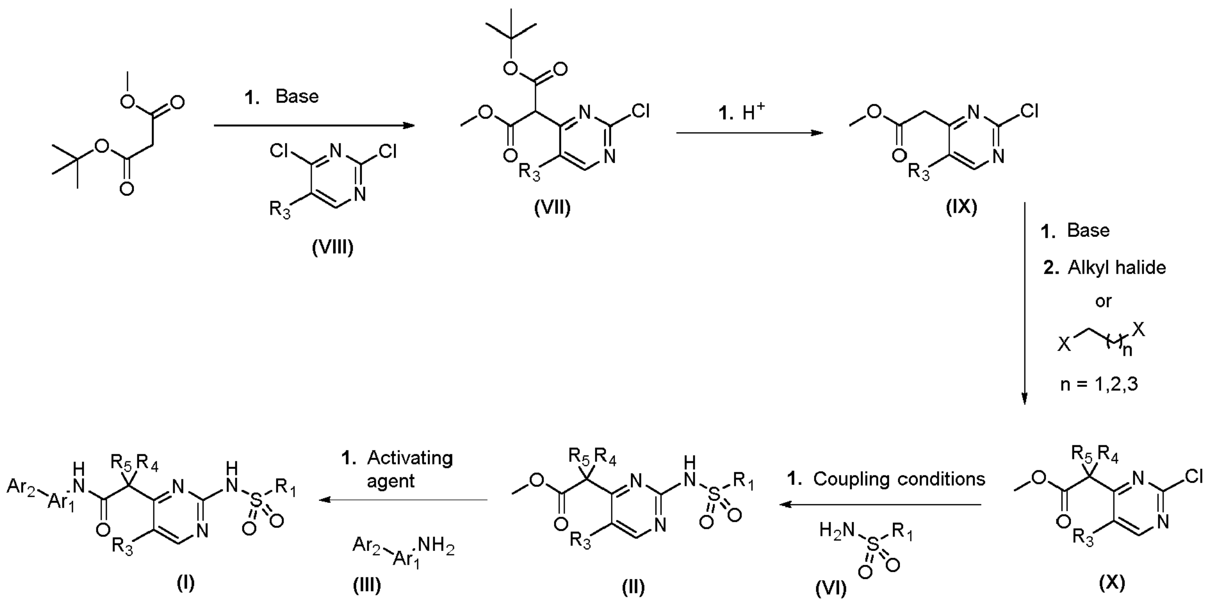


In general and as illustrated in Scheme 2, compounds of general formula **(I)** may be obtained by a five step process from a 2,4-dichloropyrimidine derivative of general formula **(VIII).** Firstly, the derivative **(VIII)** can be reacted with an unsymmetrical malonate ester as shown in Scheme 1. This intermediate compound can then be deprotected at this stage *via* a decarboxylation, initiated by the use of a strong acid such as TFA to yield intermediate derivative **(IX).** Certain intermediates such as **(IX)** where $R_3$ = H, are commercially available. Reaction of a methyl 2-(2-chloropyrimidin-4-yl)acetate derivative of general formula **(IX)** with an inorganic base such as potassium carbonate, in the presence of an alkylating agent leads to alkylation alpha to the ester. It will be understood by persons skilled in the art that both mono- and dialkylation may be achieved with careful control of the reaction conditions, but for a more reliable synthesis of the monoalkylated product, an alternative procedure should be considered (as in Scheme 1). $R_4$ and $R_5$ can be connected to form a $C_{3-6}$spirocycloalkyl ring as defined above ((**IX**) to (**X**)). Such compounds may be prepared by double alkylation with a dihaloalkane, such as 1,2-dibromoethane or 1,3-dibromobutane in the presence of an inorganic base such as sodium hydroxide.

Palladium catalysed amination of intermediate **(X)** may be achieved using a catalyst such as [*t*-BuXPhosPd(allyl)]OTf or *t*-BuXPhos-Pd-G3 and substituted sulfonamide nucleophile **(VI),** in the presence of an inorganic base, for example potassium carbonate to form intermediate derivative **(II).** Final transformation to compounds of general formula **(I)** can be prepared by conversion of intermediate **(II)** by activation of the ester moiety using trimethylaluminium (usually a 2.0 M solution in toluene or heptane) and addition of amine **(III)** (commercially available or prepared as in Scheme 4 or 5).

## Scheme 3

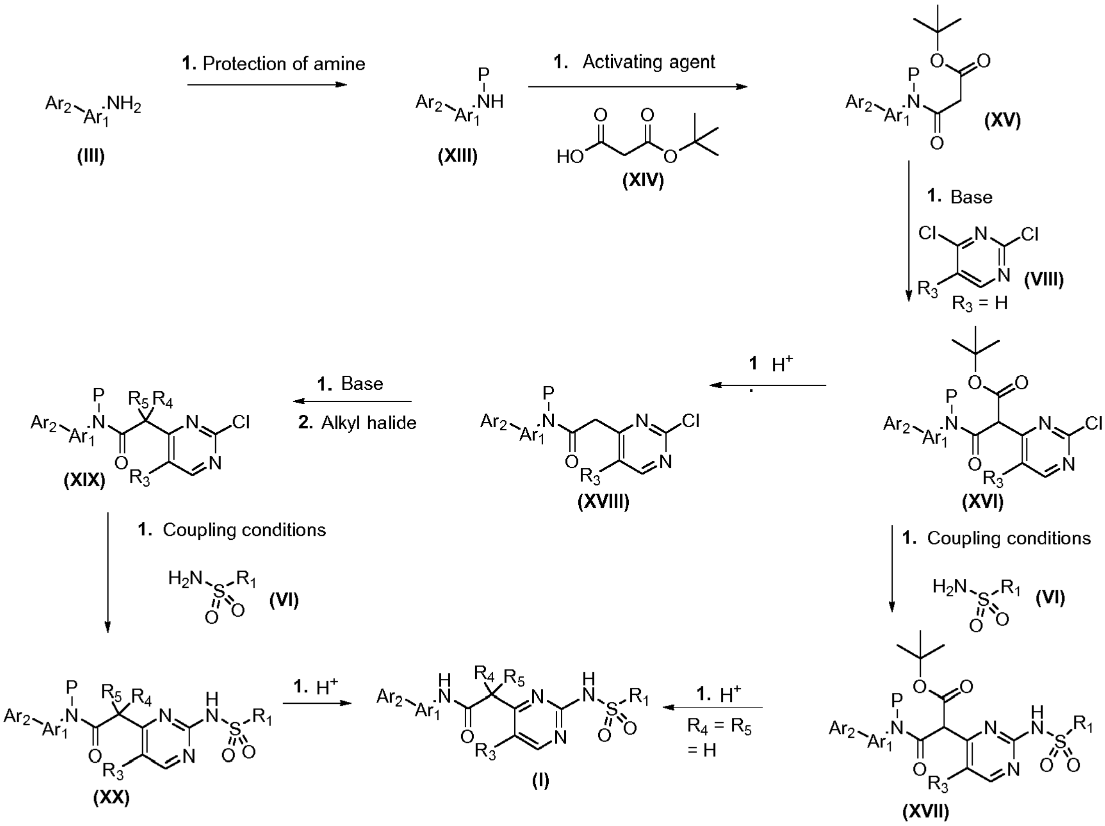


In general and as illustrated in Scheme 3, compounds of general formula **(I)** wherein $R_3$ is H may be obtained by a seven step process when $R_4$ and/or $R_5$ = alkyl (or five step process when $R_4$ = $R_5$ = H) from anilines of formula **(III)** defined in Scheme 4 and 5. Firstly, aniline **(III)** can be protected with a suitable nitrogen protecting group such as a para-methoxybenzyl ether group by reacting aniline **(III)** with 4-methoxybenzaldehyde followed by reduction in situ with reducing agents such as sodium triacetoxyborohydride. Protected aniline of formula **(XIII)** can then be reacted with 3-(tert-butoxy)-3-oxopropanoic acid **(XIV)** in presence of a coupling reagent such as HATU to obtain intermediates **(XV).** Such intermediates **(XV)** may undergo $S_NAr$ with 2,4-dichloropyrimidine **(VIII)** ($R_3$ = H) in the presence of a strong base such as NaH to give pyrimidines of formula **(XVI).** The intermediate **(XVI)** may then undergo two transformations.

Firstly, decarboxylation with a strong acid such as TFA to obtain intermediates of formula **(XVIII)** followed by alkylation in the presence of a base such as $K_2CO_3$ results in the formation compounds of formula **(XIX).** Palladium catalysed sulfonamidation of intermediate **(XIX)** may be achieved using a catalytic system such as Pd-174 in the presence of a sulphonamide of the type **(VI)** to obtain compounds of the formula **(XX).** Compounds of formula **(I)** may be obtained by deprotection of the aniline nitrogen using strong acidic system such as TFA/triflic acid.

Alternatively compounds of formula **(XVI)** may undergo sulfonamidation using sulphonamide of the type **(VI)** followed by double deprotection using a strong acidic system such as TFA/triflic acid to yield compounds of formula **(I).**

## Scheme 4

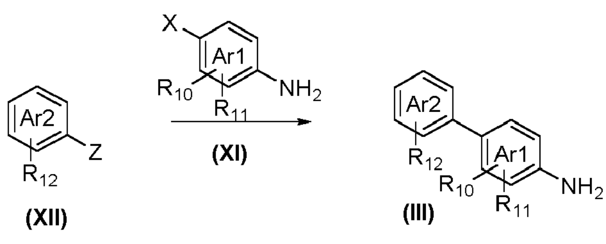

Z = $B(OH)_2$, $B(pin)_2$
X = Br, Cl

Intermediates of formula **(III)** wherein $R_{10}$, $R_{11}$ and $R_{12}$ are defined above and Ar2 is an unsubstituted or substituted 3-pyridyl ring, may be synthesised by coupling under Suzuki conditions of a boronate of general formula **(XII),** wherein $R_{12}$ is defined above and Z represents a dihydroxyboryl or dialkyloxyboryl group, usually a 4,4,5,5-tetramethyl-1,3,3,2-dioxaborolan-2-yl group, to a substituted pyridine of formula **(XI)** where X denotes a halide. The couplings according to the Suzuki method are performed, for example, by heating in the presence of a catalyst such as [1,1'-bis(diphenylphosphino)ferrocene]dichloropalladium(II) complex with dichloromethane and an inorganic base such as potassium carbonate in a solvent mixture of dioxane and water.

**Scheme 5**

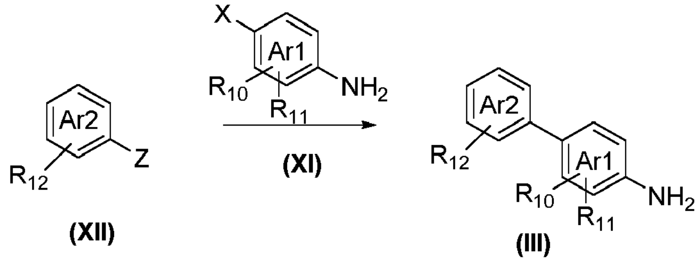
(XII) (XI) (III)

Z = Br, Cl
X = $B(OH)_2$, $B(pin)_2$

Intermediates of formula **(III)** wherein $R_{10}$, $R_{11}$ and $R_{12}$ are defined above and Ar2 is an unsubstituted or substituted 2,5-pyrazinyl ring, may be synthesised by coupling under Suzuki conditions of an aromatic halide of general formula **(XII)** and Z represents a halide, to a boronate of general formula **(XI)** where X denotes a dihydroxyboryl or dialkyloxyboryl group, usually a 4,4,5,5-tetramethyl-1,3,3,2-dioxaborolan-2-yl group. The couplings according to the Suzuki method are performed, for example, by heating in the presence of a catalyst such as tetrakis(triphenylphosphine)palladium or [1,1'-bis(diphenylphosphino)ferrocene]dichloro palladium(II) and an inorganic base such as potassium carbonate in a solvent mixture of dioxane and water.

**Intermediates of the Invention**

The present invention also relates to novel intermediates in the synthesis of compounds of formula (I) such as compounds of formula **(II)-(XX).** Particular intermediates of interest are those of the following general formulae, wherein the variable groups and associated preferences are as defined previously for compounds of formula (I):

- a compound of formula (II):

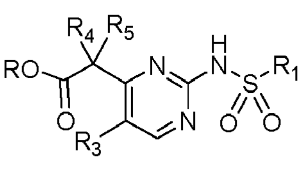
(II)

wherein R is H, $C_{1-6}$alkyl (e.g. methyl and ethyl) or benzyl;

- a compound of formula (III):

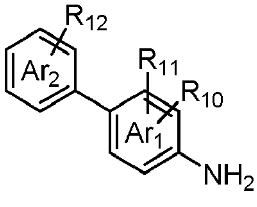
(III);

- a compound of formula (XX):

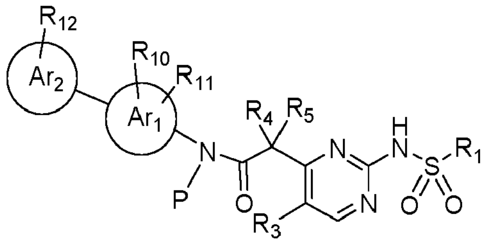

(XX);

wherein P is a nitrogen protecting group such as para-methoxybenzyl.

Included as an aspect of the invention are all novel intermediates described in the examples, including:

- Intermediates INTC1 to INTC47; and
- Intermediates INTD1 to INTD51.

**Therapeutic Methods**

Compounds of formula (I) of the present invention have utility as inhibitors of CTPS1.

Therefore, the invention also provides a compound of formula (I), or a pharmaceutically acceptable salt and/or solvate (e.g. salt) and/or derivative thereof, for use as a medicament, in particular in the treatment or prophylaxis of a disease or disorder wherein an inhibitor of CTPS1 is beneficial, for example those diseases and disorders mentioned herein below.

The invention provides a method for the treatment or prophylaxis of a disease or disorder wherein an inhibitor of CTPS1 is beneficial, for example those diseases and disorders mentioned herein below, which comprises administering to a subject in need thereof an effective amount of a compound of formula (I) or a pharmaceutically acceptable salt and/or solvate (e.g. salt) and/or derivative thereof.

The invention also provides the use of a compound of formula (I), or a pharmaceutically acceptable salt and/or solvate thereof (e.g. salt) and/or derivative, in the manufacture of a medicament for the treatment or prophylaxis of a disease or disorder wherein an inhibitor of CTPS1 is beneficial, for example those diseases and disorders mentioned herein below.

More suitably, the disease or disorder wherein an inhibitor of CTPS1 is beneficial is a disease or disorder wherein a reduction in T-cell and/or B-cell proliferation would be beneficial.

The invention also provides a compound of formula (I), or a pharmaceutically acceptable salt and/or solvate (e.g. salt) and/or derivative thereof, for use in the inhibition of CTPS1 in a subject.

The invention provides a method for the inhibition of CTPS1 in a subject, which comprises administering to the subject an effective amount of a compound of formula (I) or a pharmaceutically acceptable salt and/or solvate (e.g. salt) and/or derivative thereof.

The invention also provides the use of a compound of formula (I), or a pharmaceutically acceptable salt and/or solvate thereof (e.g. salt) and/or derivative, in the manufacture of a medicament for the inhibition of CTPS1 in a subject.

The invention also provides a compound of formula (I), or a pharmaceutically acceptable salt and/or solvate (e.g. salt) and/or derivative thereof, for use in the reduction of T-cell and/or B-cell proliferation in a subject.

The invention provides a method for the reduction of T-cell and/or B-cell proliferation in a subject, which comprises administering to the subject an effective amount of a compound of formula (I) or a pharmaceutically acceptable salt and/or solvate (e.g. salt) and/or derivative thereof.

The invention also provides the use of a compound of formula (I), or a pharmaceutically acceptable salt and/or solvate thereof (e.g. salt) and/or derivative, in the manufacture of a medicament for the reduction of T-cell and/or B-cell proliferation in a subject.

More suitably, the disease or disorder wherein an inhibitor of CTPS1 is beneficial is a disease or disorder wherein a reduction in T-cell and/or B-cell proliferation would be beneficial.

The term 'treatment' or 'treating' as used herein includes the control, mitigation, reduction, or modulation of the disease state or its symptoms.

The term 'prophylaxis' or 'preventing' is used herein to mean preventing symptoms of a disease or disorder in a subject or preventing recurrence of symptoms of a disease or disorder in an afflicted subject and is not limited to

complete prevention of an affliction.

Suitably, the disease or disorder is selected from rejection of transplanted cells and tissues, Graft-related diseases or disorders, allergies and autoimmune diseases.

In one embodiment the disease or disorder is the rejection of transplanted cells and tissues. The subject may have been transplanted with a graft selected from the group consisting of heart, kidney, lung, liver, pancreas, pancreatic islets, brain tissue, stomach, large intestine, small intestine, cornea, skin, trachea, bone, bone marrow (or any other source of hematopoietic precursor cells and stem cells including hematopoietic cells mobilized from bone marrow into peripheral blood or umbilical cord blood cells), muscle, or bladder. The compounds of the invention may be of use in preventing or suppressing an immune response associated with rejection of a donor tissue, cell, graft or organ transplant in a subject.

In a further embodiment the disease or disorder is a Graft-related disease or disorder. Graft-related diseases or disorders include graft versus host disease (GVHD), such as GVHD associated with bone marrow transplantation, and immune disorders resulting from or associated with rejection of organ, tissue, or cell graft transplantation (e.g., tissue or cell allografts or xenografts), including, e.g., grafts of skin, muscle, neurons, islets, organs, parenchymal cells of the liver, etc, and Host-Versus-Graft-Disease (HVGD). The compounds of the invention may be of use in preventing or suppressing acute rejection of such transplant in the recipient and/or for long-term maintenance therapy to prevent rejection of such transplant in the recipient (e.g., inhibiting rejection of insulin-producing islet cell transplant from a donor in the subject recipient suffering from diabetes). Thus the compounds of the invention have utility in preventing Host-Versus-Graft-Disease (HVGD) and Graft-Versus-Host-Disease (GVHD).

A CTPS1 inhibitor may be administered to the subject before, after transplantation and/or during transplantation. In some embodiments, the CTPS1 inhibitor may be administered to the subject on a periodic basis before and/or after transplantation.

In another embodiment, the disease or disorder is an allergy.

In additional embodiments the immune related disease or disorder is an autoimmune disease. As used herein, an "autoimmune disease" is a disease or disorder directed at a subject's own tissues. Examples of autoimmune diseases include, but are not limited to Addison's Disease, Adult-onset Still's disease, Alopecia Areata, Alzheimer's disease, Anti-neutrophil Cytoplasmic Antibodies (ANCA)-Associated Vasculitis, Ankylosing Spondylitis, Anti-phospholipid Syndrome (Hughes' Syndrome), Aplastic Anemia, Arthritis, Asthma, Atherosclerosis, Atherosclerotic plaque, Atopic Dermatitis, Autoimmune Hemolytic Anemia, Autoimmune Hepatitis, Autoimmune Hypophysitis (Lymphocytic Hypophysitis), Autoimmune Inner Ear Disease, Autoimmune Lymphoproliferative Syndrome, Autoimmune Myocarditis, Autoimmune Neutropenia, Autoimmune Oophoritis, Autoimmune Orchitis, Auto-Inflammatory Diseases requiring an immunosuppressive treatment, Azoospermia, Bechet's Disease, Berger's Disease, Bullous Pemphigoid, Cardiomyopathy, Cardiovascular disease, Celiac disease including Refractory Celiac Disease (type I and type II), Chronic Fatigue Immune Dysfunction Syndrome (CFIDS), Chronic Idiopathic Polyneuritis, Chronic Inflammatory Demyelinating Polyneuropathy (CIPD), Chronic Relapsing Polyneuropathy (Guillain-Barré syndrome), Churg-Strauss Syndrome (CSS), Cicatricial Pemphigoid, Cold Agglutinin Disease (CAD), chronic obstructive pulmonary disease (COPD), CREST Syndrome, Cryoglobulin Syndromes, Cutaneous Lupus, Dermatitis Herpetiformis, Dermatomyositis, Eczema, Epidermolysis Bullosa Acquisita, Essential Mixed Cryoglobulinemia, Evan's Syndrome, Exophthalmos, Fibromyalgia, Goodpasture's Syndrome, Grave's disease, Hemophagocytic Lymphohistiocytosis (HLH) (including Type 1 Hemophagocytic Lymphohistiocytosis), Histiocytosis/Histiocytic Disorders, Hashimoto's Thyroiditis, Idiopathic Pulmonary Fibrosis, Idiopathic Thrombocytopenia Purpura (ITP), IgA Nephropathy, Immunoproliferative Diseases or Disorders, Inflammatory Bowel Disease (IBD), Interstitial Lung Disease, Juvenile Arthritis, Juvenile Idiopathic Arthritis (JIA), Kawasaki's Disease, Lambert-Eaton Myasthenic Syndrome, Lichen Planus, Localized Scleroderma, Lupus Nephritis, Ménière's Disease, Microangiopathic Hemoytic Anemia, Microscopic Polyangitis, Miller Fischer Syndrome/Acute Disseminated Encephalomyeloradiculopathy, Mixed Connective Tissue Disease, Multiple Sclerosis (MS), Muscular Rheumatism, Myalgic Encephalomyelitis (ME), Myasthenia Gravis, Ocular Inflammation, Pemphigus Foliaceus, Pemphigus Vulgaris, Pernicious Anemia, Polyarteritis Nodosa, Polychondritis, Polyglandular Syndromes (Whitaker's syndrome), Polymyalgia Rheumatica, Polymyositis, Primary Agammaglobulinemia, Primary Biliary Cirrhosis/Autoimmune Cholangiopathy, Primary Glomerulonephritis, Primary Sclerosing Cholangitis, Psoriasis, Psoriatic Arthritis, Pure Red Cell Anemia, Raynaud's Phenomenon, Reiter's Syndrome/Reactive Arthritis, Relapsing Polychondritis, Restenosis, Rheumatic Fever, Rheumatic Disease, Rheumatoid Arthritis, Sarcoidosis, Schmidt's Syndrome, Scleroderma/Systemic Sclerosis, Sjörgen's Syndrome, Stiff-Man Syndrome, The Sweet Syndrome (Febrile Neutrophilic Dermatosis), Systemic Lupus Erythematosus (SLE), Systemic Scleroderma, Takayasu Arteritis, Temporal Arteritis/Giant Cell Arteritis, Thyroiditis, Type 1 diabetes, Type 2 diabetes, Uveitis, Vasculitis, Vitiligo, Wegener's Granulomatosis, and X-linked lymphoproliferative disease.

Of particular interest are diseases and disorders which are mainly driven by T-cell activation and proliferation, including:

- diseases and disorders which are not linked to alloreactivity including:

- Alopecia areata, atopic dermatitis, eczema, psoriasis, lichen planus, psoriatic arthritis, vitiligo;
- Uveitis;
- Ankylosing spondylitis, Reiter's syndrome/reactive arthritis;
- Aplastic anemia, autoimmune lymphoproliferative syndrome/disorders, hemophagocytic lymphohistiocytosis;
- Type 1 diabetes; and
- Refractory celiac disease;

- Acute rejection of grafted tissues and transplanted organs; acute graft versus host disease (GVHD) after transplantation of bone marrow cells or any other source of allogenic cells including hematopoietic precursors cells and/or stem cells.

Also of interest are diseases and disorders which are driven by both T- and B-cell activation and proliferation, with an important involvement of B-cells, including:

- diseases and disorders for which the involvement of pathogenic auto-antibodies is well characterized, including:
  - Allergy;
  - Cicatricial pemphigoid, bullous pemphigoid, epidermolysis bullosa acquisita, pemphigus foliaceus, pemphigus vulgaris, dermatitis herpetiformis;
  - ANCA-associated vasculitis and microscopic polyangitis, vasculitis, Wegener's granulomatosis; Churg-Strauss syndrome (CSS), polyarteritis nodosa, cryoglobulin syndromes and essential mixed cryglobulinemia;
  - Systemic lupus erythematosus (SLE), antiphospholipid syndrome (Hughes' syndrome), cutaneous lupus, lupus nephritis, mixed connective tissue disease;
  - Thyroiditis, Hashimoto thyroiditis, Grave's disease, exophthalmos;
  - Autoimmune hemolytic anemia, autoimmune neutropenia, ITP, pernicious anaemia, pure red cell anaemia, micro-angiopathic hemolytic anemia;
  - Primary glomerulonephritis, Berger's disease, Goodpasture's syndrome, IgA nephropathy; and
  - Chronic idiopathic polyneuritis, chronic inflammatory demyelinating polyneuropathy (CIPD), chronic relapsing polyneuropathy (Guillain-Barré syndrome), Miller Fischer syndrome, Stiff man syndrome, Lambert-Eaton myasthenic syndrome, myasthenia gravis.

- diseases and disorders for which the involvement of B-cells is less clearly characterized (although sometimes illustrated by the efficacy of anti-CD20 monoclonal antibodies or intravenous immunoglobulin infusions) and may not correspond or be limited to the production of pathogenic antibodies (nevertheless, non-pathogenic antibodies are sometimes described or even often present and used as a diagnosis biomarker), including:
  - Addison's disease, autoimmune oophoritis and azoospermia, polyglandular syndromes (Whitaker's syndrome), Schmidt's syndrome;
  - Autoimmune myocarditis, cardiomyopathy, Kawasaki's disease;
  - Rheumatoid arthritis, Sjögren's syndrome, mixed connective tissue disease, polymyositis and dermatomyositis; polychondritis;
  - Primary glomerulonephritis;

• Multiple sclerosis;

• Autoimmune hepatitis, primary biliary cirrhosis/ autoimmune cholangiopathy,

▪ Hyper acute rejection of transplanted organs;

▪ Chronic rejection of graft or transplants;

▪ Chronic Graft versus Host reaction / disease after transplantation of bone marrow cells or hematopoietic precursor cells.

Additionally of interest are diseases and disorders for which the mechanism is shared between activation/proliferation of T-cells and activation/proliferation of innate immune cells and other inflammatory cellular subpopulations (including myeloid cells such as macrophages or granulocytes) and resident cells (such as fibroblasts and endothelial cells), including:

▪ COPD, idiopathic pulmonary fibrosis, interstitial lung disease, sarcoidosis;

▪ Adult onset Still's disease, juvenile idiopathic arthritis, Systemic sclerosis, CREST syndrome where B cells and pathogen antibodies may also play a role; the Sweet syndrome; Takayasu arteritis, temporal arteritis/ giant cell arteritis;

▪ Ulcerative cholangitis, inflammatory bowel disease (IBD) including Crohn's disease and ulcerative colitis, primary sclerosing cholangitis.

Also of interest are diseases and disorders for which the mechanism remains poorly characterized but involves the activation and proliferation of T-cells, including:

▪ Alzheimer's disease, cardiovascular syndrome, type 2 diabetes, restenosis, chronic fatigue immune dysfuntion syndrome (CFIDS).

- Autoimmune Lymphoproliferative disorders, including:

▪ Autoimmune Lymphoproliferative Syndrome and X-linked lymphoproliferative disease.

Suitably the disease or disorder is selected from: inflammatory skin diseases such as psoriasis or lichen planus; acute and/or chronic GVHD such as steroid resistant acute GVHD; acute lymphoproliferative syndrome; systemic lupus erythematosus, lupus nephritis or cutaneous lupus; or transplantation..

Suitably the subject is a mammal, in particular the subject is a human.

**Pharmaceutical Compositions**

For use in therapy the compounds of the invention are usually administered as a pharmaceutical composition. The invention also provides a pharmaceutical composition comprising a compound of formula (I), or a pharmaceutically acceptable salt and/or solvate (e.g. salt) and/or derivative thereof, and a pharmaceutically acceptable carrier or excipient.

In one embodiment, there is provided a pharmaceutical composition comprising a compound of formula (I), or a pharmaceutically acceptable salt and/or solvate (e.g. salt) and/or derivative thereof, for use in the treatment or prophylaxis of a disease or disorder as described herein.

In a further embodiment, there is provided a method for the prophylaxis or treatment of a disease or disorder as described herein, which comprises administering to a subject in need thereof an effective amount of a pharmaceutical composition comprising a compound of formula (I) or a pharmaceutically acceptable salt and/or solvate (e.g. salt) and/or derivative thereof.

The invention also provides the use of a pharmaceutical composition comprising a compound of formula (I), or a pharmaceutically acceptable salt and/or solvate thereof (e.g. salt) and/or derivative thereof, in the manufacture of a medicament for the treatment or prophylaxis of a disease or disorder as described herein.

The compounds of formula (I) or their pharmaceutically acceptable salts and/or solvates and/or derivatives thereof may be administered by any convenient method, e.g. by oral, parenteral, buccal, sublingual, nasal, rectal or transdermal administration, and the pharmaceutical compositions adapted accordingly.

The compounds of formula (I) or their pharmaceutically acceptable salts and/or solvates and/or derivatives thereof which are active when given orally can be formulated as liquids or solids, e.g. as syrups, suspensions, emulsions, tablets, capsules or lozenges.

A liquid formulation will generally consist of a suspension or solution of the active ingredient (such as a compound of formula (I) or a pharmaceutically acceptable salt and/or solvate (e.g. salt) and/or derivative thereof) in a suitable liquid carrier(s) e.g. an aqueous solvent such as water, ethanol or glycerine, or a non-aqueous solvent, such as polyethylene glycol or an oil. The formulation may also contain a suspending agent, preservative, flavouring and/or colouring agent.

A composition in the form of a tablet can be prepared using any suitable pharmaceutical carrier(s) routinely used for preparing solid formulations, such as magnesium stearate, starch, lactose, sucrose and cellulose.

A composition in the form of a capsule can be prepared using routine encapsulation procedures, e.g. pellets containing the active ingredient (such as a compound of formula (I) or a pharmaceutically acceptable salt and/or solvate (e.g. salt) and/or derivative thereof) can be prepared using standard carriers and then filled into a hard gelatin capsule; alternatively a dispersion or suspension can be prepared using any suitable pharmaceutical carrier(s), e.g. aqueous gums, celluloses, silicates or oils and the dispersion or suspension then filled into a soft gelatin capsule.

Typical parenteral compositions consist of a solution or suspension of the active ingredient (such as a compound of formula (I) or a pharmaceutically acceptable salt and/or solvate (e.g. salt) and/or derivative thereof) in a sterile aqueous carrier or parenterally acceptable oil, e.g. polyethylene glycol, polyvinyl pyrrolidone, lecithin, arachis oil or sesame oil. Alternatively, the solution can be lyophilised and then reconstituted with a suitable solvent just prior to administration.

Compositions for nasal administration may conveniently be formulated as aerosols, drops, gels and powders. Aerosol formulations typically comprise a solution or fine suspension of the active ingredient in a pharmaceutically acceptable aqueous or non-aqueous solvent and are usually presented in single or multidose quantities in sterile form in a sealed container which can take the form of a cartridge or refill for use with an atomising device. Alternatively the sealed container may be a disposable dispensing device such as a single dose nasal inhaler or an aerosol dispenser fitted with a metering valve. Where the dosage form comprises an aerosol dispenser, it will contain a propellant which can be a compressed gas e.g. air, or an organic propellant such as a fluoro-chloro-hydrocarbon or hydrofluorocarbon. Aerosol dosage forms can also take the form of pump-atomisers.

Compositions suitable for buccal or sublingual administration include tablets, lozenges and pastilles where the active ingredient is formulated with a carrier such as sugar and acacia, tragacanth, or gelatin and glycerin.

Compositions for rectal administration are conveniently in the form of suppositories containing a conventional suppository base such as cocoa butter.

Compositions suitable for transdermal administration include ointments, gels and patches.

Suitably, the composition is in unit dose form such as a tablet, capsule or ampoule.

The composition may for example contain from 0.1% to 100% by weight, for example from 10 to 60% by weight, of the active material, depending on the method of administration. The composition may contain from 0% to 99% by weight, for example 40% to 90% by weight, of the carrier, depending on the method of administration. The composition may contain from 0.05 mg to 2000 mg, for example from 1.0 mg to 500 mg, of the active material, depending on the method of administration. The composition may contain from 50 mg to 1000 mg, for example from 100 mg to 400 mg of the carrier, depending on the method of administration. The dose of the compound used in the treatment or prophylaxis of the aforementioned disorders will vary in the usual way with the seriousness of the disorders, the weight of the sufferer, and other similar factors. However, as a general guide suitable unit doses may be 0.05 mg to 1000 mg, more suitably 1.0 mg to 500 mg, and such unit doses may be administered more than once a day, for example two or three a day. Such therapy may extend for a number of weeks or months.

The invention provides, in a further aspect, a combination comprising a compound of formula (I) or a pharmaceutically acceptable, salt, solvate and/or derivative thereof (e.g. a combination comprising a compound of formula (I) or a pharmaceutically acceptable derivative thereof) together with a further pharmaceutically acceptable active ingredient or ingredients.

The invention provides a compound of formula (I), for use in combination with a further pharmaceutically acceptable active ingredient or ingredients.

When the compounds are used in combination with other therapeutic agents, the compounds may be administered separately, sequentially or simultaneously by any convenient route.

Optimal combinations may depend on the disease or disorder. Possible combinations include those with one or more active agents selected from the list consisting of: 5-aminosalicylic acid, or a prodrug thereof (such as sulfasalazine, olsalazine or bisalazide); corticosteroids (e.g. prednisolone, methylprednisolone, or budesonide); immunosuppressants (e.g. cyclosporin, tacrolimus, sirolimus, methotrexate, azathioprine mycophenolate mofetil, leflunomide, cyclophosphamide, 6-mercaptopurine or antilymphocyte (or thymocyte) globulins); anti-TNF-alpha antibodies (e.g., infliximab, adalimumab, certolizumab pegol or golimumab); anti-IL12/IL23 antibodies (e.g., ustekinumab); anti-IL6 or anti-IL6R antibodies, anti-IL17 antibodies or small molecule IL12/IL23 inhibitors (e.g., apilimod); Anti-alpha-4-beta-7 antibodies (e.g., vedolizumab); MAdCAM-1 blockers (e.g., PF-00547659); antibodies against the cell adhesion molecule alpha-4-integrin

(e.g., natalizumab); antibodies against the IL2 receptor alpha subunit (e.g., daclizumab or basiliximab); JAK inhibitors including JAK1 and JAK3 inhibitors (e.g., tofacitinib, baricitinib, R348); Syk inhibitors and prodrugs thereof (e.g., fostamatinib and R-406); Phosphodiesterase-4 inhibitors (e.g., tetomilast); HMPL-004; probiotics; Dersalazine; semapimod/CPSI-2364; and protein kinase C inhibitors (e.g. AEB-071).

Some of the combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations. The individual components of combinations may also be administered separately, through the same or different routes.

When a compound of formula (I) or a pharmaceutically acceptable derivative thereof is used in combination with a second therapeutic agent active against the same disease state the dose of each compound may differ from that when the compound is used alone. Appropriate doses will be readily appreciated by those skilled in the art.

The invention is further exemplified by the following non-limiting examples.

## EXAMPLES

Abbreviations used herein are defined below. Any abbreviations not defined are intended to convey their generally accepted meaning.

### Abbreviations

| | |
|---|---|
| AcOH | glacial acetic acid |
| $AlMe_3$ | trimethylaluminium |
| aq | aqueous |
| Ar | Aromatic ring |
| BEH | ethylene bridged hybrid |
| Bispin | Bis(pinacolato)diboron; 4,4,4',4',5,5,5',5'-Octamethyl-2,2'-bi-1,3,2-dioxaborolane |
| BOC | *tert*-butyloxycarbonyl protecting group |
| $CS_2CO_3$ | Cesium carbonate |
| CSH | charged surface hybrid |
| d | doublet |
| DCM | dichloromethane |
| DIPEA | N,N-diisopropylethylamine |
| dioxane | 1,4-dioxane |
| DMF | N,N-dimethylformamide |
| DMSO | dimethyl sulfoxide |
| dppf | 1,1'-bis(diphenylphosphino)ferrocene |
| $(ES^+)$ | electrospray ionisation, positive mode |
| $(ES^-)$ | electrospray ionisation, negative mode |
| ESI | electrospray ionisation |
| Et | ethyl |
| EtOAc | ethyl acetate |
| EtOH | ethanol |
| g | grams |
| HATU | 1-[bis(dimethylamino)methylene]-1*H*-1,2,3-triazolo[4,5-*b*]pyridinium 3-oxid hexafluorophosphate |
| HPLC | high performance liquid chromatography |
| hr(s) | hour(s) |
| $IC_{50}$ | 50% inhibitory concentration |
| $K_2CO_3$ | potassium carbonate |
| LCMS | liquid chromatography-mass spectrometry |
| LiOH | lithium hydroxide |
| $(M+H)^+$ | protonated molecular ion |
| $(M-H)^-$ | unprotonated molecular ion |
| M | molar concentration |

| | |
|---|---|
| mL | millilitre |
| mm | millimiter |
| mmol | millimole |
| Me | methyl |
| MeCN | acetonitrile |
| MeI | iodomethane |
| MeOH | methanol |
| MHz | megahertz |
| min(s) | minute(s) |
| MSD | mass selective detector |
| m/z | mass-to-charge ratio |
| $N_2$ | nitrogen gas |
| $NH_3$ | ammonia |
| $NH_4Cl$ | Ammonium chloride |
| NaH | sodium hydride |
| $NaHCO_3$ | sodium bicarbonate |
| nm | nanometre |
| NMR | nuclear magnetic resonance (spectroscopy) |
| PDA | photodiode array |
| Pd 170 | chloro(crotyl)(2-dicyclohexylphosphino-2',4',6'-triisopropybiphenyl)palladium(II) or XPhos Pd(crotyl)Cl |
| Pd 174 | allyl(2-di-*tert*-butylphosphino-2',4',6'-triisopropyl-1,1'-biphenyl)palladium(II) triflate or [*t*BuXPhosPd(allyl)]OTf |
| $[Pd(allyl)Cl_2]_2$ | Bis(allyl)dichlorodipalladium |
| $PdCl_2$(dppf) | [1,1'-Bis(diphenylphosphino)ferrocene]dichloropalladium(II) |
| $Pd(PPh_3)_4$ | tetrakis(triphenylphosphine)palladium(0) |
| prep HPLC | preparative high performance liquid chromatography |
| Ph | phenyl |
| pos/neg | Positive/negative |
| q | quartet |
| RT | room temperature |
| Rt | retention time |
| RP | reverse phase |
| s | singlet |
| $S_NAr$ | nucleophilic aromatic substitution |
| sat | saturated |
| SCX | solid supported cation exchange (resin) |
| t | triplet |
| T3P | 2,4,6-tripropyl-1,3,5,2,4,6-trioxatriphosphorinane-2,4,6-trioxide |
| TBME | *tert*-butyl methyl ether |
| TFA | Trifluoroacetic acid |
| TMSOK | potassium trimethylsilanolate |
| [*t*-BuXPhos Pd(allyl)]OTf | allyl(2-di-tert -butylphosphino-2',4',6'-triisopropyl-1,1'-biphenyl)palladium(II) triflate |
| THF | tetrahydrofuran |
| UPLC | ultra performance liquid chromatography |
| UV | ultraviolet |
| v/v | volume/volume |
| VWD | variable wave detector |
| wt | weight |
| um | micrometre |
| uL | microlitre |
| °C | degrees Celsius |

**General Procedures**

All starting materials and solvents were obtained either from commercial sources or prepared according to the literature. Unless otherwise stated all reactions were stirred. Organic solutions were routinely dried over anhydrous magnesium sulfate. Hydrogenations were performed on a Thales H-cube flow reactor under the conditions stated.

Column chromatography was performed on pre-packed silica (230-400 mesh, 40-63 um) cartridges using the amount indicated. SCX was purchased from Supelco and treated with 1M hydrochloric acid prior to use. Unless stated otherwise the reaction mixture to be purified was first diluted with MeOH and made acidic with a few drops of AcOH. This solution was loaded directly onto the SCX and washed with MeOH. The desired material was then eluted by washing with 0.7 M $NH_3$ in MeOH.

*Preparative Reverse Phase High Performance Liquid Chromatography*

**Prep HPLC**

***Acidic prep***

Waters X-Select CSH column C18, 5 um (19 x 50 mm), flow rate 28 mL $min^{-1}$ eluting with a $H_2O$-MeCN gradient containing 0.1 % v/v formic acid over 6.5 min using UV detection at 254 nm.

***Basic prep***

Waters X-Bridge Prep column C18, 5 um (19 x 50 mm), flow rate 28 mL $min^{-1}$ eluting with a 10 mM $NH_4HCO_3$-MeCN gradient over 6.5 min using UV detection at 254 nm.

**Analytical Methods**

*Reverse Phase HPLC Conditions for the LCMS Analytical Methods*

**HPLC acidic:** Acidic LCMS 4 minute (5-95%)

Analytical LCMS was carried out using a Waters X-Select CSH C18, 2.5 um, 4.6x30 mm column eluting with a gradient of 0.1 % Formic acid in MeCN in 0.1 % Formic acid in water. The gradient from 5-95 % 0.1 % Formic acid in MeCN occurs between 0.00-3.00 minutes at 2.5 mL/min with a flush from 3.01-3.5 minutes at 4.5 mL/min. A column re-equilibration to 5% MeCN is from 3.60-4.00 minutes at 2.5 mL/min. UV spectra of the eluted peaks were measured using an Agilent 1260 Infinity VWD at 254 nm. Mass spectra were measured using an Agilent 6120 MSD running with positive/negative switching.

**HPLC basic:** Basic LCMS 4 minute (5-95%)

Analytical LCMS was carried out using a Waters X-Select BEH C18, 2.5 um, 4.6x30 mm column eluting with a gradient of MeCN in aqueous 10mM ammonium bicarbonate. The gradient from 5-95% MeCN occurs between 0.00-3.00 minutes at 2.5mL/min with a flush from 3.01-3.5 minutes at 4.5 mL/min. A column re-equilibration to 5% MeCN is from 3.60-4.00 minutes at 2.5mL/min. UV spectra of the eluted peaks were measured using an Agilent 1260 Infinity VWD at 254nm. Mass spectra were measured using an Agilent 6120 MSD running with positive/negative switching.

*Reverse Phase HPLC Conditions for the UPLC Analytical Methods*

**UPLC acidic:** Acidic UPLC 3 minute

Analytical UPLC/MS was carried out using a Waters Acquity CSH C18, 1.7 um, 2.1x30 mm column eluting with a gradient of 0.1% Formic acid in MeCN in0.1% Formic acid in water. The gradient is structured with a starting point of 5% MeCN held from 0.0-0.11 minutes. The gradient from 5-95% occurs between 0.11-2.15 minutes with a flush from 2.15-2.56 minutes. A column re-equilibration to 5% MeCN is from 2.56-2.83 minutes. UV spectra of the eluted peaks were measured using an Acquity PDA and mass spectra were recorded using an Acquity QDa detector with ESI pos/neg switching.

**UPLC basic:** Basic UPLC 3 minute

Analytical UPLC/MS was carried out using a Waters Acquity BEH C18, 1.7 um, 2.1x30 mm column eluting with a gradient of MeCN in aqueous 10 mM Ammonium Bicarbonate. The gradient is structured with a starting point of 5% MeCN held from 0.0-0.11 minutes. The gradient from 5-95% occurs between 0.11-2.15 minutes with a flush from 2.15-2.56 minutes. A column re-equilibration to 5% MeCN is from 2.56-2.83 minutes. UV spectra of the eluted peaks were measured

using an Acquity PDA and mass spectra were recorded using an Acquity QDa detector with ESI pos/neg switching.

Column temperature is 40 °C in all runs. Injection volume is 3 uL and the flow rate is 0.77 mL/min. PDA scan from 210-400 nm on all runs.

*$^1$H NMR Spectroscopy*

$^1$H NMR spectra were acquired on a Bruker Avance III spectrometer at 400 MHz or Bruker Avance III HD spectrometer at 500 MHz using residual undeuterated solvent as reference and unless specified otherwise were run in DMSO-d6.

**Preparation of Intermediates**

Known synthetic intermediates were procured from commercial sources or were obtained using published literature procedures. Additional intermediates were prepared by the representative synthetic processes described herein.

1-(*tert*-Butyl) 3-methyl 2-(2-chloropyrimidin-4-yl)malonate **INTC1**

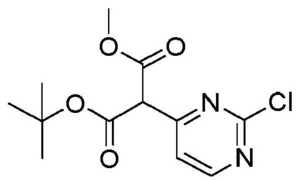

NaH (60 wt % in mineral oil, 5.10 g, 128 mmol) was added portionwise to an ice-cooled, stirred solution of *tert-butyl* methyl malonate (20.5 mL, 121 mmol) in THF (160 mL). The reaction was stirred at 0 °C for 20 mins then at RT for 60 mins until evolution of hydrogen ceased. 2,4-dichloropyrimidine (10 g, 67.1 mmol) was then added and the resulting mixture was stirred at 70 °C for 3 hrs. The reaction was allowed to cool, partitioned between $NH_4Cl$ (sat. aq, 500 mL) and EtOAc (500 mL), the two phases were separated and the organic layer was passed through a phase separator. The crude product was purified by chromatography on silica gel (220 g column, 0-30% EtOAc/*iso*-hexane) to afford 1-*tert*-butyl 3-methyl 2-(2-chloropyrimidin-4-yl)malonate (13.1 g, 44.3 mmol, 66 % yield) as a clear pale yellow oil; Rt 2.09 mins (HPLC acidic); m/z 230 $(M+H-tBu)^+$ $(ES^+)$ and 287 $(M+H)^+$ $(ES^+)$; $^1$H NMR (400 MHz, DMSO-d6) δ 8.83 (d, J = 5.1 Hz, 1H), 7.65 (d, J = 5.1 Hz, 1H), 5.21 (s, 1H), 3.73 (s, 3H), 1.42 (s, 9H).

1-(*tert*-Butyl) 3-methyl 2-(2-chloro-5-fluoropyrimidin-4-yl)malonate **INTC2**

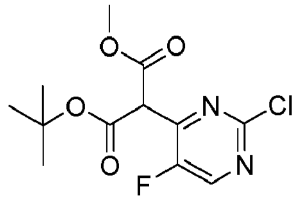

NaH (60 wt % in mineral oil, 0.575 g, 14.4 mmol) was added portionwise to a stirred solution of tert-butyl methyl malonate (2.23 mL, 13.2 mmol) in DMF (20 mL). The reaction was stirred at RT under $N_2$ for 10 mins until evolution of hydrogen ceased. The reaction was cooled to 0 °C and 2,4-dichloro-5-fluoropyrimidine (2.0 g, 12.0 mmol) was added. The resulting mixture was slowly stirred at RT for 18 hours. The reaction mixture was concentrated in *vacuo.* The crude product was purified by chromatography on silica gel (40 g column, 0-50% EtOAc/isohexane) to afford 1-*tert*-butyl 3-methyl 2-(2-chloro-5-fluoropyrimidin-4-yl)malonate (1.96 g, 5.47 mmol, 46 % yield) as a clear colourless oil; Rt 1.42 mins (HPLC acidic); m/z 305 $(M+H)^+$ $(ES^+)$; $^1$H NMR (400 MHz, DMSO-d6) δ 9.08 - 8.90 (m, 1H), 5.47 - 5.38 (m, 1H), 3.75 (s, 3H), 1.43 (s, 9H).

1-(*tert*-Butyl) 3-methyl 2-(2-chloro-5-methylpyrimidin-4-yl)malonate **INTC3**

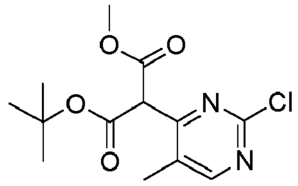

NaH (60 wt % dispersion in mineral oil, 0.466 g, 11.7 mmol) was added portionwise to an ice-cooled, stirred solution of tert-butyl methyl malonate (1.97 mL, 11.7 mmol) in THF (10 mL). The reaction was stirred at RT for 10 mins. 2,4-Dichloro-5-methylpyrimidine (1.0 g, 6.13 mmol) was then added and the resulting mixture was stirred at 70 °C for 2 hrs. The reaction was allowed to cool, partitioned between saturated $NH_4Cl$ (aq, 10 mL) and EtOAc (10 mL), the two phases were separated and the organic layer was passed through a phase separator. The crude product was purified by chromatography on silica gel (12 g column, 0-30% EtOAc/*iso*-hexane) to afford 1-tert-butyl 3-methyl 2-(2-chloro-5-methylpyrimidin-4-yl)malonate (1.40 g, 4.41 mmol, 72 % yield) as a colourless oil; Rt 1.39 mins (HPLC acidic); m/z 201 $(M-Boc+H)^+$ $(ES^+)$; $^1H$ NMR (500 MHz, DMSO-d6) $\delta$ 8.66 (d, J = 0.8 Hz, 1H), 5.38 (s, 1H), 3.74 (s, 3H), 2.19 (d, J = 0.7 Hz, 3H), 1.44 (s, 9H).

**Decarboxylation of chloro-pyrimidines**

Methyl 2-(2-chloropyrimidin-4-yl)acetate **INTC4**

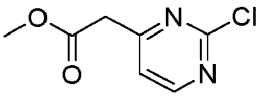

TFA (55.3 mL, 717 mmol) was added dropwise to an ice-cooled, stirred solution of 1-tert-butyl 3-methyl 2-(2-chloropyrimidin-4-yl)malonate **INTC1** (12.1 g, 42.2 mmol) in DCM (50 mL). The reaction was stirred at 25°C for 1 hr and then concentrated *in vacuo.* The residue was dissolved in EtOAc (200 mL), and basified with $NaHCO_3$ (200 mL), the organic layer was isolated and passed through a phase separator, the solvent was removed *in vacuo.* The crude product was purified by chromatography on silica gel (220 g cartridge, 0-50% EtOAc/*iso*-hexane) to afford methyl 2-(2-chloropyrimidin-4-yl)acetate (7.12 g, 37.8 mmol, 90 % yield) as a pale yellow oil. Rt 1.16 mins (HPLC acidic); m/z 187 $(M+H)^+$ $(ES^+)$; 1H NMR (500 MHz, DMSO-d6) $\delta$ 8.76 (d, J = 5.0 Hz, 1H), 7.60 (d, J = 5.0 Hz, 1H), 3.96 (s, 2H), 3.66 (s, 3H).

**Method A: Decarboxylation of chloro-pyrimidines**

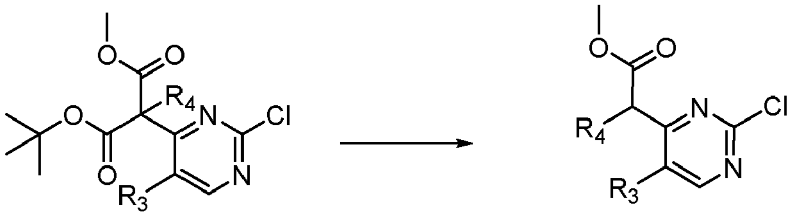

TFA (10 eq) was added dropwise to an ice-cooled, stirred solution of malonate derivative (1 eq) in DCM (15 volumes). The reaction vessel was stirred at RT for 18 hrs and then concentrated. The crude product was purified by normal phase chromatography.

**Table 1:** The following intermediates were made according to Method A.

| INTC# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **INTC5** | methyl 2-(2-chloro-5-fluoropyrimidin-4-yl) acetate 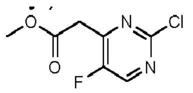 | Method A using INTC2, [HPLC acidic], 205 (0.85). | 8.91 (d, J = 1.4 Hz, 1H), 4.04 (d, J = 1.9 Hz, 2H), 3.68 (s, 3H). |
| **INTC6** | methyl 2-(2-chloro-5-methylpyrimidin-4-yl) acetate 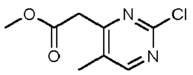 | Method A using INTC3, [HPLC acidic], 201 (0.82). | 8.60 (s, 1H), 3.96 (s, 2H), 3.66 (s, 3H), 2.24 (s, 3H). |

**Alkylation**

Methyl 2-(2-chloropyrimidin-4-yl)-2-methylpropanoate **INTC7**

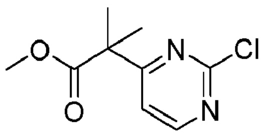

MeI (0.24 mL, 3.89 mmol) was added to a stirred suspension of methyl 2-(2-chloropyrimidin-4-yl)acetate **INTC4** (0.29 g, 1.55 mmol) and $K_2CO_3$ (0.644 g, 4.66 mmol) in acetone (5 mL). The reaction vessel was sealed and stirred at 60 °C for 18 hrs. The reaction mixture was concentrated *in vacuo,* water (40 mL) was added and extracted with DCM (2 x 40 mL). The organic phase was dried (phase separator) and concentrated *in vacuo.* The crude product was purified by chromatography on silica gel (24 g column, 0-50% EtOAc/*iso*-hexane) to afford methyl 2-(2-chloropyrimidin-4-yl)-2-methylpropanoate (0.25 g, 1.11 mmol, 71 % yield) as a clear, pale yellow liquid; Rt 1.70 mins (HPLC acidic); m/z 215 $(M+H)^+$ $(ES^+)$; $^1$H NMR (400 MHz, DMSO-d6) δ 8.78 (d, J = 5.2 Hz, 1H), 7.66 (d, J = 5.2 Hz, 1H), 3.63 (s, 3H), 1.53 (s, 6H).

Methyl 2-(2-chloropyrimidin-4-yl)propanoate **INTC8**

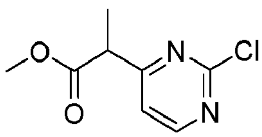

MeI (14.1 mL, 225 mmol) was added to a stirred suspension of methyl 2-(2-chloropyrimidin-4-yl)acetate **INTC4** (10.37 g, 45.0 mmol) and $K_2CO_3$ (31.1 g, 225 mmol) in acetone (150 mL). The reaction mixture was stirred at 60 °C for 40 hrs under $N_2$. The reaction mixture was concentrated *in vacuo,* the resulting mixture diluted in EtOAc and filtered. The inorganic phases were washed with EtOAc and the filtrate concentrated *in vacuo.* The crude product was purified by chromatography on silica gel (220 g column, 0-30% EtOAc/*iso*-hexane) to afford methyl 2-(2-chloropyrimidin-4-yl)propanoate (1.27 g, 5.00 mmol, 11 % yield) as the byproduct; Rt 0.89 mins (UPLC acidic); m/z 201 $(M+H)^+$ $(ES^+)$. No $^1$H-NMR data was recorded.

**Method B: Alkylation**

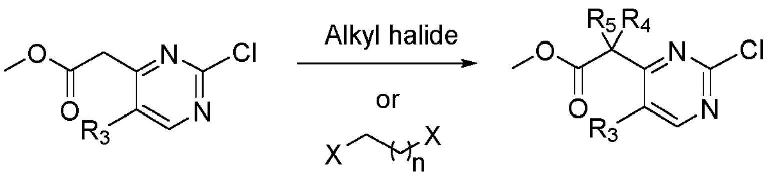


Base (2.5 - 5 eq) was added to an ice-cooled, stirred mixture of methyl 2-(2-chloropyrimidin-4-yl)acetate (1 eq) in appropriate polar aprotic solvent such as DMF or Acetone (10 volumes). After 20 min, alkyl halide (1-5 eq) was added. The reaction vessel was stirred at 0 °C for 30 mins then at RT for 2 hrs. The reaction was quenched with $NH_4Cl$ (aq) or 1M HCl (aq), stirred for 20 mins then extracted with EtOAc. The organic phases were dried (phase separator) and concentrated. The crude product was purified by normal phase chromatography.

**Table 2:** The following intermediates were made according to Method B.

| INTC# | Name/Structure (All examples containing chiral centres are racemates unless stated) | Synthesis Method, [LCMS Method], m/z (M+H)+, (Rt/min) | $^1H$ NMR Chemical Shift Data (DMSO-d6 unless stated) | Base, RX, solvent |
|---|---|---|---|---|
| **INTC9** | methyl 2-(2-chloro-5-fluoropyrimidin-4-yl)-2-methylpropanoate 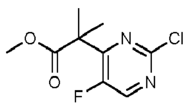 | Method B using INTC5, [UPLC acidic], 233 (1.31). | 8.88 (d, J = 2.5 Hz, 1H), 3.66 (s, 3H), 1.52 (s, 6H). | $K_2CO_3$, MeI, acetone |
| **INTC10** | methyl 2-(2-chloro-5-methylpyrimidin-4-yl)propanoate 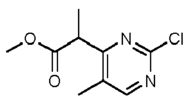 | Method B using INTC6, [UPLC acidic], 215 (1.03). | 8.60 (s, 1H), 4.25 (q, J = 7.0 Hz, 1H), 3.61 (s, 3H), 2.29 (d, J = 0.8 Hz, 3H), 1.40 (d, J = 7.0 Hz, 3H). | $K_2CO_3$, MeI, acetone |
| **INTC11** | methyl 2-(2-chloro-5-methylpyrimidin-4-yl)-2-methylpropanoate 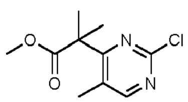 | Method B using INTC6, [UPLC acidic], 228/231 (1.26). | 8.57 (d, J = 0.8 Hz, 1H), 3.66 (s, 3H), 2.13 (d, J = 0.8 Hz, 3H), 1.50 (s, 6H). | $K_2CO_3$, MeI, acetone |
| **INTC12** | methyl 2-(2-chloropyrimidin-4-yl)-2-ethylbutanoate 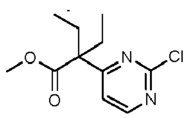 | Method B using INTC4, [UPLC acidic], 243 (1.38). | 8.83 - 8.67 (m, 1H), 7.65 - 7.52 (m, 1H), 3.63 (s, 3H), 2.07 - 1.99 (m, 4H), 0.73 - 0.59 (m, 6H). | NaOH, EtBr, DMF |
| **INTC13** | methyl 1-(2-chloropyrimidin-4-yl) cyclopropane-1-carboxylate 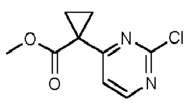 | Method B using INTC4, [UPLC acidic], 213, (1.05). | 8.78 - 8.62 (m, 1H), 7.94 - 7.81 (m, 1H), 3.68 (s, 3H), 1.70 - 1.56 (m, 4H). | NaOH, $BrCH_2CH_2Br$ DMF |

(continued)

| INTC# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) | Base, RX, solvent |
|---|---|---|---|---|
| **INTC14** | methyl 1-(2-chloropyrimidin-4-yl) cyclopentane-1-carboxylate 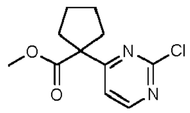 | Method B using INTC4, [UPLC acidic], 241 (1.32). | 8.79 - 8.66 (m, 1H), 7.65 - 7.55 (m, 1H), 3.62 (s, 3H), 2.41 - 2.25 (m, 2H), 2.21 -2.06 (m, 2H), 1.81 -1.57 (m, 4H). | NaOH, Br-(n-Bu)-Br DMF |
| **INTC15** | 1-(tert-butyl) 3-methyl 2-(2-chloropyrimidin-4-yl)-2-ethylmalonate 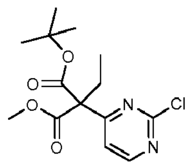 | Method B using INTC1, [UPLC acidic], 315 (1.58). | 8.83 (d, J = 5.3 Hz, 1H), 7.80 (d, J = 5.3 Hz, 1H), 3.73 (s, 3H), 2.29 - 2.14 (m, 2H), 1.40 (s, 9H), 0.82 (t, J = 7.4 Hz, 3H). | NaOH, EtBr, DMF |
| **INTC16** | 1-(tert-butyl) 3-methyl 2-(2-chloropyrimidin-4-yl)-2-(2-methoxyethyl)malonate 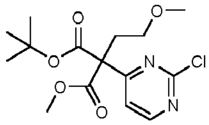 | Method B using INTC1, [UPLC acidic], 345 (1.48). | 8.83 (dd, J = 5.2, 1.0 Hz, 1H), 7.83 (d, J = 5.3 Hz, 1H), 3.72 (s, 3H), 3.31 - 3.24 (m, 2H), 3.11 (s, 3H), 2.47 - 2.40 (m, 2H), 1.39 (s, 9H). | NaOH, $BrCH_2CH_2OMe$, DMF |

**Coupling (Sulfonamidation)**

1-(*Tert*-butyl) 3-methyl 2-(2-(cyclopropanesulfonamido)pyrimidin-4-yl)malonate**INTC17**

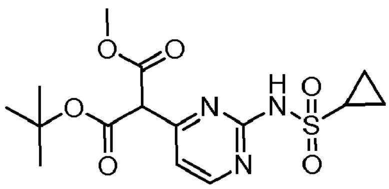

A 20 mL vial was charged with cyclopropanesulfonamide (0.254 g, 2.09 mmol), $Cs_2CO_3$ (1.14 g, 3.49 mmol), 1-tert-butyl 3-methyl 2-(2-chloropyrimidin-4-yl)malonate **INTC1** (0.50 g, 1.74 mmol) and dioxane (2 mL). The mixture was degassed ($N_2$, 5 mins). In a separate 20 mL vial, $[Pd(allyl)Cl]_2$ (16 mg, 0.044 mmol), tBuXPhos (74 mg, 0.174 mmol) and dioxane (1 mL) were stirred under $N_2$ for 5 mins then added to the first vial. The resulting reaction mixture was heated under $N_2$ at 60 °C for 2.5 hrs. The mixture was allowed to cool to RT, diluted with $H_2O$ (2 mL) and then carefully acidified with 1M HCl (aq, 5 mL) until pH 4. The residue was extracted with EtOAc (2 x 20 mL), the organic phase was filtered through a phase separator and the solvent was removed under reduced pressure. The yellow residue was triturated with TBME (10 mL), filtered and washed with TBME (10 mL) to give 1-tert-butyl 3-methyl 2-(2-(cyclopropanesulfonamido)pyrimidin-4-yl)malonate (0.394 g, 1.05 mmol, 60 % yield) as a white solid.; Rt 1.87 mins (HPLC acidic); m/z 372 $(M+H)^+$ $(ES^+)$; $^1$H NMR (400 MHz, DMSO-d6) δ 12.57 (s, 1H), 11.61 (s, 1H), 7.28 - 7.21 (m, 1H), 6.10 (s, 1H), 3.67 (s, 3H), 2.75 - 2.65 (m, 1H), 1.44 (s, 9H), 1.18 - 0.83 (m, 4H). 8:2 mixture of tautomers.

**Method C: Formation of sulfonamides from aromatic halides**

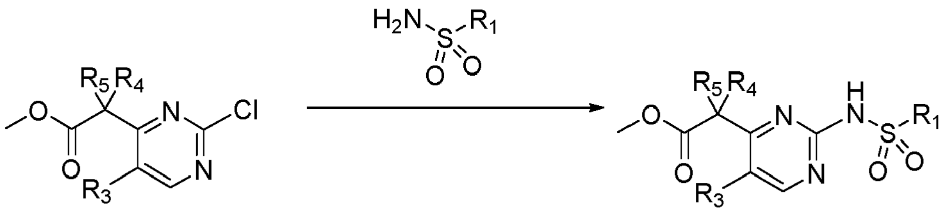

2-Chloropyrimidine intermediate (1 eq), sulfonamide (1.2 eq) and base (2 eq) were dissolved in dioxane (40 volumes). The mixture was degassed ($N_2$, 5 mins) then catalyst (5 mol %) was added. The resulting mixture was heated under nitrogen at 90 °C for 2 hrs. The mixture was filtered, washing with EtOAc or DCM and the resulting filtrate was concentrated. The crude product was purified by normal phase chromatography or trituration using a suitable solvent.

**Table 3:** The following intermediates were made according to Method C.

| INTC# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) | Catalyst, Base, Solvent |
|---|---|---|---|---|
| **INTC18** | methyl 2-(2-(cyclopropanesulfonamido) pyrimidin-4-yl)propanoate 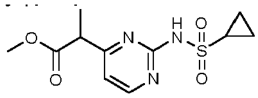 | Method C using INTC8, [UPLC acidic], 286 (0.86). | None recorded. | tBuXPhos Pd G3, $K_2CO_3$, dioxane |
| **INTC19** | methyl 2-methyl-2-(2-(methylsulfonamido) pyrimidin-4-yl)propanoate 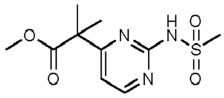 | Method C using INTC7, [HPLC acidic], 274 (1.35). | 11.30 (s, 1H), 8.59 (d, J = 5.3 Hz, 1H), 7.18 (d, J = 5.3 Hz, 1H), 3.62 (s, 3H), 3.35 (s, 3H), 1.50 (s, 6H). | $[Pd(allyl)Cl]_2$ tBuXPhos, $CS_2CO_3$, dioxane |
| **INTC20** | methyl 2-(2-(ethylsulfonamido) pyrimidin-4-yl)-2-methylpropanoate 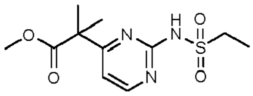 | Method C using INTC7, [UPLC acidic], 288 (0.92). | 11.20 (s, 1H), 8.58 (d, J = 5.3 Hz, 1H), 7.17 (d, J = 5.2 Hz, 1H), 3.62 (s, 3H), 3.53 (q, J = 7.4 Hz, 2H), 1.49 (s, 6H), 1.21 (t, J = 7.3 Hz, 3H). | Pd 174, $CS_2CO_3$, dioxane |
| **INTC21** | methyl 2-(2-(cyclopropanesulfonamido) pyrimidin-4-yl)-2-methylpropanoate 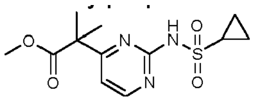 | Method C using INTC7, [HPLC acidic], 300 (1.55). | 11.28 (s, 1H), 8.55 (d, J = 5.3 Hz, 1H), 7.11 (d, J = 5.3 Hz, 1H), 3.61 (s, 3H), 3.22 - 3.11 (m, 1H), 1.49 (s, 6H), 1.14 - 0.93 (m, 4H). | Pd 174, $K_2CO_3$, dioxane |

(continued)

| INTC# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) | Catalyst, Base, Solvent |
|---|---|---|---|---|
| **INTC22** | methyl 2-(2-(cyclopropanesulfonamido)-5-fluoropyrimidin-4-yl)-2-methylpropanoate 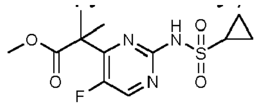 | Method C using INTC9, [UPLC acidic], 318 (1.12). | 11.38 (s, 1H), 8.64 (d, J = 2.8 Hz, 1H), 3.66 (s, 3H), 3.19 (tt, J = 7.9, 4.9 Hz, 1H), 1.52 (s, 6H), 1.14 - 1.03 (m, 4H). | $[Pd(allyl)Cl]_2$ tBuXPhos, $CS_2CO_3$, dioxane |
| INTC23 | methyl 2-(2-(cyclopropanesulfonamido)-5-methylpyrimidin-4-yl)-2-methylpropanoate 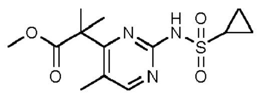 | Method C using INTC11, [UPLC acidic], 313 (1.07). | 11.07 (s, 1H), 8.36 (s, 1H), 3.66 (s, 3H), 3.26 - 3.19 (m, 1H), 2.05 (s, 3H), 1.50 (s, 6H), 1.13 - 1.00 (m, 4H). | $[Pd(allyl)Cl]_2$ tBuXPhos, $CS_2CO_3$, dioxane |
| **INTC24** | methyl 2-(2-(cyclobutanesulfonamido) pyrimidin-4-yl)-2-methylpropanoate 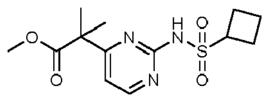 | Method C using INTC7, [HPLC acidic], 314 (1.74). | 11.11 (s, 1H), 8.56 (d, J = 5.3 Hz, 1H), 7.16 (d, J = 5.3 Hz, 1H), 4.55 (p, J = 8.4 Hz, 1H), 3.63 (s, 3H), 2.45 - 2.31 (m, 2H), 2.30 - 2.15 (m, 2H), 2.01 - 1.84 (m, 2H), 1.49 (s, 6H). | $[Pd(allyl)Cl]_2$ tBuXPhos, $CS_2CO_3$, dioxane |
| **INTC25** | methyl 2-(2-(1,1-dimethylethylsulfonamido) pyrimidin-4-yl)-2-methylpropanoate 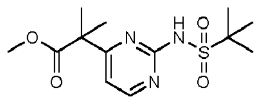 | Method C using INTC7, [UPLC acidic], 316 (1.09). | 10.73 (s, 1H), 8.55 (d, J = 5.3 Hz, 1H), 7.14 (d, J = 5.2 Hz, 1H), 3.59 (s, 3H), 1.48 (s, 6H), 1.37 (s, 9H). | Pd 174, $CS_2CO_3$, dioxane |
| **INTC26** | methyl 2-methyl-2-(2-(1-methylcyclopropanesulfonamido) pyrimidin-4-yl)propanoate 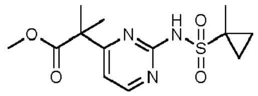 | Method C using INTC7, [HPLC acidic], 314 (1.71). | 11.04 (s, 1H), 8.57 (d, J = 5.2 Hz, 1H), 7.15 (d, J = 5.2 Hz, 1H), 3.60 (s, 3H), 1.55 - 1.45 (m, 8H), 1.43 (s, 3H), 0.89 - 0.83 (m, 2H). | Pd 174, $CS_2CO_3$, dioxane |
| **INTC27** | methyl 2-(2-(cyclopropanesulfonamido) pyrimidin-4-yl)-2-ethylbutanoate 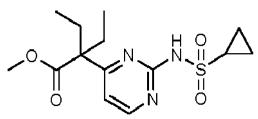 | Method C using INTC12, [UPLC acidic], 328 (1.22). | 11.24 (s, 1H), 8.66 - 8.43 (m, 1H), 7.17 -7.01 (m, 1H), 3.60 (s, 3H), 3.23 - 3.06 (m, 1H), 2.11 - 1.84 (m, 4H), 1.15 - 0.96 (m, 4H), 0.79 - 0.57 (m, 6H). | Pd 174, $CS_2CO_3$, dioxane |

(continued)

| INTC# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) | Catalyst, Base, Solvent |
|---|---|---|---|---|
| **INTC28** | methyl 1-(2-(cyclopropanesulfonamido) pyrimidin-4-yl)cyclopropane-1-carboxylate 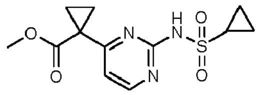 | Method C using INTC13, [UPLC acidic], 298 (0.93). | 11.19 (s, 1H), 8.57 - 8.43 (m, 1H), 7.52 - 7.32 (m, 1H), 3.67 (s, 3H), 3.20 - 3.08 (m, 1H), 1.68 - 1.52 (m, 4H), 1.15 - 0.98 (m, 4H). | Pd 174, $CS_2CO_3$, dioxane |
| **INTC29** | methyl 1-(2-(cyclopropanesulfonamido) pyrimidin-4-yl)cyclopentane-1-carboxylate 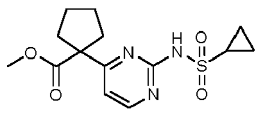 | Method C using INTC14, [UPLC acidic], 326 (1.17). | 11.23 (s, 1H), 8.59 - 8.45 (m, 1H), 7.17 - 7.05 (m, 1H), 3.61 (s, 3H), 3.25 - 3.12 (m, 1H), 2.40 - 2.24 (m, 2H), 2.21 - 2.08 (m, 2H), 1.73 - 1.59 (m, 4H), 1.18 - 0.96 (m, 4H). | Pd 174, $CS_2CO_3$, dioxane |
| **INTC30** | 1-(tert-butyl) 3-methyl 2-(2-(cyclopropanesulfonamido) pyrimidin-4-yl)-2-ethylmalonate 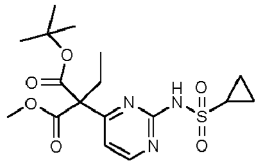 | Method C using INTC15, [UPLC acidic], 400 (1.40). | 11.30 (s, 1H), 8.62 (d, J = 5.3 Hz, 1H), 7.35 (d, J = 5.3 Hz, 1H), 3.71 (s, 3H), 3.21 - 3.10 (m, 1H), 2.30 - 2.10 (m, 2H), 1.41 (s, 9H), 1.18 - 0.97 (m, 4H), 0.83 (t, J = 7.4 Hz, 3H). | Pd 174, $CS_2CO_3$, dioxane |
| **INTC31** | 1-(tert-butyl) 3-methyl 2-(2-(cyclopropanesulfonamido) pyrimidin-4-yl)-2-(2-methoxyethyl) malonate 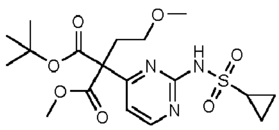 | Method C using INTC16, [UPLC acidic], 430 (1.31). | 11.31 (s, 1H), 8.63 (d, J = 5.3 Hz, 1H), 7.38 (d, J = 5.3 Hz, 1H), 3.70 (s, 3H), 3.32 - 3.24 (m, 2H), 3.20 - 3.14 (m, 1H), 3.13 (s, 3H), 2.49 - 2.32 (m, 2H), 1.39 (s, 9H), 1.15 - 0.98 (m, 4H). | Pd 174, $CS_2CO_3$, dioxane |
| **INTC32** | 1-(tert-butyl) 3-methyl 2-(2-(cyclopropanesulfonamido)-5-fluoropyrimidin-4-yl)malonate 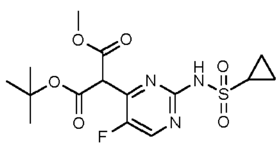 | Method C using INTC2, [UPLC acidic], 390 (1.27). | 11.47 (s, 1H), 8.81 - 8.72 (m, 1H), 5.31 - 5.20 (m, 1H), 3.75 (s, 3H), 3.20 - 3.12 (m, 1H), 1.43 (s, 9H), 1.17 - 0.99 (m, 4H). | Pd 174, $K_2CO_3$, dioxane |

**Decarboxylation**

Methyl 2-(2-(cyclopropanesulfonamido)pyrimidin-4-yl)acetate 2,2,2-trifluoroacetate **INTC33**

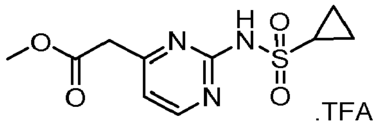

.TFA

TFA (1 mL, 13.0 mmol) was added dropwise to a stirred, ice-cooled solution of 1-*tert*-butyl 3-methyl 2-(2-(cyclopropanesulfonamido)pyrimidin-4-yl)malonate **INTC17** (0.27 g, 0.73 mmol) in DCM (2 mL). The reaction vessel was stirred at RT for 2 hrs and concentrated *in vacuo* to afford methyl 2-(2-(cyclopropanesulfonamido)pyrimidin-4-yl)acetate 2,2,2-trifluoroacetate (0.29 g, 0.68 mmol, 93 % yield) as a yellow solid; Rt 0.79 mins (HPLC basic); m/z 272 $(M+H)^+$ $(ES^+)$; $^1H$ NMR (400 MHz, DMSO-$d_6$) δ 12.19 (s, 1H), 11.39 - 10.93 (m, 2H), 8.57 (d, J = 5.1 Hz, 1H), 7.13 (d, J = 5.1 Hz, 1H), 7.00 (dd, J = 7.6, 5.1 Hz, 1H), 5.95 - 5.87 (m, 1H), 4.92 (s, 1H), 3.84 (s, 2H), 3.65 (s, 3H), 3.61 (s, 3H), 3.29 - 3.10 (m, 1H), 2.72 - 2.60 (m, 1H), 1.17 - 0.85 (m, 8H).1:1 mixture of tautomers.

**Method D: Decarboxylation of pyrimidines bearing sulfonamides**

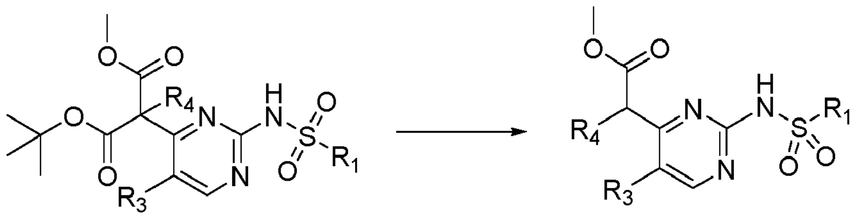

TFA (10 eq) was added dropwise to an ice-cooled, stirred solution of malonate derivative (1 eq) in DCM (15 volumes). The reaction vessel was stirred at RT for 18 hrs and then concentrated. The crude product was purified by normal phase chromatography.

**Table 4:** The following intermediates were made according to Method D.

| INTC# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **INTC34** | methyl 2-(2-(cyclopropanesulfonamido)-5-fluoropyrimidin-4-yl)acetate 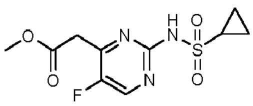 | Method D using INTC32, [HPLC acidic], 290 (0.83). | 11.37 (s, 1H), 8.76 - 8.63 (m, 1H), 3.98 - 3.89 (m, 2H), 3.67 (s, 3H), 3.26 - 3.12 (m, 1H), 1.16 - 0.98 (m, 4H). |
| **INTC35** | methyl 2-(2-(cyclopropanesulfonamido) pyrimidin-4-yl)butanoate 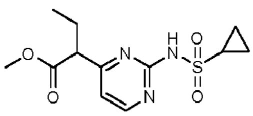 | Method D using INTC30, [UPLC acidic], 300 (0.99). | 11.26 (s, 1H), 8.57 (d, J = 5.1 Hz, 1H), 7.13 (d, J = 5.1 Hz, 1H), 3.74 (t, J = 7.5 Hz, 1H), 3.62 (s, 3H), 3.26 - 3.15 (m, 1H), 2.06 - 1.93 (m, 1H), 1.92 - 1.77 (m, 1H), 1.19 - 0.96 (m, 4H), 0.85 (t, J = 7.4 Hz, 3H). |
| **INTC36** | methyl 2-(2-(cyclopropanesulfonamido) pyrimidin-4-yl)-4-methoxybutanoate 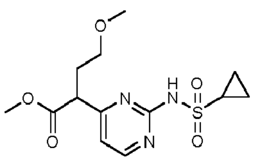 | Method D using INTC31, [UPLC basic], 330 (0.60). | 11.27 (s, 1H), 8.57 (d, J = 5.1 Hz, 1H), 7.13 (d, J = 5.1 Hz, 1H), 3.91 (t, J = 7.4 Hz, 1H), 3.62 (s, 3H), 3.33 - 3.20 (m, 3H), 3.19 (s, 3H), 2.28 - 2.18 (m, 1H), 2.11 - 2.01 (m, 1H), 1.16 - 0.99 (m, 4H). |

**Hydrolysis**

Potassium 2-(2-(cyclopropanesulfonamido)pyrimidin-4-yl)butanoate **INTC37**

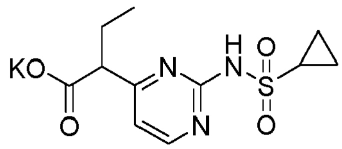

A solution of methyl 2-(2-(cyclopropanesulfonamido)pyrimidin-4-yl)butanoate **INTC35** (2.4 g, 7.22 mmol) in THF (80 mL) was treated with TMSOK (2.26 g, 15.9 mmol). The reaction mixture was allowed to stir at RT for 18 hrs. The resulting suspension was concentrated in *vacuo* to afford potassium 2-(2-(cyclopropanesulfonamido)pyrimidin-4-yl)butanoate (3 g, 6.49 mmol, 90 % yield) as a pale yellow solid; Rt 0.19 mins (UPLC basic); m/z 286 $(M+H)^+$ $(ES^+)$, ionises as free acid; $^1$H NMR (500 MHz, DMSO-$d_6$) δ 7.97 (d, *J* = 5.0 Hz, 1H), 6.35 (d, *J* = 5.0 Hz, 1H), 3.01 (tt, *J* = 8.2, 5.0 Hz, 1H), 2.88 (dd, *J* = 8.0, 6.9 Hz, 1H), 1.88 - 1.79 (m, 1H), 1.61 - 1.50 (m, 1H), 0.84 - 0.74 (m, 5H), 0.65 - 0.55 (m, 2H), NH proton not observed.

Potassium 2-(2-(cyclopropanesulfonamido)pyrimidin-4-yl)-4-methoxybutanoate **INTC38**

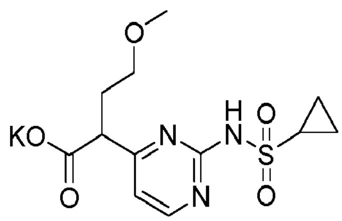

A solution of methyl 2-(2-(cyclopropanesulfonamido)pyrimidin-4-yl)-4-methoxybutanoate **INTC36** (0.23 g, 0.69 mmol) in THF (5 mL) was treated with TMSOK (0.22 g, 1.54 mmol). The reaction mixture was allowed to stir at RT for 18 hrs. The resulting suspension was quenched with MeOH (2 mL) then concentrated *in vacuo* to afford potassium 2-(2-(cyclopropanesulfonamido)pyrimidin-4-yl)-4-methoxybutanoate (0.33 g, 0.65 mmol, 94 % yield) as a pale red solid; Rt 0.14 mins (UPLC basic); m/z 316 $(M+H)^+$ $(ES^+)$, ionises as free acid; $^1$H NMR (500 MHz, DMSO-$d_6$) δ 8.03 (dd, *J* = 5.0, 2.9 Hz, 1H, minor), 7.97 (d, *J* = 5.0 Hz, 1H, major), 6.59 (d, *J* = 5.1 Hz, 1H, minor), 6.33 (d, *J* = 5.0 Hz, 1H, major), 3.28 - 3.13 (m, 7H), 3.12 - 2.96 (m, 1H), 2.22 - 1.99 (m, 1H), 1.83 - 1.77 (m, 1H), 0.84 - 0.75 (m, 2H), 0.63 - 0.57 (m, 2H). Mixture of tautomers.

Potassium 2-(2-(cyclopropanesulfonamido)pyrimidin-4-yl)acetate **INTC39**

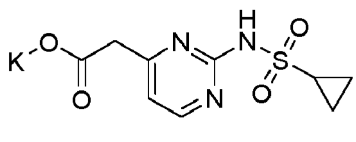

A solution of methyl 2-(2-(cyclopropanesulfonamido)pyrimidin-4-yl)acetate (600 mg, 2.212 mmol) **INTC33** in THF (12 mL) was treated with TMSOK (624 mg, 4.87 mmol). The reaction mixture was allowed to stir at RT for 18 hrs. The resulting suspension was concentrated in *vacuo* to afford potassium 2-(2-(cyclopropanesulfonamido)pyrimidin-4-yl)acetate (1.069 g, 2.208 mmol, quantitative yield) as a pale yellow solid. Rt 0.14 min (UPLC acidic); m/z 258 $(M+H)^+$ $(ES^+)$, ionises as free acid. $^1$H NMR (500 MHz, DMSO-d6) δ 7.97 (d, J = 4.9 Hz, 1H), 6.33 (d, J = 4.9 Hz, 1H), 2.99 - 2.92 (m, 1H), 0.82 - 0.76 (m, 2H), 0.66 - 0.57 (m, 2H). $CH_2$ and NH signals not observed.

2-(2-(Cyclopropanesulfonamido)-5-fluoropyrimidin-4-yl)acetic acid **INTC40**

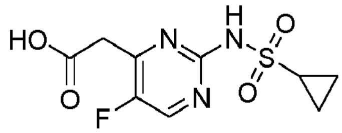

LiOH (0.105 g, 4.37 mmol) was added to a solution of methyl 2-(2-(cyclopropanesulfonamido)-5-fluoropyrimidin-4-yl)acetate (0.49 g, 1.457 mmol) **INTC34** in MeOH (5 mL) and water (2 mL) and stirred at RT for 18 hrs. The reaction mixture was concentrated in *vacuo* and the crude product was purified by chromatography by RP Flash C18 (40 g column, 0-50% MeCN/Water 0.1% Formic Acid) to afford 2-(2-(cyclopropanesulfonamido)-5-fluoropyrimidin-4-yl)acetic acid (0.44 g, 0.991 mmol, 68 % yield) as a yellow solid. Rt 0.65 mins (UPLC acidic); m/z 276 $(M+H)^+$ $(ES^+)$; $^1H$ NMR (400 MHz, DMSO-d6) δ 8.49 (s, 1H), 4.15 (s, 1H), 2.97 - 2.85 (m, 1H), 2.22 - 2.15 (m, 2H), 0.86 - 0.69 (m, 2H), 0.69 - 0.52 (m, 2H), $CO_2H$ not observed.

4-(6-Ethoxypyrazin-2-yl)-N-(4-methoxybenzyl)aniline **INTC41**

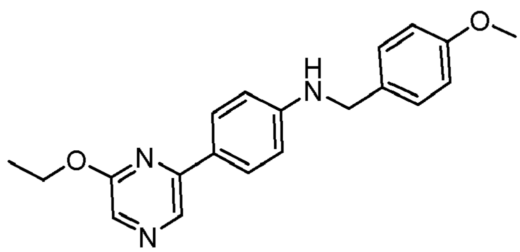

Sodium triacetoxyborohydride (0.148 g, 0.697 mmol) was added to a solution of 4-(6-ethoxypyrazin-2-yl)aniline **INTC18** (0.1 g, 0.465 mmol) and 4-methoxybenzaldehyde (0.085 mL, 0.697 mmol) in DCM (3 mL). The reaction was stirred at RT for 16 hrs. To the reaction was added saturated $NaHCO_3$ (aq) (20 mL), the aqueous extracted with DCM (3 x 20 mL) and the combined organic layers were concentrated under reduced pressure. The crude product was purified by chromatography on silica gel (24 g cartridge, 0-50% EtOAc/isohexane) to afford 4-(6-ethoxypyrazin-2-yl)-N-(4-methoxybenzyl)aniline (0.174 g, 0.467 mmol, quantitative yield) as a white solid. Rt 1.68 min (UPLC, basic); m/z 336 $(M+H)^+$ $(ES^+)$. $^1H$ NMR (500 MHz, DMSO-d6) δ 8.59 (s, 1H), 8.00 (s, 1H), 7.88 - 7.83 (m, 2H), 7.32 - 7.26 (m, 2H), 6.93 - 6.86 (m, 2H), 6.71 (t, J = 6.0 Hz, 1H), 6.70 - 6.65 (m, 2H), 4.43 (q, J = 7.0 Hz, 2H), 4.27 (d, J = 5.8 Hz, 2H), 3.73 (s, 3H), 1.37 (t, J = 7.0 Hz, 3H).

*tert*-Butyl 3-((4-(6-ethoxypyrazin-2-yl)phenyl)(4-methoxybenzyl)amino)-3-oxopropanoate **INTC42**

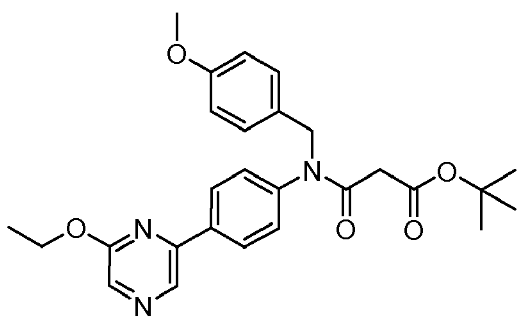

HATU (0.289 g, 0.760 mmol) was added to a stirred solution of 4-(6-ethoxypyrazin-2-yl)-N-(4-methoxybenzyl)aniline **INTC41** (0.17 g, 0.507 mmol), 3-(tert-butoxy)-3-oxopropanoic acid (0.1 mL, 0.649 mmol) and TEA (0.21 mL, 1.507 mmol) in DCM (5 mL). The resulting reaction was stirred at ambient temperature for 1 h. The reaction mixture was diluted with water (20 mL) and extracted with DCM (3 x 20 mL). The combined organic extracts were dried (phase separator) and the solvent removed under reduced pressure. The crude product was purified by chromatography on silica gel (24 g column, 0-100% EtOAc/*iso*-hexane) to afford tert-butyl 3-((4-(6-ethoxypyrazin-2-yl)phenyl)(4-methoxybenzyl)amino)-3-oxopropanoate (0.2 g, 0.377 mmol, 74 % yield) as a clear, colourless gum. Rt 1.74 min (UPLC, basic); m/z 478 $(M+H)^+$ $(ES^+)$; $^1H$ NMR (500 MHz, DMSO-d6) $\delta$ 8.81 (s, 1H), 8.25 (s, 1H), 8.15 - 8.09 (m, 2H), 7.34 - 7.27 (m, 2H), 7.18 - 7.11 (m, 2H), 6.88 - 6.82 (m, 2H), 4.87 (s, 2H), 4.47 (q, J = 7.0 Hz, 2H), 3.72 (s, 3H), 3.20 (s, 2H), 1.39 (t, J = 7.1 Hz, 3H), 1.35 (s, 9H).

*tert*-Butyl 2-(2-chloropyrimidin-4-yl)-3-((4-(6-ethoxypyrazin-2-yl)phenyl)(4-methoxybenzyl)amino)-3-oxopropanoate **INTC43**

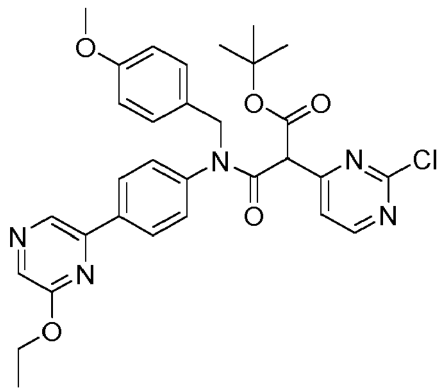

NaH (60% dispersion in mineral oil) (0.034 g, 0.838 mmol) was added to a stirred solution of tert-butyl 3-((4-(6-ethoxypyrazin-2-yl)phenyl)(4-methoxybenzyl)amino)-3-oxopropanoate **INTC42** (0.2 g, 0.419 mmol) in THF (4 mL, 0.419 mmol). The reaction was stirred at RT for 10 min then 2,4-dichloropyrimidine (0.087 g, 0.586 mmol) was added. The resulting mixture was stirred at 70°C for 2 hrs under $N_2$. The reaction mixture was quenched with brine (20 mL). The aqueous phase was extracted with DCM (3 x 50 mL), dried (phase separator) and concentrated *in vacuo.* The crude product was purified by chromatography on silica gel (24 g column, 0-100% EtOAc/*iso*-hexane) to afford tert-butyl 2-(2-chloropyrimidin-4-yl)-3-((4-(6-ethoxypyrazin-2-yl)phenyl)(4-methoxybenzyl)amino)-3-oxopropanoate (0.13 g, 0.189 mmol, 45 % yield) as a clear, colourless gum. Rt 1.86 min (UPLC, basic); m/z 591 $(M+H)^+$ $(ES^+)$; $^1H$ NMR (500 MHz, DMSO-d6) $\delta$ 8.81 (s, 1H), 8.77 (d, J = 5.1 Hz, 1H), 8.26 (s, 1H), 8.15 - 8.12 (m, 2H), 7.66 - 7.63 (m, 1H), 7.22 - 7.16 (m, 4H), 6.90 - 6.85 (m, 2H), 5.02 (d, J = 14.7 Hz, 1H), 4.84 (s, 1H), 4.74 (d, J = 14.6 Hz, 1H), 4.47 (q, J = 7.0 Hz, 2H), 3.73 (s, 3H), 1.39 (t, J = 7.1 Hz, 3H), 1.37 (s, 9H).

2-(2-Chloropyrimidin-4-yl)-N-(4-(6-ethoxypyrazin-2-yl)phenyl)-N-(4-methoxybenzyl)acetamide **INTC44**

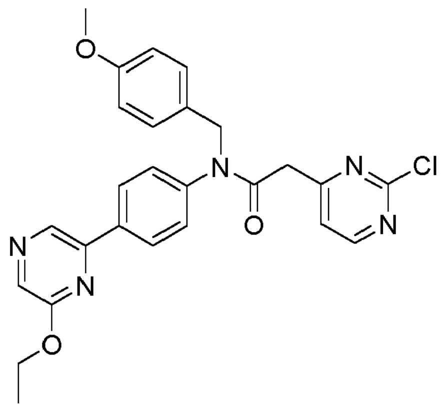

TFA (0.234 mL, 3.03 mmol) was added dropwise to an ice-cooled, stirred solution of tert-butyl 2-(2-chloropyrimidin-4-yl)-3-((4-(6-ethoxypyrazin-2-yl)phenyl)(4-methoxybenzyl)amino)-3-oxopropanoate **INTC43** (0.13 g, 0.189 mmol) in DCM (20 mL). The reaction vessel was stirred at 25°C for 7 hrs and then carefully basified with NaHCO3 (20 mL). The aqueous phase was extracted with DCM (2 x 20 mL), dried (phase separator) and the solvent was removed under reduced pressure. The crude product was purified by chromatography on silica gel (24 g column, 0-100% EtOAc/*iso*-hexane) to afford 2-(2-chloropyrimidin-4-yl)-N-(4-(6-ethoxypyrazin-2-yl)phenyl)-N-(4-methoxybenzyl)acetamide (0.05 g, 0.092 mmol, 49 % yield) as a clear, colourless gum. Rt 1.62 min (UPLC, acidic); m/z 490 $(M+H)^+$ $(ES^+)$; $^1H$ NMR (500 MHz, DMSO-d6) δ 8.81 (s, 1H), 8.66 (d, J = 5.0 Hz, 1H), 8.25 (s, 1H), 8.14 - 8.10 (m, 2H), 7.45 (d, J = 5.1 Hz, 1H), 7.39 - 7.34 (m, 2H), 7.17 (d, J = 8.2 Hz, 2H), 6.89 - 6.82 (m, 2H), 4.89 (s, 2H), 4.46 (q, J = 7.1 Hz, 2H), 3.76 (s, 2H), 3.72 (s, 3H), 1.39 (t, J = 7.0 Hz, 3H).

2-(2-Chloropyrimidin-4-yl)-N-(4-(6-ethoxypyrazin-2-yl)phenyl)-N-(4-methoxybenzyl)butanamide **INTC45**

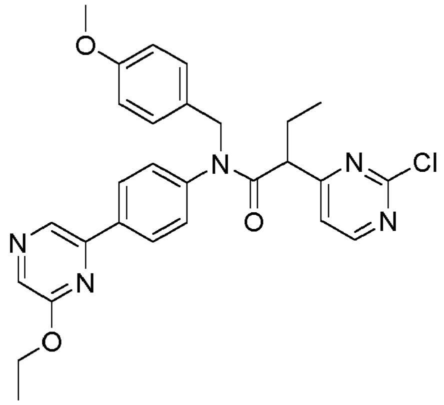

Iodoethane (0.410 mL, 5.10 mmol) was added to a stirred mixture of 2-(2-chloropyrimidin-4-yl)-N-(4-(6-ethoxypyrazin-2-yl)phenyl)-N-(4-methoxybenzyl)acetamide **INTC44** (0.5 g, 1.021 mmol) and potassium carbonate (0.705 g, 5.10 mmol) in acetone (5 mL). The reaction vessel was heated to 60°C and stirred for 18 hrs under $N_2$. The reaction mixture was concentrated, diluted in water (40 mL) and extracted into DCM (3 x 40 mL). The organics were combined, dried (phase separator) and concentrated *in vacuo.* The crude product was purified by chromatography on silica gel (40 g column, 0-100% EtOAc/*iso*-hexane) to afford 2-(2-chloropyrimidin-4-yl)-N-(4-(6-ethoxypyrazin-2-yl)phenyl)-N-(4-methoxybenzyl)butanamide (0.31 g, 0.539 mmol, 53 % yield) as a clear pale yellow gum. Rt 2.73 min (HPLC, acidic); m/z 519 $(M+H)^+$ $(ES^+)$; $^1H$ NMR (500 MHz, DMSO-d6) δ 8.80 (s, 1H), 8.63 (d, J = 5.1 Hz, 1H), 8.25 (s, 1H), 8.07 (d, J = 8.3 Hz, 2H), 7.32 (d, J = 5.2 Hz, 1H), 7.17 - 7.08 (m, 4H), 6.88 - 6.81 (m, 2H), 4.92 (d, J = 14.7 Hz, 1H), 4.82 (d, J = 14.6 Hz, 1H), 4.50 - 4.43 (m, 2H), 3.73 - 3.69 (m, 4H), 2.09 - 2.00 (m, 1H), 1.83 - 1.74 (m, 1H), 1.39 (t, J = 7.0 Hz, 3H), 0.82 (t, J = 7.3 Hz, 3H).

2-(2-(Cyclopropanesulfonamido)pyrimidin-4-yl)-N-(4-(6-ethoxypyrazin-2-yl)phenyl)-N-(4-methoxybenzyl)butanamide **INTC46**

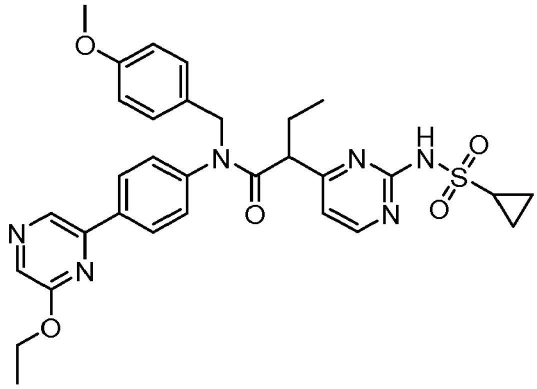

A 20 mL vial was charged with cyclopropanesulfonamide (0.078 g, 0.646 mmol), $Cs_2CO_3$ (0.351 g, 1.077 mmol), 2-(2-chloropyrimidin-4-yl)-N-(4-(6-ethoxypyrazin-2-yl)phenyl)-N-(4-methoxybenzyl)butanamide **INTC45** (0.31 g, 0.539 mmol) and dioxane (10 mL). The mixture was sparged with $N_2$ for 5 min. Pd-174 (0.012 g, 0.016 mmol) was added and the mixture was then heated at 80°C for 1 hr and then at 100°C for 7 hrs. Further cyclopropanesulfonamide (0.078 g, 0.646 mmol) and Pd-174 (0.012 g, 0.016 mmol) was added and the mixture heated at 100°C for a further 3 h. The reaction mixture was quenched with saturated $NH_4Cl$ (*aq*) (40 mL), extracted into DCM (3 x 40 mL), dried (phase separator) and concentrated *in vacuo.* The crude product was purified by chromatography on silica gel (40 g cartridge, 0-100% EtOAc/*iso*-hexane) to afford 2-(2-(cyclopropanesulfonamido)pyrimidin-4-yl)-N-(4-(6-ethoxypyrazin-2-yl)phenyl)-N-(4-methoxybenzyl)butanamide (0.18 g, 0.269 mmol, 50 % yield) as a thick yellow gum. Rt 1.65 min (UPLC, acidic); 603.6 $(M+H)^+$ $(ES^+)$; $^1H$ NMR (500 MHz, DMSO-d6) δ 11.22 (s, 1H), 8.80 (s, 1H), 8.49 - 8.42 (m, 1H), 8.25 (s, 1H), 8.08 (d, J = 8.1 Hz, 2H), 7.22 (d, J = 8.1 Hz, 2H), 7.14 - 7.08 (m, 2H), 6.96 - 6.89 (m, 1H), 6.87 - 6.81 (m, 2H), 4.99 (d, J = 14.6 Hz, 1H), 4.76 (d, J = 14.7 Hz, 1H), 4.47 (q, J = 7.0 Hz, 2H), 3.71 (s, 3H), 3.65 (t, J = 7.3 Hz, 1H), 3.22 - 3.13 (m, 1H), 2.10 - 1.96 (m, 1H), 1.83 - 1.72 (m, 1H), 1.39 (t, J = 7.0 Hz, 3H), 1.12 - 1.06 (m, 2H), 0.93 - 0.87 (m, 2H), 0.83 (t, J = 7.3 Hz, 3H).

*tert*-Butyl 2-(2-(cyclopropanesulfonamido)pyrimidin-4-yl)-3-((4-(6-ethoxypyrazin-2-yl)phenyl)(4-methoxybenzyl)amino)-3-oxopropanoate **INTC47**

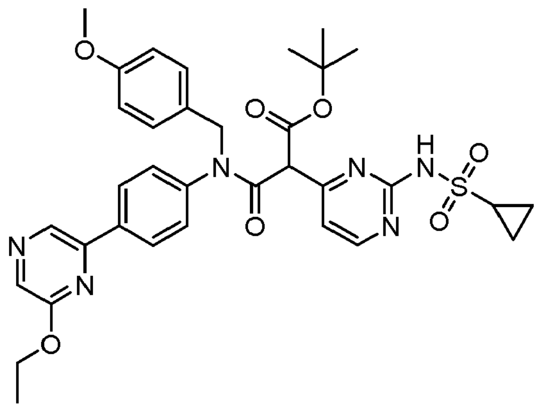

A 20 mL vial was charged with cyclopropanesulfonamide (0.074 g, 0.610 mmol), $Cs_2CO_3$ (0.331 g, 1.017 mmol), *tert*-butyl 2-(2-chloropyrimidin-4-yl)-3-((4-(6-ethoxypyrazin-2-yl)phenyl)(4-methoxybenzyl)amino)-3-oxopropanoate **INTC43** (0.3 g, 0.508 mmol) and dioxane (10 mL). The mixture was sparged with $N_2$ for 5 min. In a separate 20 mL vial was added $[Pd(allyl)Cl]_2$ (4.68 mg, 0.013 mmol), tBuXPhos (0.022 g, 0.051 mmol) and dioxane (2 mL). The mixture was stirred under $N_2$ for 5 min then added to the first mixture. The resulting mixture was heated under $N_2$ at 60°C for 4 hrs. Further cyclopropanesulfonamide (0.074 g, 0.610 mmol) was added followed by Pd-174 (11.00 mg, 0.015 mmol). The mixture was then heated at 80°C for 1 hr. The reaction mixture was quenched with saturated $NH_4Cl$ (aq) (40 mL), extracted into DCM (3 x 20 mL), dried (phase separator) and concentrated *in vacuo.* The crude product was purified by chromatography on silica gel (40 g column, 0-100% EtOAc/*iso*-hexane) to afford tert-butyl 2-(2-(cyclopropanesulfonamido)pyrimidin-4-yl)-3-((4-(6-ethoxypyrazin-2-yl)phenyl)(4-methoxybenzyl)amino)-3-oxopropanoate (0.1 g, 0.130 mmol,

26 % yield) as a white solid. Rt 2.51 min (HPLC, basic); m/z 675 $(M+H)^+$ $(ES^+)$.

**Amine intermediate preparation**

**Method E: Suzuki coupling of halo anilines with heteroaromatic boronates**

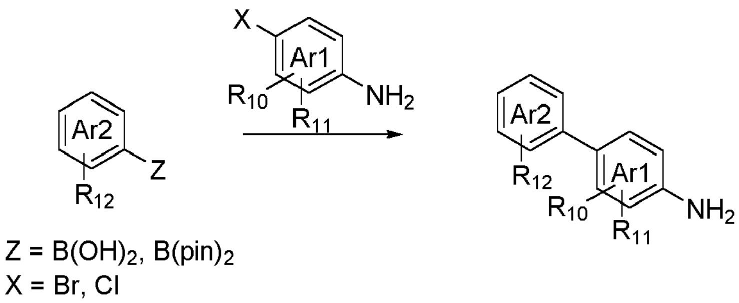


A solution of Ar1-X (1 eq) and Ar2-Z (1 eq) in solvent (3 volumes) and base (2.5 eq) was degassed ($N_2$, 5 min) and heated to 40 °C whereupon Pd catalyst (3 mol %) was added and the reaction mixture further degassed ($N_2$, 5 min) before being heated to 90 °C for 90 mins. The reaction mixture was allowed to cool to RT. In general, the desired compound is purified by column chromatography.

**Method F: Suzuki coupling of heteroaromatic halides with aniline boronates**

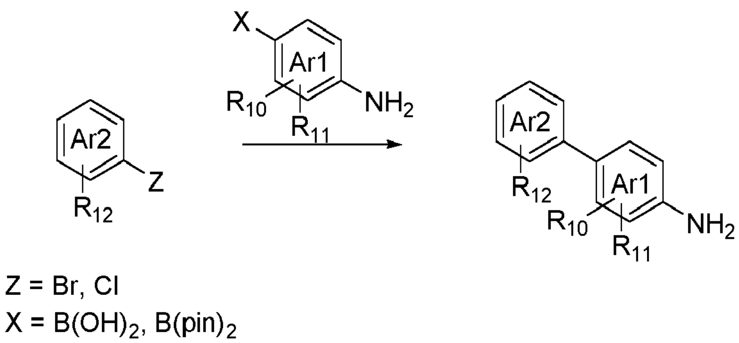


Pd catalyst (5 mol %) was added to a degassed ($N_2$, 5 mins) solution of Ar1-X (1 eq), Ar2-Z (1 eq) and base (3 eq, 6.85 mmol) in solvent (3 volumes). The solution was then degassed further ($N_2$, 5 mins) and then heated to 90 °C for 2 hrs then allowed to cool to RT. In general, the desired compound was purified by column chromatography.

**Method G: Telescoped boronate formation and Suzuki coupling**

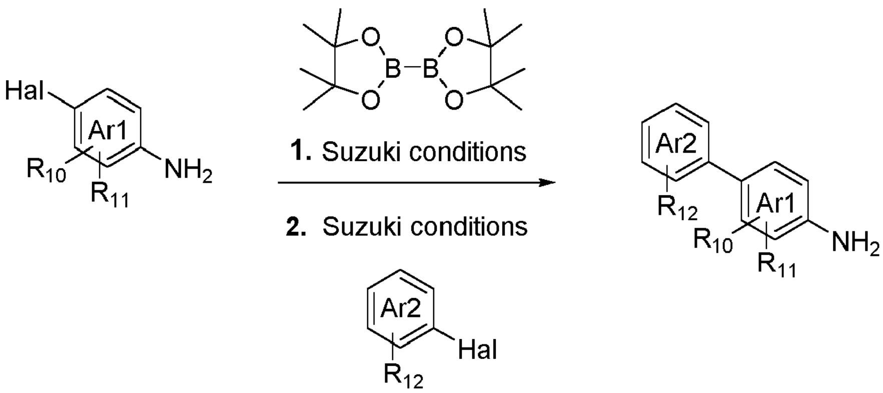


Bispin (1.1 eq) and KOAc (4 eq) were added to Ar1-Hal (1 eq) in dioxane (5 volumes). The reaction was heated to 60 °C and degassed ($N_2$, 5 mins). $PdCl_2$(dppf) (5 mol %) was added to the reaction mixture and the temperature was increased to 90 °C for 1 hr. The reaction mixture was then cooled to RT and a solution of Ar2-Hal (1 eq) in dioxane (3 volumes) was added, followed by a solution of $K_2CO_3$ (4 eq) in water (2 volumes). The temperature was then increased to 90 °C for 18 hrs. The reaction was cooled to RT, an aqueous work up was performed and the crude compound was purified by normal phase chromatography.

**Anilines**

**Table 5:** The following intermediates were made according to Methods E, F or G.

| INTD# | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| **INTD1** | 4-(6-methoxypyrazin-2-yl) aniline 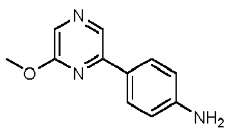 | Method F, [HPLC basic], 202, (1.63). | 8.61 (s, 1H), 8.04 (s, 1H), 7.94 - 7.75 (m, 2H), 6.75 - 6.54 (m, 2H), 5.59 (s, 2H), 3.98 (s, 3H). | $Pd(PPh_3)_4$, $NaHCO_3$, MeCN |
| **INTD2** | 5-(6-(trifluoromethyl) pyrazin-2-yl)pyridin-2-amine 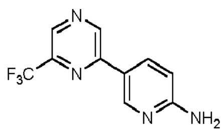 | Method F, [UPLC acidic], 241, (0.52). | 9.52 - 9.41 (m, 1H), 8.95 (t, J = 0.6 Hz, 1H), 8.81 (dd, J = 2.5, 0.8 Hz, 1H), 8.16 (dd, J = 2.5 Hz, 1H), 6.66 (s, 2H), 6.59 (dd, J = 8.8, 0.8 Hz, 1H). | $Pd(PPh_3)_4$, $NaHCO_3$, EtOH, toluene |
| **INTD3** | 3-(4-aminophenyl)-5-(difluoro-I3-methoxy) pyridine 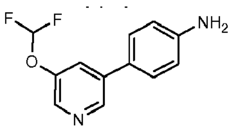 | Method E, [UPLC basic], 237, (1.05). | 8.74 - 8.66 (m, 1H), 8.36 - 8.24 (m, 1H), 7.81 - 7.74 (m, 1H), 7.53 - 7.43 (m, 2H), 7.61 - 7.15 (m, 1H), 6.72 - 6.58 (m, 2H), 5.44 (s, 2H). | $PdCl_2$(dppf), $K_2CO_3$, dioxane |

(continued)

| INTD# | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| **INTD4** | 4-(5-ethoxypyridin-3-yl) aniline 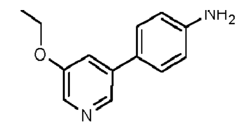 | Method F, [UPLC basic], 215, (1.05). | 8.36 (d, J = 1.9 Hz, 1H), 8.11 (d, J = 2.7 Hz, 1H), 7.48 - 7.39 (m, 3H), 6.70 - 6.62 (m, 2H), 5.34 (s, 2H), 4.17 (q, J = 6.9 Hz, 2H), 1.36 (t, J = 7.0 Hz, 3H). | $PdCl_2$(dppf), $K_2CO_3$, dioxane |
| **INTD5** | 5-(4-aminophenyl) nicotinonitrile 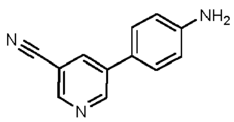 | Method E, [UPLC basic], 196, (0.89). | 9.08 (d, J = 2.4 Hz, 1H), 8.91 - 8.79 (m, 1H), 8.49 - 8.43 (m, 1H), 7.58 - 7.48 (m, 2H), 6.73 - 6.57 (m, 2H), 5.49 (d, J = 7.0 Hz, 2H). | $PdCl_2$(dppf), $K_2CO_3$, dioxane |
| **INTD6** | 4-(5-fluoropyridin-3-yl) aniline 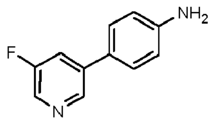 | Method E, [HPLC basic], 189, (1.53). | 8.69 (t, J = 2.0 Hz, 1H), 8.39 (d, J = 2.7 Hz, 1H), 7.87 (ddd, J = 10.9, 2.7, 1.9 Hz, 1H), 7.53 - 7.45 (m, 2H), 6.71 - 6.63 (m, 2H), 5.44 (s, 2H). | $PdCl_2$(dppf), $K_2CO_3$, dioxane |
| **INTD7** | 4-(5-(trifluoromethyl) pyridin-3-yl)aniline 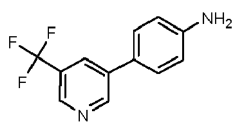 | Method E, [UPLC basic], 239, (1.21). | 9.15 - 9.06 (m, 1H), 8.82 - 8.72 (m, 1H), 8.30 - 8.23 (m, 1H), 7.60 - 7.51 (m, 2H), 6.74 - 6.64 (m, 2H), 5.50 (s, 2H). | $PdCl_2$(dppf), $K_2CO_3$, dioxane |
| **INTD8** | 4-(5-chloropyridin-3-yl) aniline 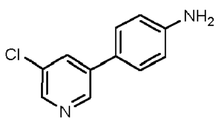 | Method E, [UPLC basic], 205, (1.11). | 8.79 - 8.74 (m, 1H), 8.49 - 8.39 (m, 1H), 8.10 - 7.98 (m, 1H), 7.54 - 7.42 (m, 2H), 6.72 - 6.61 (m, 2H), 5.45 (s, 2H). | $PdCl_2$(dppf), $K_2CO_3$, dioxane |
| **INTD9** | 4-(6-methylpyridin-3-yl) aniline 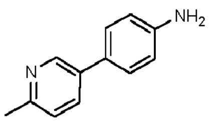 | Method E, [HPLC basic], 185, (1.43). | 8.63 (dd, J = 2.5, 0.8 Hz, 1H), 7.80 (dd, J = 8.1, 2.5 Hz, 1H), 7.40 - 7.32 (m, 2H), 7.23 (dt, J = 8.1, 0.7 Hz, 1H), 6.70 - 6.58 (m, 2H), 5.27 (s, 2H), 2.46 (s, 3H). | Pd 170, $K_3PO_4$, dioxane |
| **INTD10** | 4-(5-methylpyridin-3-yl) aniline 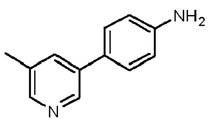 | Method E, [HPLC basic], 185, (1.47). | 8.57 (d, J = 2.3 Hz, 1H), 8.25 (dd, J = 2.0, 0.8 Hz, 1H), 7.74 (td, J = 2.2, 0.9 Hz, 1H), 7.43-7.30 (m, 2H), 6.72 - 6.52 (m, 2H), 5.31 (s, 2H), 2.33 (d, J = 0.8 Hz, 3H). | Pd 170, $K_3PO_4$, dioxane |

(continued)

| INTD# | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| **INTD11** | 4-(5-isopropoxypyridin-3-yl)aniline 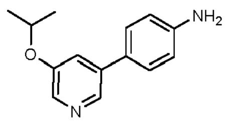 | Method F, [UPLC basic], 229, (1.15). | 8.41 - 8.30 (m, 1H), 8.13 - 8.05 (m, 1H), 7.48 - 7.35 (m, 3H), 6.72 - 6.59 (m, 2H), 5.34 (s, 2H), 4.86 - 4.69 (m, 1H), 1.37 - 1.23 (m, 6H). | $PdCl_2$(dppf), $K_2CO_3$, dioxane |
| **INTD12** | 2-fluoro-4-(5-isopropoxypyridin-3-yl) aniline 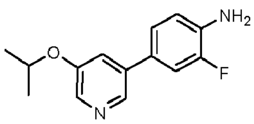 | Method F, [UPLC basic], 247, (1.25). | 8.42 - 8.34 (m, 1H), 8.17 - 8.06 (m, 1H), 7.53 - 7.42 (m, 2H), 7.35 - 7.28 (m, 1H), 6.89 - 6.80 (m, 1H), 5.39 (s, 2H), 4.90 - 4.75 (m, 1H), 1.35 - 1.26 (m, 6H). | $PdCl_2$(dppf), $K_2CO_3$, dioxane |
| **INTD13** | 4-(5-chloropyridin-3-yl)-2-fluoroaniline 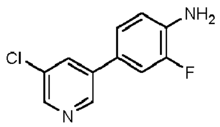 | Method E, [HPLC basic], 223, (1.9). | 8.80 (d, J = 2.0 Hz, 1H), 8.48 (d, J = 2.2 Hz, 1H), 8.14 (t, J = 2.2 Hz, 1H), 7.54 (dd, J = 13.0, 2.1 Hz, 1H), 7.38 (dd, J = 8.3, 2.1 Hz, 1H), 6.86 (dd, J = 9.5, 8.3 Hz, 1H), 5.51 (s, 2H). | $PdCl_2$(dppf), $K_2CO_3$, dioxane |
| **INTD14** | 2-fluoro-4-(5-(2,2,2-trifluoro ethoxy)pyridin-3-yl)aniline 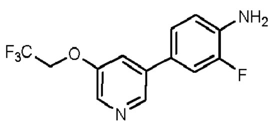 | Method F, [UPLC basic], 287, (1.26). | 8.61 - 8.50 (m, 1H), 8.31 - 8.22 (m, 1H), 7.77 - 7.64 (m, 1H), 7.59 - 7.47 (m, 1H), 7.42 - 7.33 (m, 1H), 6.93 - 6.75 (m, 1H), 5.44 (s, 2H), 5.01 - 4.88 (m, 2H). | $PdCl_2$(dppf), $K_3PO_4$, dioxane |
| **INTD15** | 4-(5-(2,2,2-trifluoro ethoxy) pyridin-3-yl)aniline 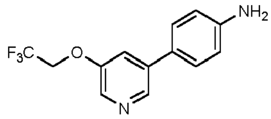 | Method F, [UPLC basic], 269, (1.18). | 8.55 - 8.41 (m, 1H), 8.29 - 8.13 (m, 1H), 7.69 - 7.61 (m, 1H), 7.53 - 7.43 (m, 2H), 6.73 - 6.61 (m, 2H), 5.38 (s, 2H), 5.02 - 4.82 (m, 2H). | $PdCl_2$(dppf), $K_3PO_4$, dioxane |
| **INTD16** | 5'-ethoxy-[3,3'-bipyridin]-6-amine 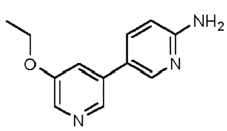 | Method F, [UPLC basic], 216, (0.88). | 8.39 (d, J = 1.9 Hz, 1H), 8.32 (d, J = 2.5 Hz, 1H), 8.17 (d, J = 2.7 Hz, 1H), 7.78 (dd, J = 8.6, 2.6 Hz, 1H), 7.51 (dd, J = 2.7, 1.9 Hz, 1H), 6.54 (dd, J = 8.6, 0.8 Hz, 1H), 6.18 (s, 2H), 4.18 (q, J = 7.0 Hz, 2H), 1.37 (t, J = 7.0 Hz, 3H). | $PdCl_2$(dppf), $K_2CO_3$, dioxane |

(continued)

| INTD# | Name/Structure | Synthesis Method, [LCMS Method], m/z (M+H)$^+$, (Rt/min) | $^1$H NMR Chemical Shift Data (DMSO-d6 unless stated) | Catalyst, Base, solvent |
|---|---|---|---|---|
| **INTD17** | 5'-(2,2,2-trifluoroethoxy)-[3,3'-bipyridin]-6-amine 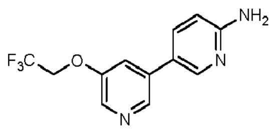 | Method F, [UPLC basic], 270, (1.00). | 8.57 - 8.46 (m, 1H), 8.41 - 8.34 (m, 1H), 8.32 - 8.23 (m, 1H), 7.88 - 7.78 (m, 1H), 7.76 - 7.68 (m, 1H), 6.60 - 6.50 (m, 1H), 6.22 (s, 2H), 5.03 - 4.87 (m, 2H). | $PdCl_2$(dppf), $K_3PO_4$, dioxane |
| **INTD18** | 4-(6-ethoxypyrazin-2-yl) aniline 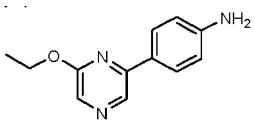 | Method F, [HPLC basic], 216, (1.78). | 8.59 (s, 1H), 8.00 (s, 1H), 7.86 - 7.75 (m, 2H), 6.69 - 6.59 (m, 2H), 5.59 (s, 2H), 4.43 (q, J = 7.0 Hz, 2H), 1.38 (t, J = 7.0 Hz, 3H). | $Pd(PPh_3)_4$, $NaHCO_3$, MeCN |
| **INTD19** | 4-(6-(trifluoromethyl) pyrazin-2-yl)aniline 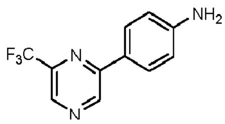 | Method F, [HPLC acidic], 240, (1.92). | 9.40 (s, 1H), 8.86 (s, 1H), 7.99 - 7.89 (m, 2H), 6.74 - 6.66 (m, 2H), 5.83 (s, 2H). | $Pd(PPh_3)_4$, $NaHCO_3$, MeCN |
| **INTD20** | 4-(6-isopropoxypyrazin-2-yl)aniline 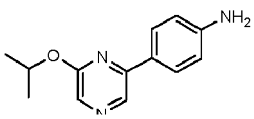 | Method F, [UPLC basic], 230, (1.31). | 8.57 (s, 1H), 7.95 (s, 1H), 7.87 - 7.73 (m, 2H), 6.73 - 6.56 (m, 2H), 5.59 (s, 2H), 5.45 - 5.27 (m, 1H), 1.47 - 1.25 (m, 6H). | $PdCl_2$(dppf), $K_3PO_4$, dioxane |
| **INTD21** | 4-(6-cyclopropoxypyrazin-2-yl)aniline 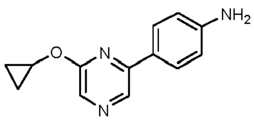 | Method F, [HPLC basic], 228, (1.83). | 8.19 - 8.02 (m, 1H), 7.58 - 7.41 (m, 1H), 7.40 - 7.22 (m, 2H), 6.21 - 5.99 (m, 2H), 5.09-4.99 (m, 2H), 3.86-3.73 (m, 1H), 0.37 - 0.09 (m, 4H). | $PdCl_2$(dppf), $K_3PO_4$, dioxane |
| **INTD22** | 4-(6-chloropyrazin-2-yl) aniline 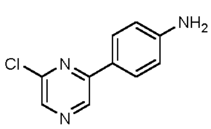 | Method F, [HPLC basic], 206, (1.75). | 9.06 (d, J = 0.6 Hz, 1H), 8.47 (d, J = 0.6 Hz, 1H), 7.89 - 7.81 (m, 2H), 6.73 - 6.62 (m, 2H), 5.79 (s, 2H). | $PdCl_2$(dppf), $K_3PO_4$, dioxane |
| **INTD23** | 2-fluoro-4-(pyrazin-2-yl) aniline 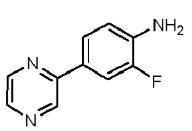 | Method F, [UPLC basic], 190, (0.84). | 9.12 (d, J = 1.6 Hz, 1H), 8.58 (dd, J = 2.6, 1.5 Hz, 1H), 8.44 (d, J = 2.5 Hz, 1H), 7.86 - 7.71 (m, 2H), 6.87 (dd, J = 9.3, 8.4 Hz, 1H), 5.67 (s, 2H). | $PdCl_2$(dppf), $K_2CO_3$, dioxane |

(continued)

| INTD# | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| **INTD24** | 4-(6-ethoxypyrazin-2-yl)-2-fluoroaniline 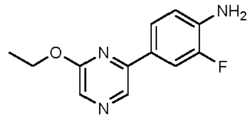 | Method F, [UPLC basic], 234, (1.31). | 8.66 (s, 1H), 8.06 (s, 1H), 7.84 - 7.63 (m, 2H), 6.93 - 6.75 (m, 1H), 5.65 (s, 2H), 4.54 - 4.34 (m, 2H), 1.47 - 1.29 (m, 3H). | $PdCl_2$(dppf), $K_2CO_3$, dioxane |
| **INTD25** | 2-fluoro-4-(6-(trifluoromethyl) pyrazin-2-yl)aniline 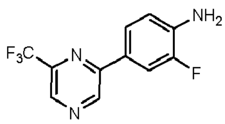 | Method F, [HPLC basic], 258, (2.13). | 9.46 (s, 1H), 8.92 (s, 1H), 7.88 (dd, J = 13.1, 2.1 Hz, 1H), 7.83 (dd, J = 8.4, 2.1 Hz, 1H), 6.90 (t, J = 8.8 Hz, 1H), 5.90 (s, 2H). | $PdCl_2$(dppf), $K_3PO_4$, dioxane |
| **INTD26** | 4-(6-chloropyrazin-2-yl)-2-methylaniline 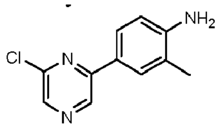 | Method F, [UPLC basic], 220, (1.24). | 9.06 (s, 1H), 8.46 (s, 1H), 7.84 - 7.65 (m, 2H), 6.78 - 6.63 (m, 1H), 5.55 (s, 2H), 2.14 (s, 3H). | $PdCl_2$(dppf), $K_3PO_4$, dioxane |
| **INTD27** | 4-(6-ethoxypyrazin-2-yl)-2-methylaniline 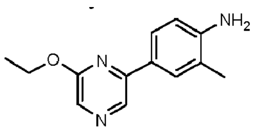 | Method F, [UPLC basic], 230, (1.27). | 8.60 (s, 1H), 8.00 (s, 1H), 7.80 - 7.63 (m, 2H), 6.75 - 6.61 (m, 1H), 5.35 (s, 2H), 4.51 - 4.33 (m, 2H), 2.13 (s, 3H), 1.45 - 1.31 (m, 3H). | $PdCl_2$(dppf), $K_3PO_4$, dioxane |
| **INTD28** | 2-fluoro-4-(6-(2,2,2-trifluoroethoxy)pyrazin-2-yl) aniline 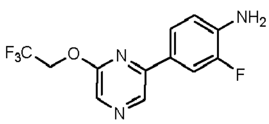 | Method F, [UPLC basic], 288, (1.38). | 8.82 (s, 1H), 8.24 (s, 1H), 7.94 - 7.85 (m, 1H), 7.81 - 7.74 (m, 1H), 6.92 - 6.82 (m, 1H), 5.72 (s, 2H), 5.23 - 5.09 (m, 2H). | $PdCl_2$(dppf), $K_3PO_4$, dioxane |
| **INTD29** | 4-(6-(2,2,2-trifluoroethoxy) pyrazin-2-yl)aniline 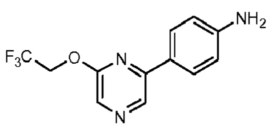 | Method F, [UPLC basic], 270, (1.31). | 8.76 (s, 1H), 8.18 (s, 1H), 7.95 - 7.84 (m, 2H), 6.73 - 6.58 (m, 2H), 5.67 (s, 2H), 5.22 - 5.04 (m, 2H). | $PdCl_2$(dppf), $K_3PO_4$, dioxane |
| **INTD30** | 5-(6-cyclopropoxypyrazin-2-yl)pyridin-2-amine 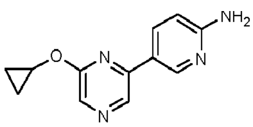 | Method F, [UPLC basic], 229, (0.98). | 8.81 - 8.64 (m, 2H), 8.18 - 8.00 (m, 2H), 6.55 (dd, J = 8.8, 0.8 Hz, 1H), 6.44 (s, 2H), 4.37 (tt, J = 6.2, 3.0 Hz, 1H), 0.94 - 0.69 (m, 4H). | $Pd(PPh_3)_4$, $Na_2CO_3$, Toluene, EtOH |

(continued)

| INTD# | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| **INTD31** | 5-(6-ethoxypyrazin-2-yl)-3-fluoropyridin-2-amine 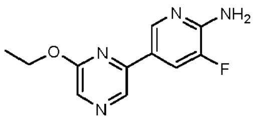 | Method F, [HPLC basic], 235, (1.73). | 8.72 (s, 1H), 8.62 - 8.57 (m, 1H), 8.12 (s, 1H), 8.04 (dd, J = 12.6, 1.9 Hz, 1H), 6.73 (s, 2H), 4.46 (q, J = 7.0 Hz, 2H), 1.39 (t, J = 7.0 Hz, 3H). | $PdCl_2$(dppf), $K_2CO_3$, dioxane |
| **INTD32** | 5-(6-isopropoxypyrazin-2-yl)pyridin-2-amine 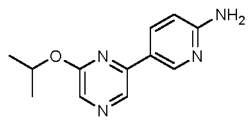 | Method F, [UPLC basic], 232, (1.09). | 8.69 (d, J = 2.4 Hz, 1H), 8.62 (s, 1H), 8.07 (dd, J = 8.7, 2.5 Hz, 1H), 8.02 (s, 1H), 6.55 (dd, J = 8.7, 0.8 Hz, 1H), 6.42 (s, 2H), 5.44 - 5.28 (m, 1H), 1.37 (d, J = 6.1 Hz, 6H). | $Pd(PPh_3)_4$, $Na_2CO_3$, dioxane |
| **INTD33** | 5-(6-ethoxypyrazin-2-yl) pyridin-2-amine 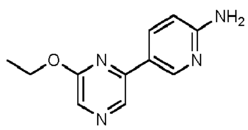 | Method F, [UPLC, basic], 217, (0.98). | 8.70 (dd, J = 2.5, 0.8 Hz, 1H), 8.64 (s, 1H), 8.10 - 8.06 (m, 2H), 6.54 (dd, J = 8.7, 0.8 Hz, 1H), 6.41 (s, 2H), 4.43 (q, J = 7.0 Hz, 2H), 1.38 (t, J = 7.0 Hz, 3H). | $PdCl_2$(dppf), $Cs_2CO_3$, dioxane |
| **INTD34** | 2-methyl-4-(6-(trifluoromethyl) pyrazin-2-yl)aniline 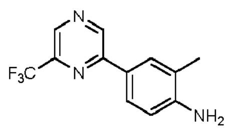 | Method F, [HPLC acidic], 254 (2.14). | 9.40 (s, 1H), 8.85 (s, 1H), 7.89 - 7.78 (m, 2H), 6.73 (d, J = 8.3 Hz, 1H), 5.60 (s, 2H), 2.15 (s, 3H). | $PdCl_2$(dppf), $K_3PO_4$, dioxane |
| **INTD35** | 2-fluoro-4-(6-isopropoxypyrazin-2-yl) aniline 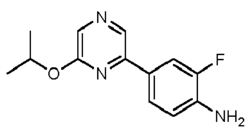 | Method F, [UPLC basic], 248 (1.41). | 8.64 (s, 1H), 8.01 (s, 1H), 7.82 - 7.63 (m, 2H), 6.92 - 6.80 (m, 1H), 5.67 (s, 2H), 5.45 - 5.28 (m, 1H), 1.46 - 1.24 (m, 6H). | $PdCl_2$(dppf), $K_2CO_3$, dioxane |
| **INTD36** | 4-(6-ethoxypyrazin-2-yl)-5-fluoro-2-methylaniline 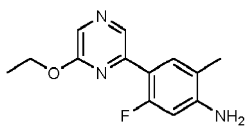 | Method G, [UPLC basic], 248 (1.36). | 8.45 (d, J = 2.2 Hz, 1H), 8.06 (s, 1H), 7.63 (d, J = 8.8 Hz, 1H), 6.47 (d, J = 14.2 Hz, 1H), 5.68 (s, 2H), 4.43 (q, J = 7.0 Hz, 2H), 2.09 (s, 3H), 1.39 (t, J = 7.0 Hz, 3H). | (i)$PdCl_2$(dppf) , KOAc, dioxane, then (ii)$PdCl_2$ (dppf ), $K_2CO_3$, dioxane |

(continued)

| INTD# | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| **INTD37** | 4-(6-ethoxypyrazin-2-yl)-5-fluoro-2-methoxyaniline 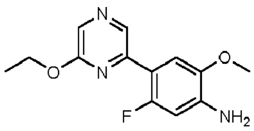 | Method G, [UPLC basic], 264 (1.37). | 8.50 (d, J = 2.0 Hz, 1H), 8.08 (s, 1H), 7.43 (d, J = 7.2 Hz, 1H), 6.51 (d, J = 13.5 Hz, 1H), 5.61 (s, 2H), 4.45 (q, J = 7.1 Hz, 2H), 3.83 (s, 3H), 1.40 (t, J = 7.0 Hz, 3H). | (i)$PdCl_2$(dppf) , KOAc, dioxane, then (ii)$PdCl_2$ (dppf ), $K_2CO_3$, dioxane |
| **INTD38** | 4-(6-ethoxypyrazin-2-yl)-2,3-dimethylaniline 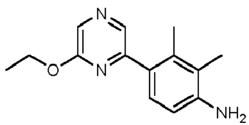 | Method G, [UPLC basic], 244 (1.28). | 8.18 (s, 1H), 8.10 (s, 1H), 7.02 (d, J = 8.3 Hz, 1H), 6.60 (d, J = 8.2 Hz, 1H), 5.11 (s, 2H), 4.36 (q, J = 7.0 Hz, 2H), 2.22 (s, 3H), 2.04 (s, 3H), 1.35 (t, J = 7.0 Hz, 3H). | (i)$PdCl_2$(dppf) , KOAc, dioxane, then (ii)$PdCl_2$ (dppf $K_2CO_3$, dioxane |
| **INTD39** | 4-(6-ethoxypyrazin-2-yl)-2-fluoro-5-methylaniline 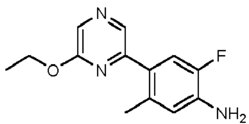 | Method G, [HPLC acidic], 248 (2.41). | 8.31 (s, 1H), 8.13 (s, 1H), 7.24 (d, J = 12.5 Hz, 1H), 6.68 (d, J = 9.1 Hz, 1H), 5.44 (s, 2H), 4.38 (q, J = 7.0 Hz, 2H), 2.32 (s, 3H), 1.36 (t, J = 7.0 Hz, 3H). | (i)$PdCl_2$(dppf) , KOAc, dioxane, then (ii)$PdCl_2$ (dppf ), $K_2CO_3$, dioxane |
| **INTD40** | 4-(6-ethoxypyrazin-2-yl)-2,5-dimethylaniline 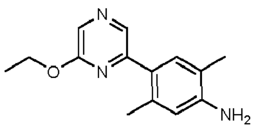 | Method G, [HPLC acidic], 244 (2.22). | 8.26 (s, 1H), 8.07 (s, 1H), 7.15 (s, 1H), 6.53 (s, 1H), 5.13 2H), 4.36 (q, J = 7.0 Hz, 2H), 2.31 (s, 3H), 2.07 (s, 3H), 1.36 (t, J = 7.1 Hz, 3H). | (i)$PdCl_2$(dppf) , KOAc, dioxane, then (ii)$PdCl_2$ (dppf ), $K_2CO_3$, dioxane |
| **INTD41** | 4-(6-ethoxypyrazin-2-yl)-2,6-difluoroaniline 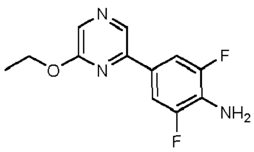 | Method G, [HPLC acidic], 252 (2.41). | 8.73 (s, 1H), 8.12 (s, 1H), 7.74 (dd, J = 8.0, 2.5 Hz, 2H), 5.73 (s, 2H), 4.46 (q, J = 7.0 Hz, 2H), 1.39 (t, J = 7.1 Hz, 3H). | (i)$PdCl_2$(dppf) , KOAc, dioxane, then (ii)$PdCl_2$ (dppf ), $K_2CO_3$, dioxane |
| **INTD42** | 3'-ethoxy-[1,1'-biphenyl]-4-amine 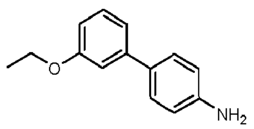 | Method F, [UPLC basic], 214 (1.39). | 7.37 - 7.32 (m, 2H), 7.29 - 7.24 (m, 1H), 7.09 (ddd, J = 7.7, 1.7, 0.9 Hz, 1H), 7.06 - 7.01 (m, 1H), 6.77 (ddd, J = 8.1, 2.5, 1.0 Hz, 1H), 6.63 (d, J = 8.3 Hz, 2H), 5.22 (s, 2H), 4.07 (q, J = 7.0 Hz, 2H), 1.34 (t, J = 6.9 Hz, 3H) | $PdCl_2$(dppf), $K_2CO_3$, dioxane |
| **INTD43** | 4-(pyrazin-2-yl)aniline 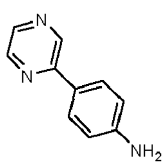 | Method F, [UPLC acidic], 172 (0.56). | 9.05 (d, J = 1.6 Hz, 1H), 8.54 (dd, J = 2.5, 1.6 Hz, 1H), 8.38 (d, J = 2.5 Hz, 1H), 7.93 - 7.77 (m, 2H), 6.72 - 6.62 (m, 2H), 5.61 (s, 2H). | $PdCl_2$(dppf), $K_2CO_3$, dioxane |

(continued)

| INTD# | Name/Structure | Synthesis Method, [LCMS Method], m/z $(M+H)^+$, (Rt/min) | $^1$H NMR Chemical Shift Data (DMSO-d6 unless stated) | Catalyst, Base, solvent |
|---|---|---|---|---|
| **INTD44** | 5'-(trifluoromethyl)-[3,3'-bipyridinl-6-amine 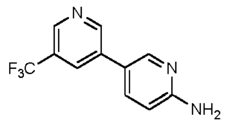 | Method F, [HPLC basic], 240 (1.59). | 9.13 (d, J = 2.2 Hz, 1H), 8.84 (dd, J = 2.1, 1.0 Hz, 1H), 8.44 (dd, J = 2.6, 0.8 Hz, 1H), 8.40 - 8.33 (m, 1H), 7.90 (dd, J = 8.7, 2.6 Hz, 1H), 6.57 (dd, J = 8.7, 0.8 Hz, 1H), 6.32 (s, 2H). | $PdCl_2$(dppf), $K_3PO_4$, dioxane |
| **INTD45** | 4-(5-methoxypyridin-3-yl) aniline 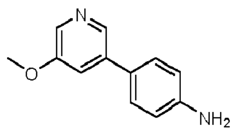 | Method E [UPLC acidic], 201 (0.91). | 8.38 (d, J = 1.9 Hz, 1H), 8.13 (d, J = 2.8 Hz, 1H), 7.49 - 7.40 (m, 3H), 6.71 - 6.62 (m, 2H), 5.35 (s, 2H), 3.88 (s, 3H). | $PdCl_2$(dppf), $K_2CO_3$, dioxane |
| **INTD46** | 2-fluoro-4-(6-methoxypyrazin-2-yl) aniline 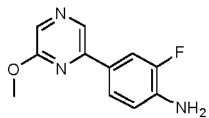 | Method F, [UPLC basic], 220 (1.20). | 8.68 (s, 1H), 8.09 (s, 1H), 7.81 (dd, J = 13.1, 2.0 Hz, 1H), 7.74 (dd, J = 8.4, 2.0 Hz, 1H), 6.90 - 6.82 (m, 1H), 5.66 (s, 2H), 3.99 (s, 3H). | $PdCl_2$(dppf), $K_2CO_3$, dioxane |
| **INTD47** | 4-(6-ethoxypyrazin-2-yl)-2-(trifluoromethoxy)aniline 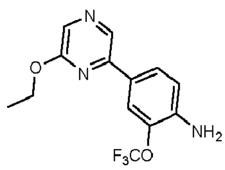 | Method F, [HPLC basic], 246 (1.99). | 8.67 (s, 1H), 8.08 (s, 1H), 7.93 - 7.78 (m, 2H), 6.91 (d, J = 8.5 Hz, 1H), 5.91 (s, 2H), 4.43 (q, J = 7.0 Hz, 2H), 1.39 (t, J = 7.0 Hz, 3H). | PdCl2(dppf), $K_3PO_4$, dioxane |
| **INTD48** | 4-(6-ethoxypyrazin-2-yl)-2-methoxyaniline 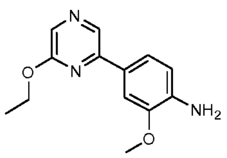 | Method F, [HPLC basic], 300 (2.38). | 8.67 (s, 1H), 8.02 (s, 1H), 7.62 - 7.46 (m, 2H), 6.72 (d, J = 8.0 Hz, 1H), 5.24 (s, 2H), 4.45 (q, J = 7.0 Hz, 2H), 3.87 (s, 3H), 1.39 (t, J = 7.0 Hz, 3H). | $PdCl_2$(dppf), $K_3PO_4$, dioxane |
| **INTD49** | 2-chloro-4-(6-ethoxypyrazin-2-yl)aniline 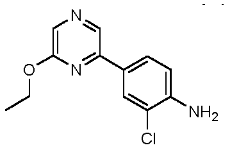 | Method F, [HPLC basic], 250 (2.26). | 8.66 (d, J = 0.5 Hz, 1H), 8.07 (d, J = 0.5 Hz, 1H), 7.99 (d, J = 2.1 Hz, 1H), 7.83 (dd, J = 8.5, 2.1 Hz, 1H), 6.89 (d, J = 8.5 Hz, 1H), 5.85 (s, 2H), 4.44 (q, J = 7.0 Hz, 2H), 1.39 (t, J = 7.0 Hz, 3H). | $PdCl_2$(dppf), $K_3PO_4$, dioxane |

(continued)

| INTD# | Name/Structure | Synthesis Method, [LCMS Method], m/z $(M+H)^+$, (Rt/min) | $^1$H NMR Chemical Shift Data (DMSO-d6 unless stated) | Catalyst, Base, solvent |
|---|---|---|---|---|
| **INTD50** | 2-fluoro-4-(pyridin-3-yl) aniline 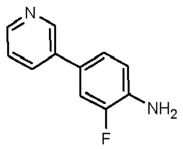 | Method E, [UPLC basic], 189 (0.90). | 8.82 (dd, J = 2.5, 0.9 Hz, 1H), 8.45 (dd, J = 4.7, 1.6 Hz, 1H), 7.97 (ddd, J = 8.0, 2.5, 1.6 Hz, 1H), 7.45 (dd, J = 13.0, 2.1 Hz, 1H), 7.40 (ddd, J = 8.0, 4.7, 0.9 Hz, 1H), 7.31 (dd, J = 8.2, 2.1 Hz, 1H), 6.86 (dd, J = 9.5, 8.3 Hz, 1H), 5.39 (s, 2H). | $PdCl_2$(dppf), $K_2CO_3$, dioxane |
| **INTD51** | 2-amino-5-(6-ethoxypyrazin-2-yl) benzonitrile 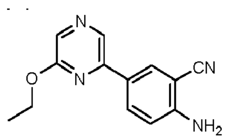 | Method G, [HPLC basic], 241 (1.99). | 8.70 (s, 1H), 8.19 (d, J = 2.2 Hz, 1H), 8.13 - 8.06 (m, 2H), 6.91 (d, J = 8.9 Hz, 1H), 6.52 (s, 2H), 4.45 (q, J = 7.0 Hz, 2H), 1.39 (t, J = 7.0 Hz, 3H). | (i) $PdCl_2$(dppf), KOAc, dioxane, then (ii)$PdCl_2$ (dppf ), $K_2CO_3$, dioxane |

**Preparation of Examples**

**Amide formation**

**Method 1: Amide coupling using HATU**

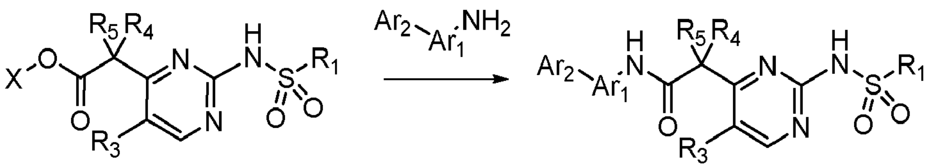

To a stirred suspension of the acid or the potassium salt (1 eq, X= H or K) and DIPEA (6 eq) in DMF (15 vol) the aniline (1 eq) and HATU (1.5 eq) were added. The reaction was stirred at RT for 18 hrs then concentrated *in vacuo.* MeOH and 2M NaOH (aq) were added. The mixture was stirred for 30 min then concentrated *in vacuo.* The aqueous phase acidified to pH 6 with 1M HCl (aq) and the product extracted into DCM. The organics were combined, dried (phase separator) and concentrated *in vacuo.*

The crude product was purified by reverse or normal phase chromatography or a combination of both.

*N*-(4-(5-chloropyridin-3-yl)phenyl)-2-(2-(cyclopropanesulfonamido)pyrimidin-4-yl)butanamide **P1**

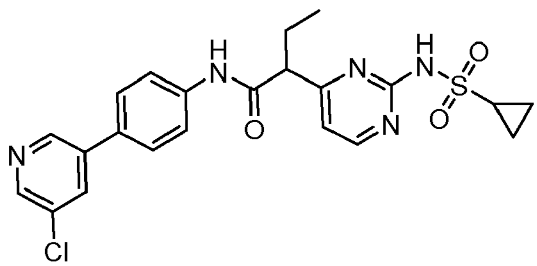

4-(5-chloropyridin-3-yl)aniline **INTD8** (0.117 g, 0.573 mmol) and HATU (0.327 g, 0.859 mmol) were added to a stirred suspension of potassium 2-(2-(cyclopropanesulfonamido)pyrimidin-4-yl)butanoate **INTC37** (0.265 g, 0.573 mmol) and DIPEA (0.60 mL, 3.44 mmol) in DMF (6 mL). The reaction was stirred at RT 18 hrs then concentrated *in vacuo.* The crude material was dissolved in MeOH (20 mL) and 2M NaOH (aq) (20 mL) was added. The mixture was stirred for 30 min then concentrated *in vacuo.* The aqueous phase acidified to pH 6 with 1M HCl (aq) (40 mL) and the product extracted into DCM (3 x 20 mL). The organics were combined, dried (phase separator) and concentrated *in vacuo.*

The crude product was purified by chromatography on silica gel (12 g column, 0-100% EtOAc/*iso*-hexane) followed by chromatography on RP Flash C18 (5-75% MeCN/Water 0.1% formic acid) to afford N-(4-(5-chloropyridin-3-yl)phenyl)-2-(2 (cyclopropanesulfonamido)pyrimidin-4-yl)butanamide (0.158 g, 0.318 mmol, 56 % yield) as a white solid. Rt 1.36 min; m/z 472 $(M+H)^+$ $(ES^+)$; $^1H$ NMR (500 MHz, DMSO-d6) $\delta$ 11.28 (s, 1H), 10.39 (s, 1H), 8.86 (d, J = 2.0 Hz, 1H), 8.63 - 8.48 (m, 2H), 8.22 (t, J = 2.2 Hz, 1H), 7.81 - 7.71 (m, 4H), 7.19 (d, J = 5.2 Hz, 1H), 3.80 - 3.71 (m, 1H), 3.31 - 3.24 (m, 1H), 2.14 - 2.01 (m, 1H), 2.00 - 1.88 (m, 1H), 1.16 - 1.04 (m, 2H), 1.03 - 0.84 (m, 5H).

**Method 2: $AlMe_3$ mediated amide coupling from ester**

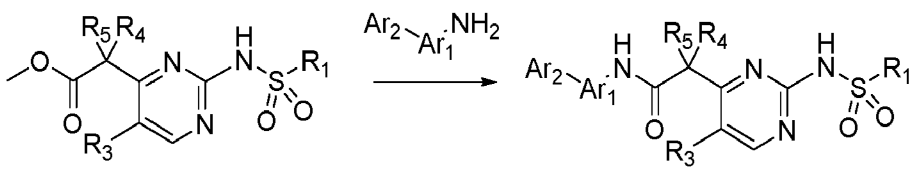

To an ice cooled solution of aniline (2 eq) in toluene (40 volumes) was added $AlMe_3$ (2.0 M in heptane, 2 eq). The mixture was stirred at this temperature for 5 mins then at RT for 10 mins. To this solution was added ester (1 eq) in one portion and the resultant mixture heated and stirred at 80 °C for 2 hrs. The reaction mixture was cooled in an ice bath and carefully quenched with MeOH (10 volumes). After stirring for 20 mins the mixture was diluted in a mixture of DCM/MeOH (10 volumes), filtered through celite and the filtrate concentrated. The crude product was purified by reverse or normal phase chromatography.

1-(2-(Cyclopropanesulfonamido)pyrimidin-4-yl)-N-(4-(6-ethoxypyrazin-2-yl)phenyl)cyclopentanecarboxamide **P2**

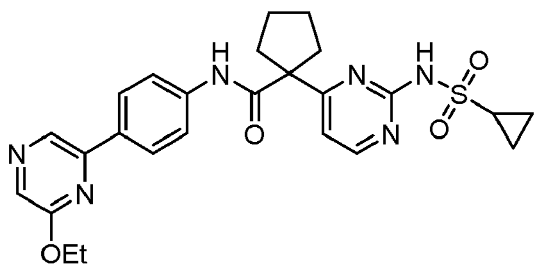

To an ice cooled solution of 4-(6-ethoxypyrazin-2-yl)aniline **INTD18** (0.099 g, 0.461 mmol) in toluene (4 mL) was added $AlMe_3$ (2.0 M in toluene) (0.307 mL, 0.615 mmol). The mixture was stirred at this temperature for 5 mins then at RT for 20 mins. To this solution was added methyl 1-(2-(cyclopropanesulfonamido)pyrimidin-4-yl)cyclopentanecarboxylate **INTC29** (0.1 g, 0.307 mmol) in one portion and the resultant mixture heated and stirred at 100°C for 3 h under $N_2$. The reaction mixture was carefully quenched with MeOH (2 mL). After stirring for 20 mins the mixture was diluted in MeOH (50 mL), filtered through celite (5 g) and the filtrate was concentrated in *vacuo*. The crude product was purified by chromatography on RP Flash C18 (25-75% MeCN/Water 0.1% formic acid) to afford 1-(2-(cyclopropanesulfonamido)pyrimidin-4-yl)-N-(4-(6-ethoxypyrazin-2-yl)phenyl)cyclopentanecarboxamide (0.053 g, 0.099 mmol, 32 % yield) as a white solid. Rt 1.59 min (UPLC, acidic); m/z 509 $(M+H)^+$ $(ES^+)$; $^1H$ NMR (500 MHz, DMSO-d6) δ 11.33 (s, 1H), 9.58 (s, 1H), 8.76 (s, 1H), 8.62 - 8.46 (m, 1H), 8.18 (s, 1H), 8.11 - 8.00 (m, 2H), 7.83 - 7.70 (m, 2H), 7.17 - 6.96 (m, 1H), 4.56 - 4.37 (m, 2H), 3.28 - 3.16 (m, 1H), 2.51 - 2.40 (m, 2H), 2.25 - 2.09 (m, 2H), 1.82 - 1.60 (m, 4H), 1.46 - 1.34 (m, 3H), 1.12 - 0.99 (m, 2H), 0.95 - 0.80 (m, 2H).

2-(2-(cyclopropanesulfonamido)pyrimidin-4-yl)-N-(4-(6-methoxypyrazin-2-yl)phenyl)-2-methylpropanamide **P3**

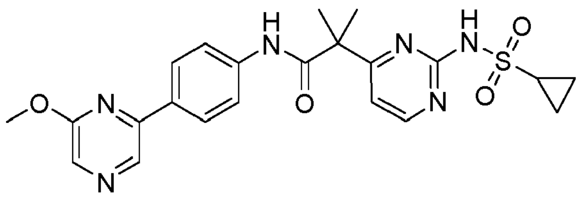

4-(6-methoxypyrazin-2-yl)aniline **INTD1** (101 mg, 0.501 mmol) was added to an ice cooled solution of $AlMe_3$ (2M in heptane) (0.33 mL, 0.668 mmol) in toluene (4 mL). The mixture was stirred at this temperature for 5 mins then at RT for 10 mins. Methyl 2-(2-(cyclopropanesulfonamido)pyrimidin-4-yl)-2-methylpropanoate **INTC21** (100 mg, 0.334 mmol) was added in one portion and the resultant mixture heated at 100°C for 2 hrs. The reaction mixture was cooled in an ice bath and carefully quenched with MeOH (10 mL). After stirring for 20 mins the mixture was diluted with a mixture of DCM/MeOH (10 mL, 1:1), filtered through celite and the solvent removed to give an orange oil. The crude product was purified by chromatography on silica gel (24 g column, 0-100% EtOAc/*iso*-hexane) to afford 2-(2-(cyclopropanesulfonamido)pyrimidin-4-yl)-N-(4-(6-methoxypyrazin-2-yl)phenyl)-2-methylpropanamide (37 mg, 0.077 mmol, 23 % yield) as a pale beige solid. Rt 2.03 min (HPLC acidic); m/z 469 $(M+H)^+$ $(ES^+)$; $^1H$ NMR (400 MHz, DMSO-d6) δ 11.27 (s, 1H), 9.51 (s, 1H), 8.78 (s, 1H), 8.61 (d, J = 5.3 Hz, 1H), 8.21 (s, 1H), 8.14 - 8.04 (m, 2H), 7.84 - 7.74 (m, 2H), 7.20 (d, J = 5.3 Hz, 1H), 4.02 (s, 3H), 3.25 - 3.18 (m, 1H), 1.60 (s, 6H), 1.08 - 0.99 (m, 2H), 0.85 - 0.74 (m, 2H).

2-(2-(cyclopropanesulfonamido)pyrimidin-4-yl)-2-methyl-N-(4-(5-(trifluoromethyl)pyridin-3-yl)phenyl)propanamide **P4**

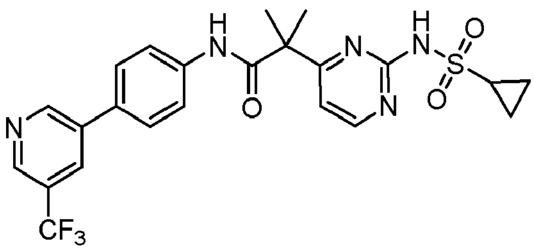

To an ice cooled solution of 4-(5-(trifluoromethyl)pyridin-3-yl)aniline **INTD7** (0.119 g, 0.501 mmol) in toluene (4 mL) and THF (2 mL) was added $AlMe_3$ (2.0 M in heptane) (0.334 mL, 0.668 mmol). The mixture was stirred at this temperature for 5 mins then at RT for 10 min. To this solution was added methyl 2-(2-(cyclopropanesulfonamido)pyrimidin-4-yl)-2-methylpropanoate **INTC21** (0.1 g, 0.334 mmol) in one portion and the resultant mixture stirred and heated at 80°C for 2 hrs in a sealed vessel. The reaction mixture was cooled in an ice bath and carefully quenched with MeOH. After stirring for 20 min the mixture was diluted in a mixture of DCM/MeOH, filtered through celite and the filtrate concentrated in *vacuo*. The crude product was purified by chromatography on RP Flash C18 (5-75% MeCN/Water 0.1% formic acid) to afford 2-(2-(cyclopropanesulfonamido)pyrimidin-4-yl)-2-methyl-N-(4-(5-(trifluoromethyl)pyridin-3-yl)phenyl)propanamide (0.109 g, 0.205 mmol, 61 % yield) as a white solid. Rt 2.17 (HPLC acidic); m/z 506 $(M+H)^+$ $(ES^+)$; $^1H$ NMR (400 MHz, DMSO-d6) δ 11.28 (s, 1H), 9.49 (s, 1H), 9.28 - 9.11 (m, 1H), 8.98 - 8.84 (m, 1H), 8.68 - 8.54 (m, 1H), 8.50 - 8.37 (m, 1H), 7.95 - 7.71 (m, 4H), 7.28 - 7.12 (m, 1H), 3.27 - 3.13 (m, 1H), 1.60 (s, 6H), 1.13 - 0.95 (m, 2H), 0.91

- 0.69 (m, 2H).

2-Methyl-N-(2-methyl-4-(6-methylpyrazin-2-yl)phenyl)-2-(2-(methylsulfonamido)pyrimidin-4-yl)propanamide **P5**

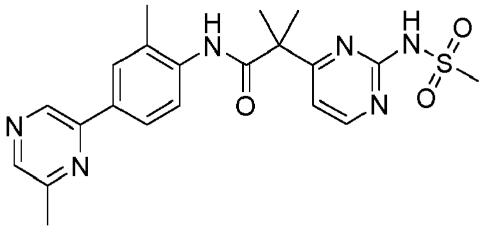

To an ice cooled solution of 4-(6-chloropyrazin-2-yl)-2-methylaniline **INTD26** (0.549 mmol, 121 mg) in toluene (2 mL) was added $AlMe_3$ (0.55 mL, 1.098 mmol). The mixture was stirred at this temperature for 5 min then at RT for 10 min. To this solution was added methyl 2-methyl-2-(2-(methylsulfonamido)pyrimidin-4-yl)propanoate **INTC19** (100 mg, 0.366 mmol) in one portion and the resultant mixture stirred and heated at 90°C for 2 hrs. The reactions were cooled to 0 °C, 1M HCl (5 mL) was added and the residues were extracted with EtOAc (2 x 20 mL). The combined organic extract was passed through a phase separator and the solvent was removed under reduced pressure. The crude product was purified by chromatography on RP Flash C18 (0-100% MeCN/Water 0.1% formic acid) to afford 2-methyl-N-(2-methyl-4-(6-methylpyrazin-2-yl)phenyl)-2-(2-(methylsulfonamido)pyrimidin-4-yl)propanamide (78.9 mg, 0.170 mmol, 47 % yield) as an off-white solid. Rt 1.74 (HPLC, acidic); m/z 441 $(M+H)^+$ $(ES^+)$; $^1H$ NMR (500 MHz, DMSO-d6) $\delta$ 11.35 (s, 1H), 9.07 - 8.99 (m, 2H), 8.62 (d, J = 5.3 Hz, 1H), 8.48 (s, 1H), 7.99 (d, J = 2.1 Hz, 1H), 7.93 (dd, J = 8.3, 2.2 Hz, 1H), 7.42 (d, J = 8.3 Hz, 1H), 7.23 (d, J = 5.3 Hz, 1H), 3.39 (s, 3H), 2.56 (s, 3H), 2.19 (s, 3H), 1.62 (s, 6H).

**Method 3: Amide coupling from potassium salt using T3P**

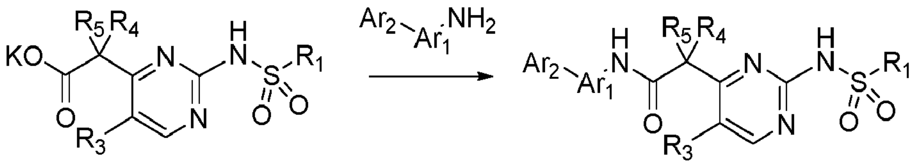

Pyridine (10 eq) followed by T3P (50 wt % in DMF, 2 eq) was added to a stirring solution of amine (1.1 eq) and potassium 2-(2-(cyclopropanesulfonamido)pyrimidin-4-yl)butanoate (1 eq) in DMF (16 volumes). The resulting reaction was stirred at RT for 24 hrs. The crude reaction mixture was concentrated *in vacuo* then diluted with $NH_4Cl$ (sat. aq) and extracted with DCM. The combined organic extracts were dried (phase separator) and the solvent removed. The crude product was purified by reverse or normal phase chromatography.

2-(2-(cyclopropanesulfonamido)pyrimidin-4-yl)-N-(2-fluoro-4-(pyrazin-2-yl)phenyl)butanamide **P6**

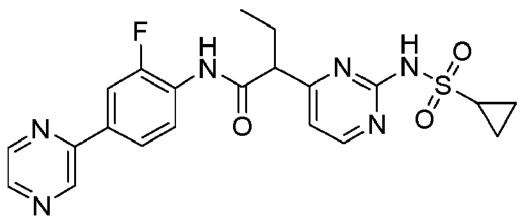

T3P (50 wt % in DMF) (1.120 mL, 1.546 mmol) was added to a stirred suspension of 2-fluoro-4-(pyrazin-2-yl)aniline **INTD23** (154 mg, 0.773 mmol), potassium 2-(2-(cyclopropanesulfonamido)pyrimidin-4-yl)butanoate **INTC37**

(250 mg, 0.773 mmol) and pyridine (0.313 mL, 3.87 mmol) in DMF (1 mL). The resulting reaction was stirred at RT for 18 hrs. Water (5 mL) was added and the newly formed precipitate filtered. The product was recovered by dissolving in DCM (10 mL) and concentrated *in vacuo.* The crude product was purified by preparative HPLC (20-50% MeCN/Water 0.1% formic acid) to afford 2-(2-(cyclopropanesulfonamido)pyrimidin-4-yl)-N-(2-fluoro-4-(pyrazin-2-yl)phenyl)butanamide (32 mg, 0.069 mmol, 9 % yield) as a colourless powder. Rt 1.15 min (UPLC acidic); m/z 457 $(M+H)^+$ $(ES^+)$; $^1H$ NMR (500 MHz, DMSO-d6) δ 11.26 (s, 1H), 10.25 (s, 1H), 9.29 (d, J = 1.6 Hz, 1H), 8.72 (dd, J = 2.5, 1.5 Hz, 1H), 8.62 (d, J = 2.5 Hz, 1H), 8.57 (d, J = 5.2 Hz, 1H), 8.12 - 8.03 (m, 2H), 8.03 - 7.97 (m, 1H), 7.20 (d, J = 5.2 Hz, 1H), 4.00 (dd, J = 7.5 Hz, 1H), 3.31 - 3.28 (m, 1H), 2.12 - 2.02 (m, 1H), 2.00 - 1.92 (m, 1H), 1.16 - 1.07 (m, 2H), 1.03 - 0.93 (m, 5H).

2-(2-(cyclopropanesulfonamido)pyrimidin-4-yl)-N-(4-(5-(trifluoromethyl)pyridin-3-yl)phenyl)butanamide **P7**

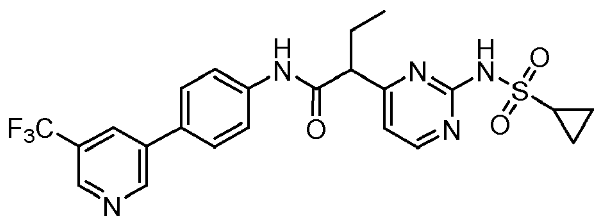

T3P (50 wt % in DMF) (0.78 mL, 1.082 mmol) was added to a stirred suspension of potassium 2-(2-(cyclopropanesulfonamido)pyrimidin-4-yl)butanoate **INTC37** (250 mg, 0.541 mmol) and 4-(5-(trifluoromethyl)pyridin-3-yl)aniline **INTD7** (129 mg, 0.541 mmol) in pyridine (0.13 mL, 1.623 mmol) and DMF (3 mL). The resulting reaction was stirred at RT for 18 hrs. The crude reaction mixture was diluted with saturated $NH_4Cl$ (aq) (10 mL) and extracted with DCM (3 x 10 mL). The combined organic extracts were dried (phase separator) and the solvent removed under reduced pressure. The crude product was purified by chromatography on silica gel (0-10% MeOH in DCM), followed by chromatography on RP Flash C18 (15-75% MeCN/Water 0.1% formic acid) to afford 2-(2-(cyclopropanesulfonamido)pyrimidin-4-yl)-N-(4-(5-(trifluoromethyl)pyridin-3-yl)phenyl)butanamide (19 mg; 0.036 mmol; 7 % yield). Rt 1.44 (UPLC, acidic); m/z 506 $(M+H)^+$ $(ES^+)$; $^1H$ NMR (500 MHz, DMSO-d6) δ 11.25 (s, 1H), 10.41 (s, 1H), 9.20 (d, J = 2.2 Hz, 1H), 8.94 - 8.92 (m, 1H), 8.57 (d, J = 5.2 Hz, 1H), 8.45 - 8.42 (m, 1H), 7.87 - 7.83 (m, 2H), 7.79 - 7.75 (m, 2H), 7.21 (d, J = 5.2 Hz, 1H), 3.77 (dd, J = 8.7, 6.3 Hz, 1H), 3.31 - 3.26 (m, 1H), 2.13 - 2.03 (m, 1H), 1.98 - 1.89 (m, 1H), 1.13 - 1.06 (m, 2H), 1.01 - 0.89 (m, 5H).

2-(2-(cyclopropanesulfonamido)pyrimidin-4-yl)-N-(4-(6-(trifluoromethyl)pyrazin-2-yl)phenyl)acetamide **P8**

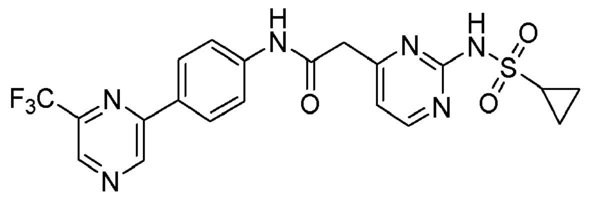

T3P (50 wt % in DMF) (0.343 mL, 0.474 mmol) was added to a stirred suspension of potassium 2-(2-(cyclopropanesulfonamido)pyrimidin-4-yl)acetate **INTC39** (100 mg, 0.237 mmol), 4-(6-(trifluoromethyl)pyrazin-2-yl)aniline **INTD19** (56.7 mg, 0.237 mmol) and pyridine (0.096 mL, 1.185 mmol) in DMF (1 mL). The resulting reaction was stirred at RT for 18 hrs. Water (5 mL) was added and the newly formed precipitate was filtered to afford the crude product. The crude product was purified by chromatography on silica gel (0-10% MeOH in DCM) followed by preparative HPLC (5-95% MeCN/Water 0.1% formic acid) to afford 2-(2-(cyclopropanesulfonamido)pyrimidin-4-yl)-N-(4-(6-(trifluoromethyl)pyrazin-2-yl)phenyl)acetamide (10 mg, 0.021 mmol, 9 % yield) as a yellow powder. Rt 1.31 min (UPLC, acidic); 479 m/z $(M+H)^+$ $(ES^+)$; $^1H$ NMR (500 MHz, DMSO-d6) observed as mixture of tautomers δ 12.81 (s, 1H, minor), 11.24 (s, 1H, major), 10.95 (s, 1H, minor), 10.58 (s, 1H, major), 10.09 (s, 1H, minor), 9.58 (s, 1H, major), 9.57 (s, 1H, minor), 9.09 (s, 1H, major), 9.06 (s, 1H, minor), 8.57 (d, J = 5.1 Hz, 1H, major), 8.24 - 8.13 (m, 2 x 2H, major and minor), 7.85 - 7.79 (m, 2 x 2H, major and minor), 7.18 (d, J = 5.0 Hz, 1H, major), 6.95 (d, J = 7.5 Hz, 1H, minor), 5.89 (d, J = 7.5 Hz, 1H, minor), 5.06 (s, 1H, minor), 3.89 (s, 2H, major), 3.28 - 3.22 (m, 1H, major), 2.73 - 2.65 (m, 1H, minor), 1.13 - 0.90 (m, 2 x 4H,

major and minor).

**NH-Amide formation *via* amide deprotection and/or decarboxylation**

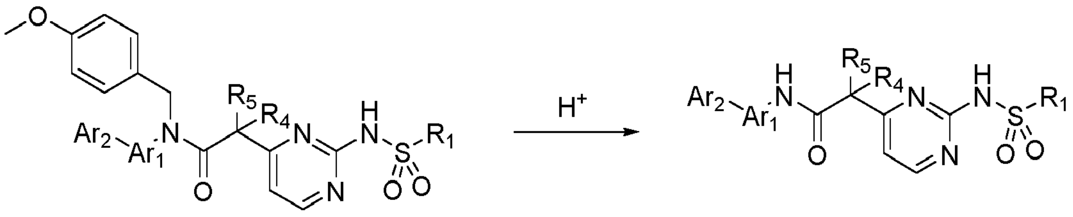


$R_4$ = Alkyl, $R_5$ = Alkyl or H
or
$R_4$ = *t*Bu-ester, $R_5$ = H

$R_4$ = Alkyl, $R_5$ = Alkyl or H
or
$R_4$ = $R_5$ = H

To a solution of the protected amide in DCM a mixture of TFA (88 eq) and triflic acid (1-6 eq) was added and the mixture left stirring at RT for 18-36 hrs and then concentrated *in vacuo.* The crude product was purified by column chromatography on silica gel or by RP chromatography.

2-(2-(cyclopropanesulfonamido)pyrimidin-4-yl)-N-(4-(6-ethoxypyrazin-2-yl)phenyl)butanamide **P105**

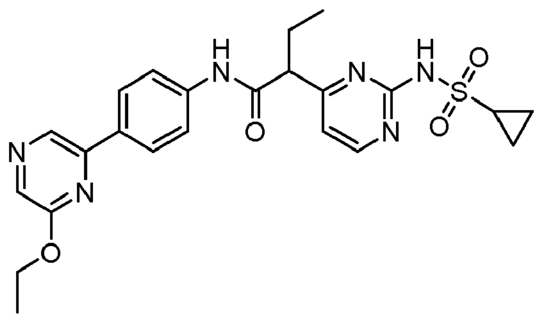

A solution of 2-(2-(cyclopropanesulfonamido)pyrimidin-4-yl)-N-(4-(6-ethoxypyrazin-2-yl)phenyl)-N-(4-methoxybenzyl)butanamide **INTC46** (0.18 g, 0.299 mmol) in a mixture of TFA (2 mL, 26.0 mmol) and DCM (2 mL) was stirred at 25°C for 18 hrs. The reaction was heated at 50°C for 2 hrs. To the reaction was added triflic acid (0.027 mL, 0.299 mmol) and the mixture stirred at 25°C for 2 hrs. The reaction mixture was concentrated and then diluted in 1 N HCl (aq) (20 mL). The aqueous phase was extracted with DCM (3 x 20 mL), dried (phase separator) and the solvent was removed under reduced pressure. The crude product was purified by chromatography on RP Flash C18 (24 g column, 5-75% MeCN/Water 0.1% formic acid) to afford 2-(2-(cyclopropanesulfonamido)pyrimidin-4-yl)-N-(4-(6-ethoxypyrazin-2-yl)phenyl)butanamide (0.02 g, 0.041 mmol, 14 % yield) as a white solid. Rt 2.23 min (HPLC acidic); 483 $(M+H)^+$ $(ES^+)$.

2-(2-(cyclopropanesulfonamido)pyrimidin-4-yl)-N-(4-(6-ethoxypyrazin-2-yl)phenyl)acetamide **P18**

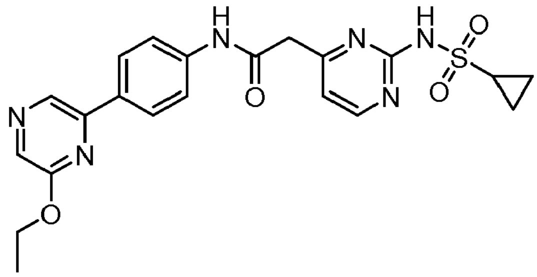

To a solution of tert-butyl 2-(2-(cyclopropanesulfonamido)pyrimidin-4-yl)-3-((4-(6-ethoxypyrazin-2-yl)phenyl)(4-methoxybenzyl)amino)-3-oxopropanoate **INTC47** (0.1 g, 0.148 mmol) in a mixture of TFA (1 mL, 12.98 mmol) and DCM (20 mL) was added triflic Acid (0.039 mL, 0.445 mmol). The mixture was stirred at 25°C for 18 hrs. Further triflic acid (0.039 mL, 0.445 mmol) was added and the mixture stirred at 25°C for a further 18 hrs. The reaction mixture was concentrated under reduced pressure. The crude product was purified by chromatography on silica gel (12 g column, 0-10% MeOH/DCM,) to afford 2-(2-(cyclopropanesulfonamido)pyrimidin-4-yl)-N-(4-(6-ethoxypyrazin-2-yl)phenyl)acetamide (0.03 g, 0.063 mmol, 42 % yield) as a pale yellow solid. Rt 1.98 min (HPLC, acidic); m/z 455 $(M+H)^+$ $(ES^+)$.

**Table 6:** Preparation methods and characterisation data of examples **P9-P111**

| P# | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P9** | 2-(2-(cyclopropanesulfonamido)pyrimidin-4-yl)-N-(5-(6-isopropoxypyrazin-2-yl)pyridin-2-yl)-2-methylpropanamide 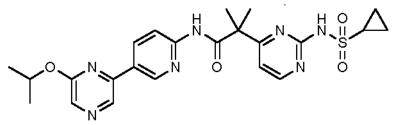 | Method 2: using INTC24 and INTD32 [HPLC acidic], 498, (2.28) | 11.23 (s, 1H), 10.14 (s, 1H), 9.03 - 8.97 (m, 1H), 8.82 (s, 1H), 8.60 (d, J = 5.3 Hz, 1H), 8.53 - 8.42 (m, 1H), 8.25 - 8.16 (m, 2H), 7.21 (d, J = 5.3 Hz, 1H), 5.46 - 5.34 (m, 1H), 3.23 - 3.10 (m, 1H), 1.61 (s, 6H), 1.40 - 1.37 (m, 6H), 1.04 - 0.98 (m, 2H), 0.81 - 0.74 (m, 2H). |
| **P10** | 2-(2-(cyclopropanesulfonamido)pyrimidin-4-yl)-N-(4-(6-ethoxypyrazin-2-yl)phenyl)-2-ethylbutanamide 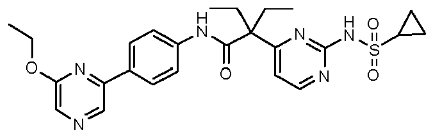 | Method 2 using INTC27 and INTD18, [UPLC acidic], 511, (1.57) | 11.27 (s, 1H), 9.47 (s, 1H), 8.75 (s, 1H), 8.59 (d, J = 5.3 Hz, 1H), 8.18 (s, 1H), 8.09 - 8.01 (m, 2H), 7.79 - 7.73 (m, 2H), 7.14 (d, J = 5.3 Hz, 1H), 4.47 (q, J = 7.1 Hz, 2H), 3.23 - 3.11 (m, 1H), 2.12 (q, J = 7.6 Hz, 4H), 1.40 (t, J = 7.1 Hz, 3H), 1.05 - 0.96 (m, 2H), 0.80 - 0.66 (m, 8H). |
| **P11** | 2-(2-(cyclopropanesulfonamido)pyrimidin-4-yl)-N-(2-fluoro-4-(6-(trifluoromethyl)pyrazin-2-yl)phenyl)acetamide 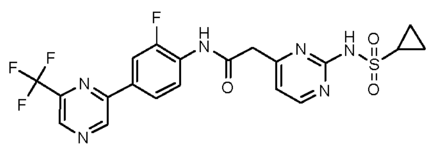 | Method 2 using INTC33 and INTD25, [HPLC Acidic], 497, (2.09) | 12.77 (s, 1H, minor), 11.24 (s, 1H, major), 10.98 (s, 1H, minor), 10.37 (s, 1H, major), 9.81 (s, 1H, minor), 9.64 (s, 1H, major), 9.63 (s, 1H, minor), 9.14 (s, 1H, major), 9.12 (s, 1H, minor), 8.57 (d, J = 5.1 Hz, 1H, major), 8.34 (t, J = 8.3 Hz, 1H, minor), 8.24 (t, J = 8.3 Hz, 1H, major), 8.17 - 8.04 (m, 2 x 2H, major and minor), 7.18 (d, J = 5.1 Hz, 1H, major), 6.96 (d, J = 7.6 Hz, 1H, minor), 5.85 (dd, J = 7.7, 1.6 Hz, 1H, minor), 5.34 (s, 1H, minor), 4.00 (s, 2H, major), 3.30 - 3.23 (m, 1H, major), 2.71 - 2.61 (m, 1H, minor), 1.15 - 0.88 (m, 2 x 4H, major and minor). |

(continued)

| P# | Name/Structure (All examples containing chiral centres are racemates unless stated) | Synthesis Method, [LCMS Method], m/z(M+H)+, (RT/Min) | $^1$H NMR Chemical Shift Data (DMSO-d6 unless stated) |
|---|---|---|---|
| **P12** | 2-(2-(cyclopropanesulfonamido)pyrimidin-4-yl)-N-(2-fluoro-4-(6-isopropoxypyrazin-2-yl) phenyl)acetamide 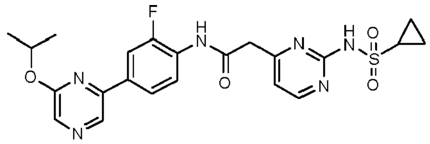 | Method 2 using INTC33 and INTD35, [HPLC Acidic], 487, (2.17) | 12.79 (s, 1H, minor), 11.23 (s, 1H, major), 10.95 (s, 1H, minor), 10.29 (s, 1H, major), 9.74 (s, 1H, minor), 8.81 (s, 1H, major), 8.80 (s, 1H, minor), 8.57 (d, J = 5.0 Hz, 1H, major), 8.23 (t, J = 8.5 Hz, 1H, minor), 8.19 (s, 1H, major), 8.17 (s, 1H, minor), 8.13 (t, J = 8.3 Hz, 1H, major), 8.06 - 7.93 (m, 2 x 2H, major and minor), 7.17 (d, J = 5.2 Hz, 1H, major), 6.94 (d, J = 7.5 Hz, 1H, minor), 5.84 (dd, J = 7.6, 1.7 Hz, 1H, minor), 5.47 - 5.38 (m, 2 x 1H, major and minor), 5.31 (s, 1H, minor), 3.98 (s, 2H, major), 3.26 (s, 1H, major), 2.69 - 2.62 (m, 1H, minor), 1.39 (dd, J = 6.2, 1.9 Hz, 2 x 6H, major and minor), 1.13 - 0.90 (m, 2 x 4H, major and minor). |
| **P13** | 2-(2-(cyclopropanesulfonamido)pyrimidin-4-yl)-N-(4-(5-(trifluoromethyl)pyridin-3-yl)phenyl) acetamide 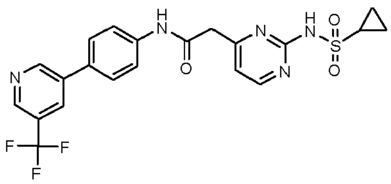 | Method 3 using INTC39 and INTD7, [UPLC Acidic], 478, (1.27) | 12.82 (s, 1H, minor), 11.25 (s, 1H, major), 10.92 (s, 1H, minor), 10.47 (s, 1H, major), 9.98 (s, 1H, minor), 9.23 - 9.19 (m, 2 x 1H, major and minor), 8.95 - 8.90 (m, 2 x 1H, major and minor), 8.56 (d, J = 5.0 Hz, 1H, major), 8.46 - 8.41 (m, 2 x 1H, major and minor), 7.89 - 7.81 (m, 2 x 2H, major and minor), 7.80 - 7.74 (m, 2 x 2H, major and minor), 7.17 (d, J = 5.0 Hz, 1H, major), 6.93 (d, J = 7.5 Hz, 1H, minor), 5.88 (d, J = 7.6 Hz, 1H, minor), 5.04 (s, 1H, minor), 3.87 (s, 2H, major), 3.28 - 3.22 (m, 1H, major), 2.70 - 2.65 (m, 1H, minor), 1.13 - 0.90 (m, 2 x 4H, major and minor). |
| **P14** | 2-(2-(cyclopropanesulfonamido)pyrimidin-4-yl)-N-(4-(5-(2,2,2-trifluoroethoxy)pyridin-3-yl) phenyl)acetamide 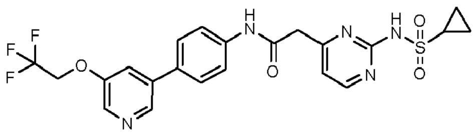 | Method 2 using INTC33 and INTD15, [HPLC Acidic], 508, (1.87) | 12.83 (s, 1H, minor), 11.26 (s, 1H, major), 10.89 (s, 1H, minor), 10.43 (s, 1H, major), 9.96 (s, 1H, minor), 8.59 (t, J = 2.2 Hz, 2 x 1H, major and minor), 8.57 (d, J = 5.1 Hz, 1H, major), 8.35 (dd, J = 4.6, 2.8 Hz, 2 x 1H, major and minor), 7.83 - 7.68 (m, 2 x 5H, major and minor), 7.17 (d, J = 5.1 Hz, 1H, major), 6.92 (d, J = 7.6 Hz, 1H, minor), 5.88 (dd, J = 7.6, 1.6 Hz, 1H, minor), 5.05 (s, 1H, minor), 5.01 - 4.94 (m, 2 x 2H, major and minor), 3.87 (s, 2H, major), 3.30 - 3.23 (m, 1H, major), 2.71 - 2.62 (m, 1H, minor), 1.13 - 0.89 (m, 2 x 4H, major and minor. |

(continued)

| P# | Name/Structure (All examples containing chiral centres are racemates unless stated) | Synthesis Method, [LCMS Method], m/z(M+H)$^+$, (RT/Min) | $^1$H NMR Chemical Shift Data (DMSO-d6 unless stated) |
|---|---|---|---|
| **P15** | 2-(2-(cyclopropanesulfonamido)-5-fluoropyrimidin-4-yl)-N-(4-(pyridin-3-yl)phenyl) acetamide 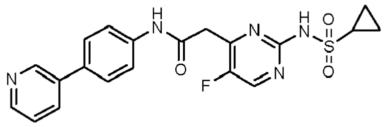 | Method 1 using INTC40 and a commercial aniline, [UPLC acidic], 428, (0.68) | 11.36 (s, 1H), 10.51 (s, 1H), 8.89 (s, 1H), 8.74 - 8.61 (m, 1H), 8.60 - 8.42 (m, 1H), 8.11 - 7.99 (m, 1H), 7.78 - 7.59 (m, 4H), 7.55 - 7.38 (m, 1H), 4.03 - 3.85 (m, 2H), 3.24 - 3.11 (m, 1H), 1.15 - 1.01 (m, 2H), 0.99 - 0.88 (m, 2H). |
| **P16** | 2-(2-(cyclopropanesulfonamido)pyrimidin-4-yl)-N-(4-(pyridin-3-yl)phenyl)acetamide 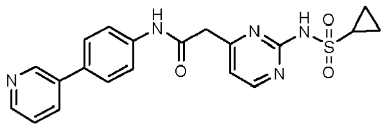 | Method 2 using INTC33 and a commercial aniline, [HPLC basic], 410, (1.20) | 12.82 (s, 1H, minor), 11.22 (s, 2 x 1H, major and minor), 10.42 (s, 2 x 1H, major and minor), 9.92 (s, 1H, minor), 8.89 (d, *J* = 2.2 Hz, 3H), 8.60 - 8.50 (m, 4H), 8.11 - 8.01 (m, 3H), 7.80 - 7.67 (m, 11H), 7.46 (ddd, *J* = 7.3, 4.8, 1.7 Hz, 2H), 7.16 (d, *J* = 5.1 Hz, 1H, major), 6.92 (d, *J* = 7.5 Hz, 1H, minor), 5.87 (dd, *J* = 7.6, 1.6 Hz, 1H, minor), 5.03 (s, 1H, minor), 3.86 (s, 2H, major), 3.29 - 3.22 (m, 1H, major), 3.18 (s, 3H), 2.73 - 2.61 (m, 1H, minor), 1.13 - 1.06 (m, 2H, major), 1.05 - 0.98 (m, 2H, minor), 0.98 - 0.88 (m, 2 x 2H, major and minor). |
| **P17** | N-([1,1'-biphenyl]-4-yl)-2-(2-(cyclopropanesulfonamido)pyrimidin-4-yl) acetamide 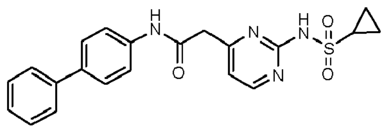 | Method 2, using INTC33 and a commercial aniline, [HPLC basic], 409, (1.84) | 12.84 (s, 1H, minor), 11.19 (s, 2H, major), 10.37 (s, 1H, major), 9.89 (s, 1H, minor), 8.59 - 8.53 (m, 1H, major), 7.71 - 7.40 (m, 2 x 8H, major and minor) 7.38 - 7.28 (m, 2 x 1H, major and minor), 7.19 - 7.13 (m, 1H, major), 6.94 - 6.87 (m, 1H, minor), 5.90 - 5.82 (m, 1H, minor), 5.04 (s, 1H, minor), 3.86 (s, 2H, major), 3.30 - 3.14 (m, 1H, major), 2.69 - 2.61 (m, 1H, minor), 1.14 - 0.88 (m, 2 x 4H, major and minor). |

(continued)

| P# | Name/Structure (All examples containing chiral centres are racemates unless stated) | Synthesis Method, [LCMS Method], m/z(M+H)+, (RT/Min) | $^1$H NMR Chemical Shift Data (DMSO-d6 unless stated) |
|---|---|---|---|
| **P18** | 2-(2-(cyclopropanesulfonamido)pyrimidin-4-yl)-N-(4-(6-ethoxypyrazin-2-yl)phenyl) acetamide 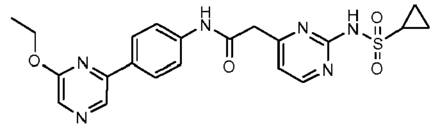 | Method 3 using INTC39 and INTD18, [UPLC Acidic], 455, (1.26) | 12.83 (s, 1H, minor), 11.23 (s, 1H, major), 10.91 (s, 1H, minor), 10.49 (s, 1H, major), 10.01 (s, 1H, minor), 8.77 (s, 1H, major), 8.76 (s, 1H, minor), 8.57 (d, J = 5.2 Hz, 1H, major), 8.19 (s, 1H, major), 8.17 (s, 1H, minor), 8.13 - 8.06 (m, 2 x 2H, major and minor), 7.79 - 7.73 (m, 2 x 2H, major and minor), 7.17 (d, J = 5.0 Hz, 1H, major), 6.93 (d, J = 7.6 Hz, 1H, minor), 5.88 (d, J = 7.6 Hz, 1H, minor), 5.05 (s, 1H, minor), 4.48 (q, J = 7.0 Hz, 2 x 2H, major and minor), 3.87 (s, 2H, major), 3.28 - 3.22 (m, 1H, major), 2.71 - 2.65 (m, 1H, minor), 1.40 (t, J = 7.0 Hz, 2 x 3H, major and minor), 1.13 - 0.90 (m, 2 x 4H, major and minor). |
| **P19** | 2-(2-(cyclopropanesulfonamido)pyrimidin-4-yl)-N-(4-(6-methoxypyrazin-2-yl)phenyl) acetamide 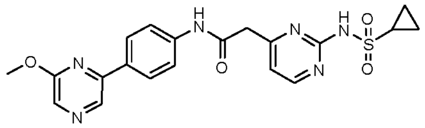 | Method 2, using INTC33 and INTD1, [HPLC Acidic], 441, (1.83) | 12.83 (s, 1H, minor), 11.24 (s, 1H, major), 10.91 (s, 1H, minor), 10.50 (s, 1H, major), 10.02 (s, 1H, minor), 8.79 (s, 1H, major), 8.78 (s, 1H, minor), 8.57 (d, J = 5.1 Hz, 1H, major), 8.22 (s, 1H, major), 8.20 (s, 1H, minor), 8.16 - 8.09 (m, 2 x 2H, major and minor), 7.79 - 7.73 (m, 2 x 2H, major and minor), 7.18 (d, J = 5.1 Hz, 1H, major), 6.93 (d, J = 7.6 Hz, 1H, minor), 5.88 (dd, J = 7.7, 1.6 Hz, 1H, minor), 5.06 (s, 1H, minor), 4.02 (s, 2 x 3H, major and minor), 3.88 (s, 2H, major), 3.30 - 3.21 (m, 1H, major), 2.70 - 2.62 (m, 1H, minor), 1.14 - 0.88 (m, 2 x 4H, major and minor). |
| **P20** | 2-(2-(cyclopropanesulfonamido)pyrimidin-4-yl)-N-(4-(6-(2,2,2-trifluoroethoxy)pyrazin-2-yl) phenyl)acetamide 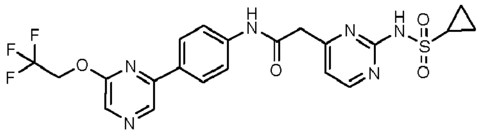 | Method 2, using INTC33 and INTD29, [HPLC Acidic], 509, (2.12) | 12.84 (s, 1H, minor), 11.24 (s, 1H, major), 10.92 (s, 1H, minor), 10.51 (s, 1H, major), 10.04 (s, 1H, minor), 8.94 (s, 1H, major), 8.92 (s, 1H, minor), 8.57 (d, J = 5.1 Hz, 1H, major), 8.38 (s, 1H, major), 8.36 (s, 1H, minor), 8.20 - 8.14 (m, 2 x 2H, major and minor), 7.79 - 7.75 (m, 2 x 2H, major and minor), 7.18 (d, J = 5.1 Hz, 1H, major), 6.93 (d, J = 7.6 Hz, 1H, minor), 5.89 (dd, J = 7.7, 1.6 Hz, 1H, minor), 5.24 - 5.15 (m, 2 x 2H, major and minor), 5.06 (s, 1H, minor), 3.88 (s, 2H, major), 3.29 - 3.22 (m, 1H, major), 2.70 - 2.63 (m, 1H, minor), 1.12 - 0.91 (m, 2 x 4H, major and minor). |

(continued)

| P# | Name/Structure (All examples containing chiral centres are racemates unless stated) | Synthesis Method, [LCMS Method], m/z(M+H)$^+$, (RT/Min) | $^1$H NMR Chemical Shift Data (DMSO-d6 unless stated) |
|---|---|---|---|
| **P21** | 2-(2-(cyclopropanesulfonamido)pyrimidin-4-yl)-N-(4-(6-isopropoxypyrazin-2-yl)phenyl) acetamide 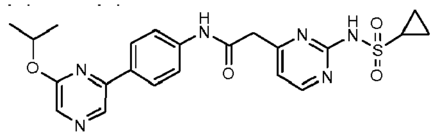 | Method 2, using INTC33 and INTD20, [HPLC Acidic], 469, (2.12) | 12.84 (s, 1H, minor), 11.24 (s, 1H, major), 10.91 (s, 1H, minor), 10.49 (s, 1H, major), 10.02 (s, 1H, minor), 8.74 (s, 1H, major), 8.73 (s, 1H, minor), 8.57 (d, J = 5.1 Hz, 1H, major), 8.13 (s, 1H, major), 8.11 (s, 1H, minor), 8.11 - 8.05 (m, 2 x 2H, major and minor), 7.78 - 7.72 (m, 2 x 2H, major and minor), 7.18 (d, J = 5.1 Hz, 1H, major), 6.93 (d, J = 7.6 Hz, 1H, minor), 5.88 (dd, J = 7.6, 1.6 Hz, 1H, minor), 5.46 - 5.36 (m, 2 x 1H, major and minor), 5.06 (s, 1H, minor), 3.88 (s, 2H, major), 3.29 - 3.21 (m, 1H, major), 2.70 - 2.61 (m, 1H, minor), 1.42 - 1.35 (m, 2 x 6H, major and minor), 1.13 - 0.90 (m, 2 x 4H, major and minor). |
| **P22** | 2-(2-(cyclobutanesulfonamido)pyrimidin-4-yl)-N-(5-(6-ethoxypyrazin-2-yl)pyridin-2-yl)-2-methylpropanamide 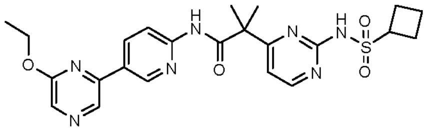 | Method 2, using INTC24 and INTD16, [HPLC Acidic], 498, (2.24) | 11.15 (s, 1H), 10.19 (s, 1H), 9.05 (d, J = 2.4 Hz, 1H), 8.85 (s, 1H), 8.60 - 8.51 (m, 2H), 8.29 (d, J = 8.8 Hz, 1H), 8.25 (s, 1H), 7.17 (d, J = 5.3 Hz, 1H), 4.55 - 4.43 (m, 3H), 2.32 - 2.22 (m, 2H), 2.04 - 1.92 (m, 2H), 1.80 1.70 (m, 1H), 1.59 (s, 6H), 1.57 - 1.52 (m, 1H), 1.40 (t, J = 7.0 Hz, 3H) |
| **P23** | 2-(2-(cyclobutanesulfonamido)pyrimidin-4-yl)-N-(2-fluoro-4-(6-isopropoxypyrazin-2-yl) phenyl)-2-methylpropanamide 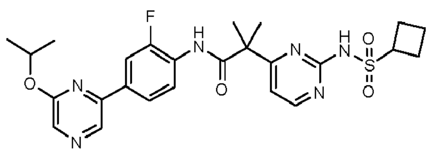 | Method 2, using INTC24 and INTD35, [HPLC Acidic], 529, (2.48) | 11.15 (s, 1H), 9.36 (s, 1H), 8.82 (s, 1H), 8.59 (d, J = 5.2 Hz, 1H), 8.19 (s, 1H), 8.02 - 7.94 (m, 2H), 7.79 - 7.73 (m, 1H), 7.20 (d, J = 5.3 Hz, 1H), 5.46 - 5.38 (m, 1H), 4.64 - 4.55 (m, 1H), 2.44 - 2.33 (m, 2H), 2.23 - 2.12 (m, 2H), 1.90 - 1.76 (m, 2H), 1.60 (s, 6H), 1.38 (d, J = 6.3 Hz, 6H); |
| **P24** | 2-(2-(cyclobutanesulfonamido)pyrimidin-4-yl)-N-(4-(6-ethoxypyrazin-2-yl)-2-methylphenyl)-2-methylpropanamide 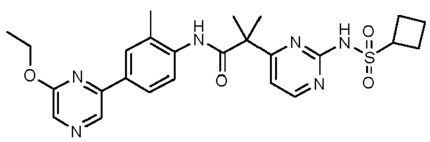 | Method 2, using INTC24 and INTD27, [HPLC Acidic], 511, (2.31) | 11.18 (s, 1H), 9.03 (s, 1H), 8.78 (s, 1H), 8.59 (d, J = 5.2 Hz, 1H), 8.20 (s, 1H), 7.98 (d, J = 2.0 Hz, 1H), 7.93 (dd, J = 8.3, 2.1 Hz, 1H), 7.46 (d, J = 8.3 Hz, 1H), 7.21 (d, J = 5.3 Hz, 1H), 4.66 - 4.56 (m, 1H), 4.48 (q, J = 7.0 Hz, 2H), 2.45 - 2.34 (m, 2H), 2.26 - 2.12 (m, 5H), 1.94 - 1.83 (m, 2H), 1.61 (s, 6H), 1.40 (t, J = 7.0 Hz, 3H). |
| **P25** | 2-(2-(cyclobutanesulfonamido)pyrimidin-4-yl)-N-(4-(6-methoxypyrazin-2-yl)phenyl)-2-methylpropanamide 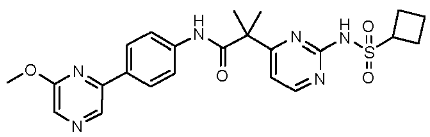 | Method 2, using INTC24 and INTD1, [HPLC Acidic], 483, (2.18) | 11.11 (s, 1H), 9.50 (s, 1H), 8.79 (s, 1H), 8.59 (d, J = 5.3 Hz, 1H), 8.21 (s, 1H), 8.15 - 8.09 (m, 2H), 7.90 - 7.81 (m, 2H), 7.21 (d, J = 5.3 Hz, 1H), 4.54 (p, J = 8.4 Hz, 1H), 4.01 (s, 3H), 2.37 - 2.22 (m, 2H), 2.07 - 1.97 (m, 2H), 1.64 - 1.54 (m, 6H), 0.89 - 0.82 (m, 2H), |

(continued)

| P# | Name/Structure (All examples containing chiral centres are racemates unless stated) | Synthesis Method, [LCMS Method], m/z(M+H)$^+$, (RT/Min) | $^1$H NMR Chemical Shift Data (DMSO-d6 unless stated) |
|---|---|---|---|
| **P26** | 2-(2-(cyclobutanesulfonamido)pyrimidin-4-yl)-N-(4-(6-ethoxypyrazin-2-yl)phenyl)-2-methylpropanamide 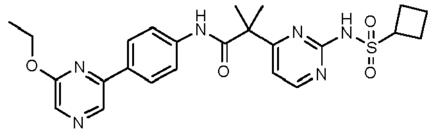 | Method 2, using INTC24 and INTD18, [HPLC Basic], 497, (2.33) | 11.14 (s, 1H), 9.51 (s, 1H), 8.77 (s, 1H), 8.58 (d, J = 5.3 Hz, 1H), 8.18 (s, 1H), 8.13 - 8.06 (m, 2H), 7.88 - 7.77 (m, 2H), 7.19 (d, J = 5.4 Hz, 1H), 4.54 (q, J = 8.3 Hz, 1H), 4.47 (q, J = 7.1 Hz, 2H), 2.36 - 2.24 (m, 2H), 2.06 - 1.95 (m, 2H), 1.81 - 1.69 (m, 1H), 1.66 - 1.60 (m, 1H), 1.58 (s, 6H), 1.40 (t, J = 7.0 Hz, 3H). |
| **P27** | 2-(2-(cyclopropanesulfonamido)pyrimidin-4-yl)-N-(5-(6-ethoxypyrazin-2-yl)-3-fluoropyridin-2-yl)-2-methylpropanamide 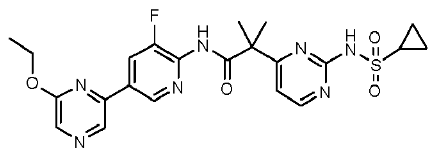 | Method 2, using INTC21 and INTD31, [UPLC Acidic], 502, (1.27) | 11.28 (s, 1H), 10.08 (s, 1H), 8.99 - 8.97 (m, 1H), 8.93 (s, 1H), 8.62 (d, J = 5.3 Hz, 1H), 8.45 - 8.40 (m, 1H), 8.32 (s, 1H), 7.19 (d, J = 5.3 Hz, 1H), 4.50 (q, J = 7.0 Hz, 2H), 3.33 - 3.27 (m, 1H), 1.62 (s, 6H), 1.41 (t, J = 7.0 Hz, 3H), 1.15 - 1.07 (m, 2H), 1.06 - 0.98 (m, 2H). |
| **P28** | 2-(2-(cyclopropanesulfonamido)pyrimidin-4-yl)-N-(5'-ethoxy-[3,3'-bipyridin]-6-yl)-2-methylpropanamide 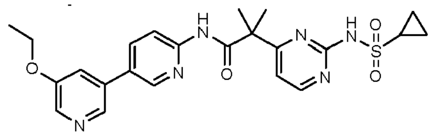 | Method 2, using INTC21 and INTD16, [UPLC acidic], 483, (1.09) | 11.25 (s, 1H), 10.06 (s, 1H), 8.72 - 8.68 (m, 1H), 8.60 (d, J = 5.3 Hz, 1H), 8.52 (d, J = 1.9 Hz, 1H), 8.29 (d, J = 2.7 Hz, 1H), 8.24 - 8.12 (m, 2H), 7.70 - 7.65 (m, 1H), 7.20 (d, J = 5.3 Hz, 1H), 4.21 (q, J = 7.0 Hz, 2H), 3.23 - 3.13 (m, 1H), 1.61 (s, 6H), 1.38 (t, J = 7.0 Hz, 3H), 1.07 - 0.97 (m, 2H), 0.83 - 0.73 (m, 2H). |
| **P29** | N-([3,3'-bipyridin]-6-yl)-2-(2-(cyclopropanesulfonamido)pyrimidin-4-yl)-2-methylpropanamide 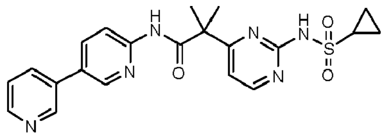 | Method 2, using INTC21 and a commercial aniline, [HPLC acidic], 439, (1.29) | 11.25 (s, 1H), 10.06 (s, 1H), 8.94 (d, J = 2.3 Hz, 1H), 8.70 - 8.67 (m, 1H), 8.61 - 8.56 (m, 2H), 8.22 - 8.08 (m, 3H), 7.54 - 7.46 (m, 1H), 7.18 (d, J = 5.3 Hz, 1H), 3.23 - 3.13 (m, 1H), 1.61 (s, 6H), 1.07 - 0.96 (m, 2H), 0.83 - 0.71 (m, 2H). |
| **P30** | 2-(2-(cyclopropanesulfonamido)pyrimidin-4-yl)-2-methyl-N-(5-(6-(trifluoromethyl)pyrazin-2-yl)pyridin-2-yl)propanamide 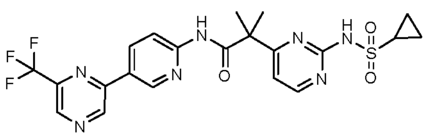 | Method 2 using INTC21 and INTD2, [UPLC Acidic], 508, (1.41) | 11.24 (s, 1H), 10.29 (s, 1H), 9.66 (s, 1H), 9.21 - 9.02 (m, 2H), 8.69 - 8.49 (m, 2H), 8.36 - 8.19 (m, 1H), 7.21 (d, J = 5.3 Hz, 1H), 3.23 - 3.11 (m, 1H), 1.61 (s, 6H), 1.07 - 0.91 (m, 2H), 0.83 - 0.71 (m, 2H). |

(continued)

| P# | Name/Structure (All examples containing chiral centres are racemates unless stated) | Synthesis Method, [LCMS Method], m/z (M+H)$^+$, (RT/Min) | $^1$H NMR Chemical Shift Data (DMSO-d6 unless stated) |
|---|---|---|---|
| **P31** | 2-(2-(cyclopropanesulfonamido)pyrimidin-4-yl)-N-(5-(6-ethoxypyrazin-2-yl)pyridin-2-yl)-2-methylpropanamide 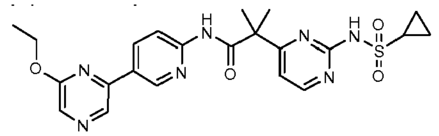 | Method 2, using INTC21 and INTD33, [UPLC Acidic], 484, (1.37) | 11.25 (s, 1H), 10.16 (s, 1H), 9.09 - 8.98 (m, 1H), 8.84 (s, 1H), 8.59 (d, J = 5.3 Hz, 1H), 8.52 - 8.46 (m, 1H), 8.25 (s, 1H), 8.23 - 8.18 (m, 1H), 7.20 (d, J = 5.3 Hz, 1H), 4.48 (q, J = 7.0 Hz, 2H), 3.24 - 3.11 (m, 1H), 1.61 (s, 6H), 1.40 (t, J = 7.0 Hz, 3H), 1.06 - 0.95 (m, 2H), 0.83 - 0.70 (m, 2H). |
| **P32** | 2-(2-(cyclopropanesulfonamido)pyrimidin-4-yl)-N-(5-(6-cyclopropoxypyrazin-2-yl)pyridin-2-yl)-2-methylpropanamide 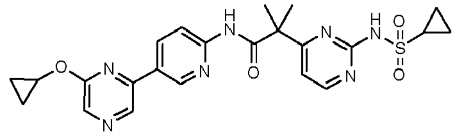 | Method 2, using INTC21 and INTD30, [UPLC acidic], 496, (1.39) | 11.30 (s, 1H), 10.20 (s, 1H), 9.05 (d, J = 2.4 Hz, 1H), 8.92 (s, 1H), 8.58 (d, J = 5.3 Hz, 1H), 8.55 - 8.49 (m, 1H), 8.31 (s, 1H), 8.21 (d, J = 8.8 Hz, 1H), 7.18 (d, J = 5.3 Hz, 1H), 4.48 - 4.35 (m, 1H), 3.23 - 3.10 (m, 1H), 1.61 (s, 6H), 1.06 - 0.94 (m, 2H), 0.92 - 0.68 (m, 6H). |
| **P33** | N-(2-chloro-4-(6-ethoxypyrazin-2-yl)phenyl)-2-(2-(cyclopropanesulfonamido)pyrimidin-4-yl)-2-methylpropanamide 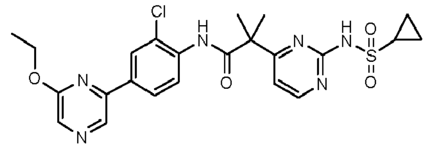 | Method 2, using INTC21 and INTD49, [UPLC Acidic], 517, (1.53) | 11.30 (s, 1H), 9.24 (s, 1H), 8.85 (s, 1H), 8.63 (d, J = 5.3 Hz, 1H), 8.27 - 8.20 (m, 2H), 8.10 (dd, J = 8.5, 2.1 Hz, 1H), 7.78 (d, J = 8.4 Hz, 1H), 7.25 (d, J = 5.3 Hz, 1H), 4.48 (q, J = 7.0 Hz, 2H), 3.31 - 3.20 (m, 1H), 1.63 (s, 6H), 1.40 (t, J = 7.0 Hz, 3H), 1.17 - 1.07 (m, 2H), 1.03 - 0.95 (m, 2H). |
| **P34** | N-(2-cyano-4-(6-ethoxypyrazin-2-yl)phenyl)-2-(2-(cyclopropanesulfonamido)pyrimidin-4-yl)-2-methylpropanamide 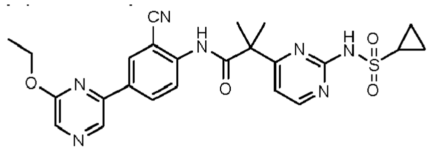 | Method 2, using INTC21 and INTD51, [UPLC Acidic], 508, (1.45) | 11.28 (s, 1H), 9.85 (s, 1H), 8.90 (s, 1H), 8.62 (d, J = 5.3 Hz, 1H), 8.55 (d, J = 2.1 Hz, 1H), 8.43 (dd, J = 8.6, 2.1 Hz, 1H), 8.29 (s, 1H), 7.69 - 7.62 (m, 1H), 7.25 (d, J = 5.3 Hz, 1H), 4.50 (q, J = 7.0 Hz, 2H), 3.28 - 3.19 (m, 1H), 1.63 (s, 6H), 1.40 (t, J = 7.0 Hz, 3H), 1.17 - 1.08 (m, 2H), 1.04 - 0.96 (m, 2H). |
| **P35** | 2-(2-(cyclopropanesulfonamido)pyrimidin-4-yl)-N-(2-fluoro-4-(5-isopropoxypyridin-3-yl) phenyl)-2-methylpropanamide 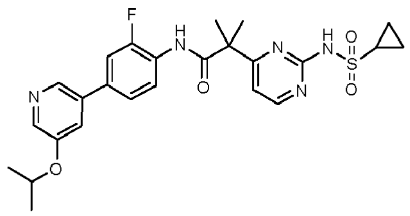 | Method 2, using INTC21 and INTD12, [UPLC acidic], 514, (1.25) | 11.52 (s, 1H), 9.48 (s, 1H), 8.57 (d, J = 5.3 Hz, 1H), 8.53 - 8.46 (m, 1H), 8.25 (d, J = 2.7 Hz, 1H), 7.75 - 7.53 (m, 4H), 7.12 (d, J = 5.3 Hz, 1H), 4.99 - 4.78 (m, 1H), 3.26 - 3.16 (m, 1H), 1.60 (s, 6H), 1.32 (d, J = 6.0 Hz, 6H), 1.12 - 1.03 (m, 2H), 1.00 - 0.89 (m, 2H). |

(continued)

| P# | Name/Structure (All examples containing chiral centres are racemates unless stated) | Synthesis Method, [LCMS Method], m/z(M+H)$^+$, (RT/Min) | $^1$H NMR Chemical Shift Data (DMSO-d6 unless stated) |
|---|---|---|---|
| P36 | 2-(2-(cyclopropanesulfonamido)pyrimidin-4-yl)-N-(2-fluoro-4-(pyridin-3-yl)phenyl)-2-methylpropanamide 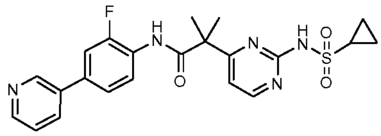 | Method 2, using INTC21 and INTD50, [HPLC Acidic], 456, (1.37) | 11.41 (s, 1H), 9.41 (s, 1H), 8.97 - 8.90 (m, 1H), 8.62 - 8.55 (m, 2H), 8.15 - 8.09 (m, 1H), 7.72 - 7.54 (m, 3H), 7.52 - 7.46 (m, 1H), 7.16 (d, J = 5.3 Hz, 1H), 3.27 - 3.19 (m, 1H), 1.61 (s, 6H), 1.15 - 1.05 (m, 2H), 1.02 - 0.90 (m, 2H). |
| P37 | 2-(2-(cyclopropanesulfonamido)pyrimidin-4-yl)-N-(2-fluoro-4-(6-(trifluoromethyl)pyrazin-2-yl)phenyl)-2-methylpropanamide 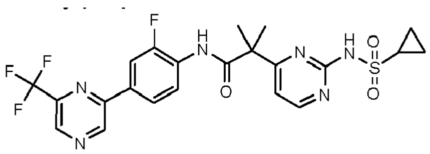 | Method 2, using INTC21 and INTD25, [UPLC Acidic], 525, (1.47) | 11.30 (s, 1H), 9.65 (s, 1H), 9.47 (s, 1H), 9.15 (s, 1H), 8.61 (d, J = 5.3 Hz, 1H), 8.13 - 8.02 (m, 2H), 7.85 - 7.77 (m, 1H), 7.21 (d, J = 5.3 Hz, 1H), 3.28 - 3.21 (m, 1H), 1.62 (s, 6H), 1.16 - 1.05 (m, 2H), 1.03 - 0.92 (m, 2H). |
| P38 | 2-(2-(cyclopropanesulfonamido)pyrimidin-4-yl)-N-(4-(6-ethoxypyrazin-2-yl)-2-fluorophenyl)-2-methylpropanamide 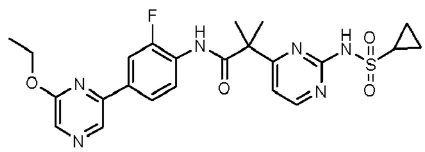 | Method 2, using INTC21 and INTD24; [UPLC acidic], 501, (1.46) | 11.32 (s, 1H), 9.41 (s, 1H), 8.84 (s, 1H), 8.70 - 8.51 (m, 1H), 8.25 (s, 1H), 8.06 - 7.90 (m, 2H), 7.77 - 7.63 (m, 1H), 7.28 - 7.13 (m, 1H), 4.56 - 4.42 (m, 2H), 3.28 - 3.19 (m, 1H), 1.61 (s, 6H), 1.47 - 1.32 (m, 3H), 1.18 - 1.05 (m, 2H), 1.03 - 0.91 (m, 2H). |
| P39 | 2-(2-(cyclopropanesulfonamido)pyrimidin-4-yl)-N-(2-fluoro-4-(6-isopropoxypyrazin-2-yl) phenyl)-2-methylpropanamide 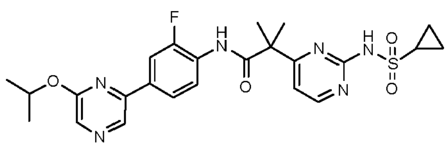 | Method 2, using INTC21 and INTD35, [UPLC acidic], 515, (1.54) | 11.33 (s, 1H), 9.41 (s, 1H), 8.82 (s, 1H), 8.61 (d, J = 5.3 Hz, 1H), 8.19 (s, 1H), 8.04 - 7.90 (m, 2H), 7.79 - 7.63 (m, 1H), 7.19 (d, J = 5.3 Hz, 1H), 5.51 - 5.33 (m, 1H), 3.29 - 3.17 (m, 1H), 1.61 (s, 6H), 1.39 (d, J = 6.1 Hz, 6H), 1.15 - 1.06 (m, 2H), 1.02 - 0.92 (m, 2H). |
| P40 | 2-(2-(cyclopropanesulfonamido)pyrimidin-4-yl)-N-(4-(6-ethoxypyrazin-2-yl)-2-fluoro-5-methylphenyl)-2-methylpropanamide 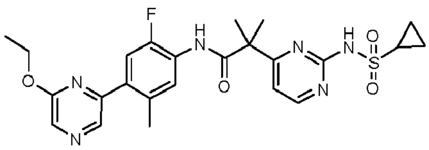 | Method 2, using INTC21 and INTD39, [UPLC Acidic], 515, (1.46) | 11.29 (s, 1H), 9.34 (s, 1H), 8.61 (d, J = 5.3 Hz, 1H), 8.41 (s, 1H), 8.27 (s, 1H), 7.48 (d, J = 7.7 Hz, 1H), 7.42 (d, J = 11.2 Hz, 1H), 7.19 (d, J = 5.3 Hz, 1H), 4.39 (q, J = 7.1 Hz, 2H), 3.30 - 3.21 (m, 1H), 2.37 (s, 3H), 1.61 (s, 6H), 1.37 (t, J = 7.0 Hz, 3H), 1.14 - 1.08 (m, 2H), 1.03 - 0.96 (m, 2H). |

(continued)

| P# | Name/Structure (All examples containing chiral centres are racemates unless stated) | Synthesis Method, [LCMS Method], m/z(M+H)$^+$, (RT/Min) | $^1$H NMR Chemical Shift Data (DMSO-d6 unless stated) |
|---|---|---|---|
| **P41** | 2-(2-(cyclopropanesulfonamido)pyrimidin-4-yl)-N-(4-(6-ethoxypyrazin-2-yl)-2,6-difluorophenyl)-2-methylpropanamide 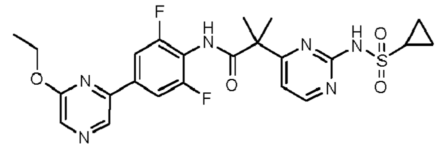 | Method 2, using INTC21 and INTD41, [UPLC Acidic], 519, (1.42) | 11.29 (s, 1H), 9.47 (s, 1H), 8.90 (s, 1H), 8.62 (d, J = 5.3 Hz, 1H), 8.30 (s, 1H), 7.92 (d, J = 8.9 Hz, 2H), 7.16 (d, J = 5.3 Hz, 1H), 4.49 (q, J = 7.0 Hz, 2H), 3.31 - 3.22 (m, 1H), 1.61 (s, 6H), 1.39 (t, J = 7.0 Hz, 3H), 1.15 - 1.08 (m, 2H), 1.10 - 0.99 (m, 2H). |
| **P42** | 2-(2-(cyclopropanesulfonamido)pyrimidin-4-yl)-N-(2-fluoro-4-(pyrazin-2-yl)phenyl)-2-methylpropanamide 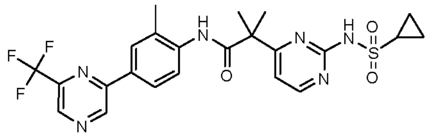 | Method 2, using INTC21 and INTD23, [UPLC Acidic], 457, (1.14) | 11.30 (s, 1H), 9.40 (s, 1H), 9.30 (d, J = 1.5 Hz, 1H), 8.74 - 8.69 (m, 1H), 8.67 - 8.56 (m, 2H), 8.07 - 7.95 (m, 2H), 7.73 (t, J = 8.1 Hz, 1H), 7.20 (d, J = 5.3 Hz, 1H), 3.28 - 3.19 (m, 1H), 1.61 (s, 6H), 1.16 - 1.03 (m, 2H), 1.02 - 0.92 (m, 2H). |
| **P43** | 2-(2-(cyclopropanesulfonamido)pyrimidin-4-yl)-2-methyl-N-(2-methyl-4-(6-(trifluoromethyl) pyrazin-2-yl)phenyl)propanamide 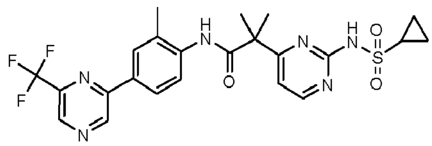 | Method 2, using INTC21 and INTD34; [UPLC Acidic], 521, (1.45) | 11.28 (s, 1H), 9.60 (s, 1H), 9.12 - 9.05 (m, 2H), 8.62 (d, J = 5.3 Hz, 1H), 8.09 - 7.99 (m, 2H), 7.52 (d, J = 8.3 Hz, 1H), 7.23 (d, J = 5.3 Hz, 1H), 3.31 - 3.21 (m, 1H), 2.21 (s, 3H), 1.62 (s, 6H), 1.17 - 1.06 (m, 2H), 1.04 - 0.96 (m, 2H). |
| **P44** | 2-(2-(cyclopropanesulfonamido)pyrimidin-4-yl)-N-(4-(6-ethoxypyrazin-2-yl)-2,3-dimethylphenyl)-2-methylpropanamide 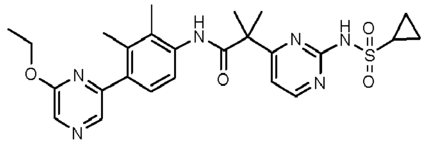 | Method 2, using INTC21 and INTD38, [UPLC Acidic], 512, (1.39) | 11.28 (s, 1H), 9.16 (s, 1H), 8.62 (d, J = 5.3 Hz, 1H), 8.26 (d, J = 11.0 Hz, 2H), 7.26 - 7.19 (m, 2H), 7.15 (d, J = 8.2 Hz, 1H), 4.37 (q, J = 7.1 Hz, 2H), 3.31 - 3.23 (m, 1H), 2.23 (s, 3H), 2.05 (s, 3H), 1.63 (s, 6H), 1.36 (t, J = 7.0 Hz, 3H), 1.16 - 1.09 (m, 2H), 1.08 - 0.99 (m, 2H). |
| **P45** | 2-(2-(cyclopropanesulfonamido)pyrimidin-4-yl)-N-(4-(6-ethoxypyrazin-2-yl)-5-fluoro-2-methylphenyl)-2-methylpropanamide 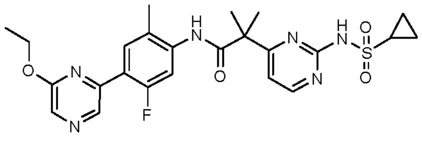 | Method 2, using INTC21 and INTD36; [UPLC Acidic], 515, (1.50) | 11.29 (s, 1H), 9.08 (s, 1H), 8.65 - 8.55 (m, 2H), 8.25 (d, J = 2.9 Hz, 1H), 7.82 (dd, J = 8.6, 2.5 Hz, 1H), 7.42 (dd, J = 12.9, 2.6 Hz, 1H), 7.27 - 7.21 (m, 1H), 4.50 - 4.41 (m, 2H), 3.26 - 3.20 (m, 1H), 2.16 (s, 3H), 1.61 (s, 6H), 1.42 - 1.35 (m, 3H), 1.14 - 1.05 (m, 2H), 1.03 - 0.97 (m, 2H). |

(continued)

| P# | Name/Structure (All examples containing chiral centres are racemates unless stated) | Synthesis Method, [LCMS Method], m/z(M+H)+, (RT/Min) | $^1$H NMR Chemical Shift Data (DMSO-d6 unless stated) |
|---|---|---|---|
| **P46** | 2-(2-(cyclopropanesulfonamido)pyrimidin-4-yl)-N-(4-(6-ethoxypyrazin-2-yl)-2,5-dimethylphenyl)-2-methylpropanamide 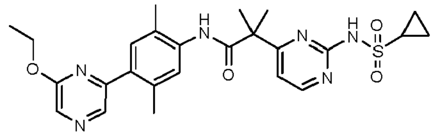 | Method 2, using INTC21 and INTD40, [UPLC Acidic], 511, (1.44) | 11.28 (s, 1H), 9.01 (s, 1H), 8.62 (d, J = 5.3 Hz, 1H), 8.35 (s, 1H), 8.23 (s, 1H), 7.34 (s, 1H), 7.25 - 7.20 (m, 2H), 4.38 (q, J = 7.0 Hz, 2H), 3.31 - 3.22 (m, 1H), 2.34 (s, 3H), 2.10 (s, 3H), 1.62 (s, 6H), 1.36 (t, J = 7.0 Hz, 3H), 1.16 - 1.09 (m, 2H), 1.05 - 0.98 (m, 2H). |
| **P47** | 2-(2-(cyclopropanesulfonamido)pyrimidin-4-yl)-N-(4-(6-ethoxypyrazin-2-yl)-2-(trifluoromethoxy)phenyl)-2-methylpropanamide 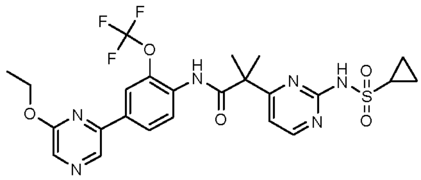 | Method 2, using INTC21 and INTD47, [UPLC Acidic], 567, (1.59) | 11.28 (s, 1H), 9.36 (s, 1H), 8.87 (s, 1H), 8.61 (d, J = 5.3 Hz, 1H), 8.27 (s, 1H), 8.16 (dd, J = 8.5, 2.0 Hz, 1H), 8.12 - 8.07 (m, 1H), 7.78 (d, J = 8.4 Hz, 1H), 7.21 (d, J = 5.2 Hz, 1H), 4.48 (q, J = 7.0 Hz, 2H), 3.27 - 3.21 (m, 1H), 1.61 (s, 6H), 1.40 (t, J = 7.0 Hz, 3H), 1.15 - 1.08 (m, 2H), 1.03 - 0.96 (m, 2H). |
| **P48** | 2-(2-(cyclopropanesulfonamido)pyrimidin-4-yl)-N-(4-(6-ethoxypyrazin-2-yl)-5-fluoro-2-methoxyphenyl)-2-methylpropanamide 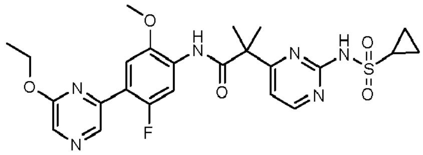 | Method 2, using INTC21 and INTD37, [UPLC Acidic], 531, (1.59) | 11.36 (s, 1H), 9.01 (s, 1H), 8.66 - 8.58 (m, 2H), 8.25 (s, 1H), 8.00 (d, J = 13.1 Hz, 1H), 7.59 (d, J = 6.9 Hz, 1H), 7.26 (d, J = 5.3 Hz, 1H), 4.51 - 4.42 (m, 2H), 3.89 (s, 3H), 3.23 - 3.14 (m, 1H), 1.63 (s, 6H), 1.43 - 1.36 (m, 3H), 1.13 - 1.08 (m, 2H), 1.00 - 0.94 (m, 2H). |
| **P49** | 2-(2-(cyclopropanesulfonamido)pyrimidin-4-yl)-N-(4-(6-ethoxypyrazin-2-yl)-2-methoxyphenyl)-2-methylpropanamide 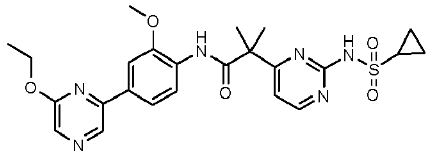 | Method 2, using INTC21 and INTD48; [UPLC Acidic], 513, (1.48) | 11.34 (s, 1H), 8.86 - 8.81 (m, 2H), 8.63 (d, J = 5.3 Hz, 1H), 8.21 - 8.17 (m, 1H), 8.05 (d, J = 8.7 Hz, 1H), 7.74 - 7.69 (m, 2H), 7.26 (d, J = 5.3 Hz, 1H), 4.47 (q, 2H), 3.91 (s, 3H), 3.26 - 3.17 (m, 1H), 1.63 (s, 6H), 1.39 (t, J = 7.0 Hz, 3H), 1.13 - 1.06 (m, 2H), 0.99 - 0.91 (m, 2H). |
| **P50** | 2-(2-(cyclopropanesulfonamido)pyrimidin-4-yl)-2-methyl-N-(4-(pyrimidin-5-yl)phenyl) propanamide 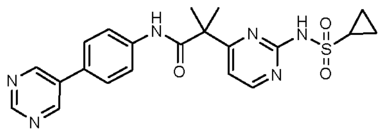 | Method 2, using INTC21 and a commercial aniline, [UPLC Acidic], 439, (1.04) | 11.25 (s, 1H), 9.46 (s, 1H), 9.17 - 9.09 (m, 3H), 8.59 (d, J = 5.3 Hz, 1H), 7.81 - 7.73 (m, 4H), 3.24 - 3.14 (m, 1H), 1.59 (s, 6H), 1.07 - 0.99 (m, 2H), 0.85 - 0.77 (m, 2H). 1 exchangeable proton not observed. |

(continued)

| P# | Name/Structure (All examples containing chiral centres are racemates unless stated) | Synthesis Method, [LCMS Method], m/z(M+H)$^+$, (RT/Min) | $^1$H NMR Chemical Shift Data (DMSO-d6 unless stated) |
|---|---|---|---|
| **P51** | N-(4-(5-chloropyridin-3-yl)phenyl)-2-(2-(cyclopropanesulfonamido)pyrimidin-4-yl)-2-methylpropanamide 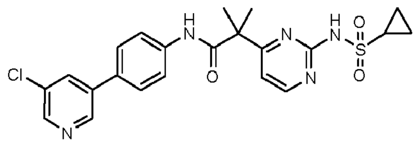 | Method 2, using INTC21 and INTD8; [UPLC acidic], 472, (1.36) | 11.30 (s, 1H), 9.48 (s, 1H), 8.86 (d, J = 2.0 Hz, 1H), 8.63 - 8.55 (m, 2H), 8.23 (t, J = 2.2 Hz, 1H), 7.76 (s, 4H), 7.18 (d, J = 5.3 Hz, 1H), 3.25 - 3.13 (m, 1H), 1.59 (s, 6H), 1.09 - 0.98 (m, 2H), 0.85 - 0.75 (m, 2H). |
| **P52** | N-(4-(5-cyanopyridin-3-yl)phenyl)-2-(2-(cyclopropanesulfonamido)pyrimidin-4-yl)-2-methylpropanamide 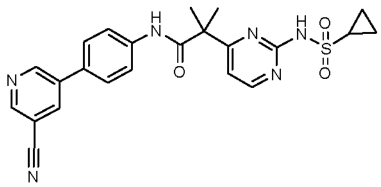 | Method 2, using INTC21 and INTD5, [HPLC acidic], 463, (2.46) | 11.34 (s, 1H), 9.52 (s, 1H), 9.19 (d, J = 2.3 Hz, 1H), 8.96 (d, J = 1.9 Hz, 1H), 8.64 (t, J = 2.1 Hz, 1H), 8.59 (d, J = 5.3 Hz, 1H), 7.83 - 7.73 (m, 4H), 7.17 (d, J = 5.3 Hz, 1H), 3.26 - 3.13 (m, 1H), 1.59 (s, 6H), 1.08 - 0.98 (m, 2H), 0.86 - 0.74 (m, 2H). |
| **P53** | 2-(2-(cyclopropanesulfonamido)pyrimidin-4-yl)-N-(4-(5-fluoropyridin-3-yl)phenyl)-2-methylpropanamide 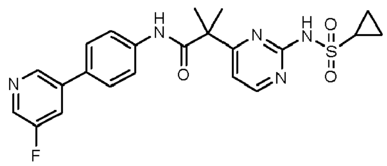 | Method 2, using INTC21 and INTD6, [UPLC acidic], 456, (1.25) | 11.30 (s, 1H), 9.48 (s, 1H), 8.88 - 8.76 (m, 1H), 8.60 (d, J = 5.3 Hz, 1H), 8.53 (d, J = 2.7 Hz, 1H), 8.09 - 8.00 (m, 1H), 7.76 (s, 4H), 7.19 (d, J = 5.3 Hz, 1H), 3.25 - 3.15 (m, 1H), 1.60 (s, 6H), 1.08 - 0.99 (m, 2H), 0.87 - 0.74 (m, 2H). |
| **P54** | 2-(2-(cyclopropanesulfonamido)pyrimidin-4-yl)-2-methyl-N-(4-(5-methylpyridin-3-yl)phenyl) propanamide 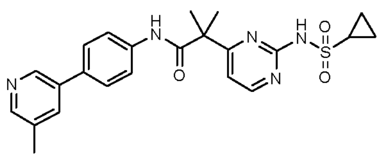 | Method 2, using INTC21 and INTD10, [HPLC acidic], 452, (1.26) | 11.29 (s, 1H), 9.43 (s, 1H), 8.67 (d, J = 2.2 Hz, 1H), 8.60 (d, J = 5.3 Hz, 1H), 8.39 - 8.35 (m, 1H), 7.90 - 7.86 (m, 1H), 7.77 - 7.62 (m, 4H), 7.18 (d, J = 5.3 Hz, 1H), 3.28 - 3.11 (m, 1H), 2.36 (s, 3H), 1.60 (s, 6H), 1.09 - 0.97 (m, 2H), 0.86 - 0.74 (m, 2H). |
| **P55** | 2-(2-(cyclopropanesulfonamido)pyrimidin-4-yl)-N-(4-(5-(difluoromethoxy)pyridin-3-yl) phenyl)-2-methylpropanamide 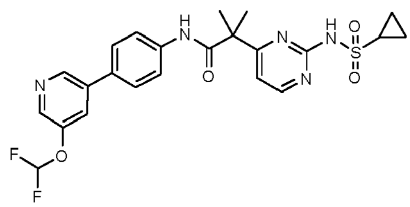 | Method 2, using INTC21 and INTD3, [UPLC acidic], 504, (1.32) | 11.29 (s, 1H), 9.51 (s, 1H), 8.80 (d, J = 1.9 Hz, 1H), 8.59 (d, J = 5.3 Hz, 1H), 8.44 (d, J = 2.6 Hz, 1H), 7.95 - 7.89 (m, 1H), 7.81 - 7.71 (m, 4H), 7.34 (t, J = 73.5 Hz, 1H), 7.16 (d, J = 5.3 Hz, 1H), 3.25 - 3.13 (m, 1H), 1.60 (s, 6H), 1.08 - 0.98 (m, 2H), 0.87 - 0.74 (m, 2H). |

(continued)

| P# | Name/Structure (All examples containing chiral centres are racemates unless stated) | Synthesis Method, [LCMS Method], m/z(M+H)+, (RT/Min) | $^1$H NMR Chemical Shift Data (DMSO-d6 unless stated) |
|---|---|---|---|
| **P56** | 2-(2-(cyclopropanesulfonamido)pyrimidin-4-yl)-N-(4-(5-methoxypyridin-3-yl)phenyl)-2-methylpropanamide 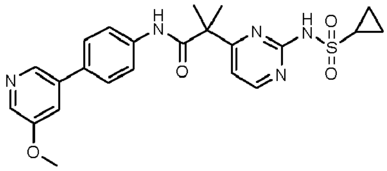 | Method 2, using INTC21 and INTD45, [HPLC acidic], 468, (1.89) | 11.34 (s, 1H), 9.46 (s, 1H), 8.60 (d, J = 5.3 Hz, 1H), 8.47 (d, J = 1.9 Hz, 1H), 8.25 (d, J = 2.8 Hz, 1H), 7.79 - 7.67 (m, 4H), 7.63 - 7.56 (m, 1H), 7.17 (d, J = 5.3 Hz, 1H), 3.91 (s, 3H), 3.26 - 3.14 (m, 1H), 1.60 (s, 6H), 1.09 - 0.96 (m, 2H), 0.88 - 0.75 (m, 2H). |
| **P57** | 2-(2-(cyclopropanesulfonamido)pyrimidin-4-yl)-N-(4-(5-ethoxypyridin-3-yl)phenyl)-2-methylpropanamide 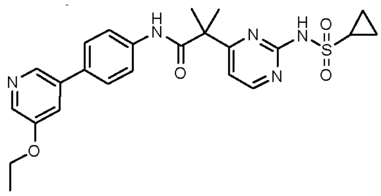 | Method 2, using INTC21 and INTD4, [UPLC acidic], 482, (1.06) | 11.29 (s, 1H), 9.43 (s, 1H), 8.60 (d, J = 5.3 Hz, 1H), 8.46 (d, J = 1.9 Hz, 1H), 8.23 (d, J = 2.7 Hz, 1H), 7.80 - 7.64 (m, 4H), 7.60 - 7.54 (m, 1H), 7.18 (d, J = 5.3 Hz, 1H), 4.20 (q, J = 7.0 Hz, 2H), 3.26 - 3.13 (m, 1H), 1.60 (s, 6H), 1.38 (t, J = 7.0 Hz, 3H), 1.10 - 0.96 (m, 2H), 0.88 - 0.75 (m, 2H). |
| **P58** | 2-(2-(cyclopropanesulfonamido)pyrimidin-4-yl)-N-(4-(5-isopropoxypyridin-3-yl)phenyl)-2-methylpropanamide 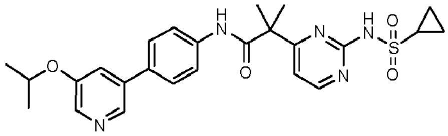 | Method 2, using INTC21 and INTD11, [UPLC acidic], 496, (1.16) | 11.34 (s, 1H), 9.45 (s, 1H), 8.59 (d, J = 5.2 Hz, 1H), 8.44 (d, J = 1.9 Hz, 1H), 8.21 (d, J = 2.7 Hz, 1H), 7.78 - 7.65 (m, 4H), 7.59 - 7.53 (m, 1H), 7.16 (d, J = 5.3 Hz, 1H), 4.92 - 4.76 (m, 1H), 3.27 - 3.13 (m, 1H), 1.60 (s, 6H), 1.32 (d, J = 6.0 Hz, 6H), 1.08 - 0.98 (m, 2H), 0.87 - 0.73 (m, 2H). |
| **P59** | 2-(2-(cyclopropanesulfonamido)pyrimidin-4-yl)-2-methyl-N-(4-(pyridin-3-yl)phenyl) propanamide 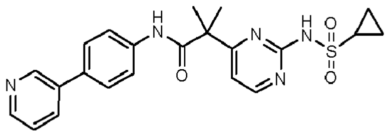 | Method 2, using INTC21 and a commercial aniline, [HPLC acidic], 438, (1.28) | 9.44 (s, 1H), 8.89 - 8.85 (m, 1H), 8.60 (d, J = 5.3 Hz, 1H), 8.53 (dd, J = 4.7, 1.6 Hz, 1H), 8.15 (s, 1H), 8.07 - 8.03 (m, 1H), 7.80 - 7.61 (m, 4H), 7.49 - 7.41 (m, 1H), 7.18 (d, J = 5.3 Hz, 1H), 3.26 - 3.15 (m, 1H), 1.60 (s, 6H), 1.08 - 0.99 (m, 2H), 0.89 - 0.73 (m, 2H). |
| **P60** | 2-(2-(cyclopropanesulfonamido)pyrimidin-4-yl)-2-methyl-N-(3'-(trifluoromethyl)-[1,1'-biphenyl]-4-yl)propanamide 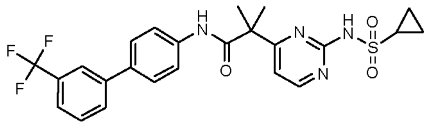 | Method 2, using INTC21 and a commercial aniline; [UPLC Acidic], 505, (1.65) | 11.25 (s, 1H), 9.42 (s, 1H), 8.59 (d, J = 5.3 Hz, 1H), 7.99 - 7.91 (m, 2H), 7.77 - 7.63 (m, 6H), 7.18 (d, J = 5.3 Hz, 1H), 3.24 - 3.15 (m, 1H), 1.58 (s, 6H), 1.06 - 0.98 (m, 2H), 0.82 - 0.75 (m, 2H). |

(continued)

| P# | Name/Structure (All examples containing chiral centres are racemates unless stated) | Synthesis Method, [LCMS Method], m/z (M+H)$^+$, (RT/Min) | $^1$H NMR Chemical Shift Data (DMSO-d6 unless stated) |
|---|---|---|---|
| **P61** | N-(3'-chloro-[1,1'-biphenyl]-4-yl)-2-(2-(cyclopropanesulfonamido)pyrimidin-4-yl)-2-methylpropanamide 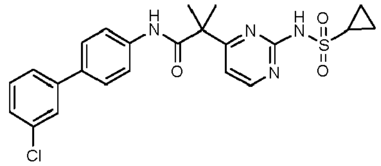 | Method 2, using INTC21 and a commercial aniline, [UPLC Acidic], 471, (1.63) | 11.26 (s, 1H), 9.41 (s, 1H), 8.59 (d, J = 5.3 Hz, 1H), 7.75 - 7.69 (m, 3H), 7.67 - 7.60 (m, 3H), 7.46 (td, J = 7.8, 1.6 Hz, 1H), 7.38 (d, J = 8.0 Hz, 1H), 7.18 (d, J = 5.3 Hz, 1H), 3.24 - 3.15 (m, 1H), 1.59 (s, 6H), 1.06 - 0.99 (m, 2H), 0.83 - 0.75 (m, 2H). |
| **P62** | N-(3'-cyano-[1,1'-biphenyl]-4-yl)-2-(2-(cyclopropanesulfonamido)pyrimidin-4-yl)-2-methylpropanamide 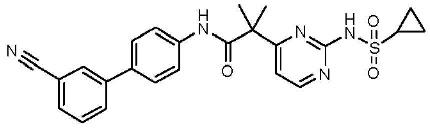 | Method 2, using INTC21 and a commercial aniline, [UPLC Acidic], 463, (1.42) | 11.27 (s, 1H), 9.42 (s, 1H), 8.59 (d, J = 5.3 Hz, 1H), 8.13 (d, J = 1.7 Hz, 1H), 8.03 - 7.97 (m, 1H), 7.80 - 7.69 (m, 5H), 7.63 (t, J = 7.8 Hz, 1H), 7.18 (d, J = 5.3 Hz, 1H), 3.24 - 3.15 (m, 1H), 1.59 (s, 6H), 1.06 - 0.99 (m, 2H), 0.83 - 0.75 (m, 2H). |
| **P63** | 2-(2-(cyclopropanesulfonamido)pyrimidin-4-yl)-N-(3'-ethoxy-[1,1'-biphenyl]-4-yl)-2-methylpropanamide 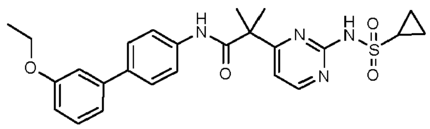 | Method 2, using INTC21 and INTD42, [UPLC Acidic], 481, (1.58) | 11.26 (s, 1H), 9.38 (s, 1H), 8.60 (d, J = 5.3 Hz, 1H), 7.72 - 7.66 (m, 2H), 7.63 - 7.57 (m, 2H), 7.37 - 7.30 (m, 1H), 7.22 - 7.12 (m, 3H), 6.88 (dd, J = 8.2, 2.5 Hz, 1H), 4.09 (q, J = 6.9 Hz, 2H), 3.25 - 3.16 (m, 1H), 1.59 (s, 6H), 1.35 (t, J = 6.9 Hz, 3H), 1.07 - 1.00 (m, 2H), 0.84 - 0.76 (m, 2H). |
| **P64** | 2-(2-(cyclopropanesulfonamido)pyrimidin-4-yl)-2-methyl-N-(4-(6-(trifluoromethyl)pyrazin-2-yl)phenyl)propanamide 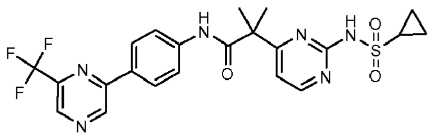 | Method 2, using INTC21 and INTD19, [HPLC acidic], 507, (2.25) | 11.27 (s, 1H), 9.59 (d, J = 5.0 Hz, 2H), 9.07 (s, 1H), 8.61 (d, J = 5.3 Hz, 1H), 8.22 - 8.13 (m, 2H), 7.90 - 7.78 (m, 2H), 7.20 (d, J = 5.3 Hz, 1H), 3.25 - 3.15 (m, 1H), 1.60 (s, 6H), 1.06 - 0.98 (m, 2H), 0.85 - 0.75 (m, 2H). |
| **P65** | 2-(2-(cyclopropanesulfonamido)pyrimidin-4-yl)-N-(4-(6-ethoxypyrazin-2-yl)phenyl)-2-methylpropanamide 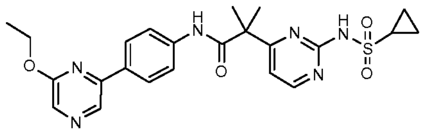 | Method 2, using INTC21 and INTD18, [HPLC acidic], 483, (2.19) | 11.27 (s, 1H), 9.50 (s, 1H), 8.76 (d, J = 0.5 Hz, 1H), 8.61 (d, J = 5.3 Hz, 1H), 8.18 (d, J = 0.5 Hz, 1H), 8.12 - 8.02 (m, 2H), 7.83 - 7.73 (m, 2H), 7.19 (d, J = 5.3 Hz, 1H), 4.48 (q, J = 7.0 Hz, 2H), 3.26 - 3.15 (m, 1H), 1.60 (s, 6H), 1.40 (t, J = 7.0 Hz, 3H), 1.08 - 1.00 (m, 2H), 0.87 - 0.75 (m, 2H). |

(continued)

| P# | Name/Structure (All examples containing chiral centres are racemates unless stated) | Synthesis Method, [LCMS Method], m/z(M+H)+, (RT/Min) | $^1$H NMR Chemical Shift Data (DMSO-d6 unless stated) |
|---|---|---|---|
| **P66** | 2-(2-(cyclopropanesulfonamido)pyrimidin-4-yl)-N-(4-(6-cyclopropoxypyrazin-2-yl)phenyl)-2-methylpropanamide 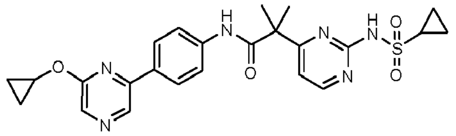 | Method 2, using INTC21 and INTD21, [UPLC acidic], 495, (1.44) | 11.32 (s, 1H), 9.53 (s, 1H), 8.84 (s, 1H), 8.60 (d, J = 5.3 Hz, 1H), 8.23 (s, 1H), 8.14 - 8.05 (m, 2H), 7.83 - 7.74 (m, 2H), 7.19 (d, J = 5.3 Hz, 1H), 4.47 - 4.33 (m, 1H), 3.27 - 3.12 (m, 1H), 1.60 (s, 6H), 1.09 - 0.96 (m, 2H), 0.93 - 0.69 (m, 6H). |
| **P67** | 2-(2-(cyclopropanesulfonamido)pyrimidin-4-yl)-N-(4-(6-isopropoxypyrazin-2-yl)phenyl)-2-methylpropanamide 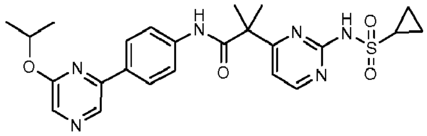 | Method 2, using INTC21 and INTD20, [UPLC acidic], 497, (1.53) | 11.34 (s, 1H), 9.52 (s, 1H), 8.73 (s, 1H), 8.60 (d, J = 5.2 Hz, 1H), 8.12 (s, 1H), 8.08 - 8.03 (m, 2H), 7.80 - 7.75 (m, 2H), 7.18 (d, J = 5.3 Hz, 1H), 5.47 - 5.35 (m, 1H), 3.25 - 3.12 (m, 1H), 1.60 (s, 6H), 1.38 (d, J = 6.2 Hz, 6H), 1.08 - 0.97 (m, 2H), 0.87 - 0.74 (m, 2H). |
| **P68** | 2-(2-(cyclopropanesulfonamido)-5-fluoropyrimidin-4-yl)-N-(4-(6-ethoxypyrazin-2-yl)phenyl)-2-methylpropanamide 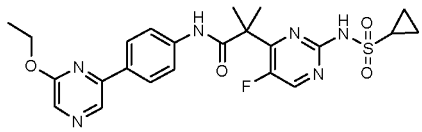 | Method 2, using INTC22 and INTD18, [UPLC Acidic], 501, (1.48) | 11.36 (s, 1H), 9.70 (s, 1H), 8.76 (s, 1H), 8.61 (s, 1H), 8.19 (s, 1H), 8.12 - 8.05 (m, 2H), 7.80 - 7.72 (m, 2H), 4.48 (q, J = 7.0 Hz, 2H), 3.27 - 3.16 (m, 1H), 1.61 (s, 6H), 1.40 (t, J = 7.0 Hz, 3H), 1.14 - 1.07 (m, 2H), 1.02 - 0.97 (m, 2H). |
| **P69** | N-(4-(6-ethoxypyrazin-2-yl)phenyl)-2-methyl-2-(2-((1-methylcyclopropane)-1-sulfonamido) pyrimidin-4-yl)propanamide 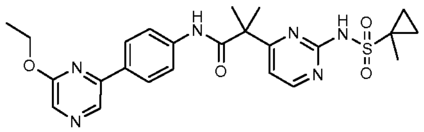 | Method 2, using INTC26 and INTD18, [UPLC Acidic], 497, (1.49) | 11.12 - 11.08 (m, 1H), 9.48 (s, 1H), 8.77 (s, 1H), 8.59 (d, J = 5.2 Hz, 1H), 8.18 (s, 1H), 8.10 - 8.04 (m, 2H), 7.79 - 7.74 (m, 2H), 7.16 (d, J = 5.2 Hz, 1H), 4.47 (q, J = 7.0 Hz, 2H), 1.58 (s, 6H), 1.50 - 1.46 (m, 2H), 1.43 - 1.36 (m, 6H), 0.84 - 0.80 (m, 2H). |
| **P70** | 2-(2-(cyclopropanesulfonamido)-5-methylpyrimidin-4-yl)-N-(4-(6-ethoxypyrazin-2-yl)phenyl)-2-methylpropanamide 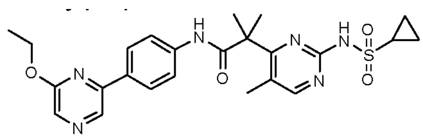 | Method 2, using INTC23 and INTD18; [UPLC Basic], 497, (1.27) | 11.07 (s, 1H), 9.69 (s, 1H), 8.76 (s, 1H), 8.34 (s, 1H), 8.18 (s, 1H), 8.10 - 8.05 (m, 2H), 7.79 - 7.74 (m, 2H), 4.47 (q, J = 7.1 Hz, 2H), 3.30 - 3.25 (m, 1H), 2.09 (s, 3H), 1.56 (s, 6H), 1.40 (t, J = 7.0 Hz, 3H), 1.14 - 1.09 (m, 2H), 1.06 - 1.01 (m, 2H). |
| **P71** | 2-(2-(cyclopropanesulfonamido)pyrimidin-4-yl)-2-methyl-N-(4-(pyrazin-2-yl)phenyl) propanamide 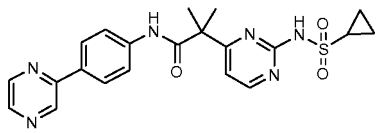 | Method 2, using INTC21 and INTD43, [HPLC acidic], 439, (1.72) | 11.26 (s, 1H), 9.50 (s, 1H), 9.22 (d, J = 1.5 Hz, 1H), 8.68 (dd, J = 2.5, 1.5 Hz, 1H), 8.61 (d, J = 5.3 Hz, 1H), 8.56 (d, J = 2.5 Hz, 1H), 8.15 - 8.04 (m, 2H), 7.85 - 7.74 (m, 2H), 7.20 (d, J = 5.3 Hz, 1H), 3.26 - 3.15 (m, 1H), 1.60 (s, 6H), 1.06 - 0.99 (m, 2H), 0.85 - 0.75 (m, 2H). |

(continued)

| P# | Name/Structure (All examples containing chiral centres are racemates unless stated) | Synthesis Method, [LCMS Method], m/z (M+H)$^+$, (RT/Min) | $^1$H NMR Chemical Shift Data (DMSO-d6 unless stated) |
|---|---|---|---|
| **P72** | N-(4-(6-ethoxypyrazin-2-yl)-2-fluorophenyl)-2-(2-(ethylsulfonamido)pyrimidin-4-yl)-2-methylpropanamide 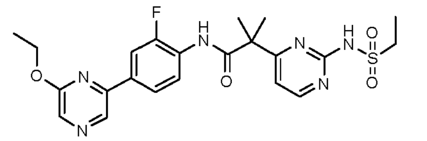 | Method 2 using INTC20 and INTD24, [UPLC Acidic], 489, (1.41) | 11.23 (s, 1H), 9.35 (s, 1H), 8.85 (s, 1H), 8.61 (d, J = 5.3 Hz, 1H), 8.25 (s, 1H), 8.03 - 7.93 (m, 2H), 7.70 (t, J = 8.1 Hz, 1H), 7.21 (d, J = 5.3 Hz, 1H), 4.49 (q, J = 7.0 Hz, 2H), 3.55 (q, J = 7.3 Hz, 2H), 1.60 (s, 6H), 1.40 (t, J = 7.0 Hz, 3H), 1.19 (t, J = 7.3 Hz, 3H). |
| **P73** | 2-(2-(ethylsulfonamido)pyrimidin-4-yl)-2-methyl-N-(4-(6-(trifluoromethyl)pyrazin-2-yl) phenyl)propanamide 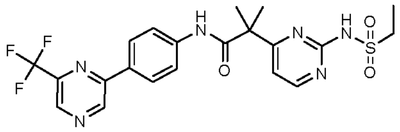 | Method 2, using INTC20 and INTD19, [UPLC Acidic], 495, (1.44) | 11.21 (s, 1H), 9.57 (d, J = 9.3 Hz, 2H), 9.07 (s, 1H), 8.61 (d, J = 5.3 Hz, 1H), 8.22 - 8.13 (m, 2H), 7.91 - 7.79 (m, 2H), 7.20 (d, J = 5.3 Hz, 1H), 3.49 (q, J = 7.3 Hz, 2H), 1.59 (s, 6H), 1.09 (t, J = 7.3 Hz, 3H). |
| **P74** | N-(4-(6-ethoxypyrazin-2-yl)phenyl)-2-(2-(ethylsulfonamido)pyrimidin-4-yl)-2-methylpropanamide 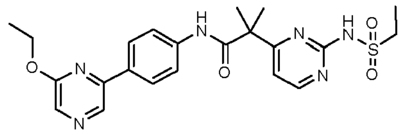 | Method 2, using INTC20 and INTD18, [UPLC Acidic], 471, (1.4) | 11.21 (s, 1H), 9.47 (s, 1H), 8.77 (s, 1H), 8.60 (d, J = 5.3 Hz, 1H), 8.18 (s, 1H), 8.11 - 8.04 (m, 2H), 7.82 -7.73 (m, 2H), 7.19 (d, J = 5.3 Hz, 1H), 4.48 (q, J = 7.0 Hz, 2H), 3.50 (q, J = 7.3 Hz, 2H), 1.59 (s, 6H), 1.40 (t, J = 7.0 Hz, 3H), 1.10 (t, J = 7.3 Hz, 3H). |
| **P75** | N-(5-(6-ethoxypyrazin-2-yl)-3-fluoropyridin-2-yl)-2-methyl-2-(2-(methylsulfonamido) pyrimidin-4-yl)propanamide 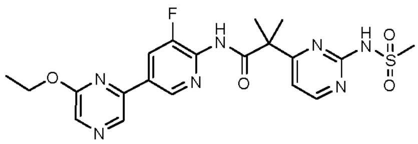 | Method 2, using INTC19 and INTD31, [HPLC Acidic], 476, (1.88) | 11.37 (s, 1H), 10.13 - 10.05 (m, 1H), 9.00 (s, 1H), 8.93 (d, J = 2.6 Hz, 1H), 8.62 (dd, J = 5.3, 2.3 Hz, 1H), 8.43 (dq, J = 10.9, 1.8 Hz, 1H), 8.35 - 8.30 (m, 1H), 7.17 (d, J = 5.1 Hz, 1H), 4.55-4.46 (m, 2H), 3.32 (s, 3H), 1.61 (d, J = 2.6 Hz, 6H), 1.45 - 1.37 (m, 3H) |
| **P76** | N-(5-(6-ethoxypyrazin-2-yl)pyridin-2-yl)-2-methyl-2-(2-(methylsulfonamido)pyrimidin-4-yl) propanamide 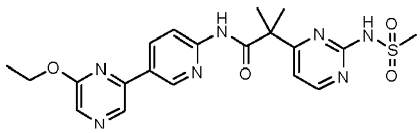 | Method 2, using INTC19 and INTD33, [HPLC Acidic], 458, (2.03) | 11.34 (s, 1H), 10.14 (s, 1H), 9.08-8.99 (m, 1H), 8.84 (s, 1H), 8.59 (d, J = 5.3 Hz, 1H), 8.49 (dd, J = 8.8, 2.5 Hz, 1H), 8.25 (s, 1H), 8.23 - 8.18 (m, 1H), 7.17 (d, J = 5.3 Hz, 1H), 4.48 (q, J = 7.0 Hz, 2H), 3.32 (s, 3H), 1.61 (s, 6H), 1.40 (t, J = 7.0 Hz, 3H) |
| **P77** | N-(2-fluoro-4-(5-isopropoxypyridin-3-yl) phenyl)-2-methyl-2-(2-(methylsulfonamido) pyrimidin-4-yl)propanamide 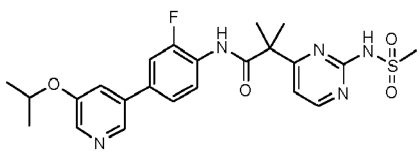 | Method 2, using INTC19 and INTD12, [HPLC Acidic], 488, (1.79) | 11.39 (s, 1H), 9.35 (s, 1H), 8.61 (d, J = 5.3 Hz, 1H), 8.49 (d, J = 1.9 Hz, 1H), 8.25 (d, J = 2.7 Hz, 1H), 7.72 - 7.68 (m, 1H), 7.66 (dd, J = 2.7, 1.9 Hz, 1H), 7.62 - 7.55 (m, 2H), 7.18 (d, J = 5.3 Hz, 1H), 4.91 - 4.82 (m, 1H), 3.37 (s, 3H), 1.60 (s, 6H), 1.32 (d, J = 6.0 Hz, 6H). |

(continued)

| P# | Name/Structure (All examples containing chiral centres are racemates unless stated) | Synthesis Method, [LCMS Method], m/z(M+H)$^+$, (RT/Min) | $^1$H NMR Chemical Shift Data (DMSO-d6 unless stated) |
|---|---|---|---|
| **P78** | N-(2-fluoro-4-(6-isopropoxypyrazin-2-yl) phenyl)-2-methyl-2-(2-(methylsulfonamido) pyrimidin-4-yl)propanamide 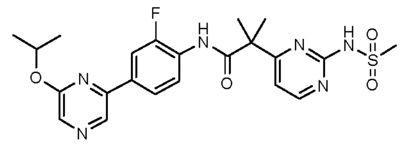 | Method 2, using INTC19 and INTD35, [HPLC Acidic], 489, (2.26) | 11.39 (s, 1H), 9.42 (s, 1H), 8.84 - 8.81 (m, 1H), 8.61 (d, J = 5.3 Hz, 1H), 8.19 (s, 1H), 8.01-7.94 (m, 2H), 7.67 (t, J = 8.1 Hz, 1H), 7.18 (d, J = 5.3 Hz, 1H), 5.45 - 5.39 (m, 1H), 3.36 (s, 3H), 1.61 (s, 6H), 1.39 (d, J = 6.2 Hz, 6H) |
| **P79** | 2-methyl-N-(2-methyl-4-(6-(trifluoromethyl) pyrazin-2-yl)phenyl)-2-(2-(methylsulfonamido) pyrimidin-4-yl)propanamide 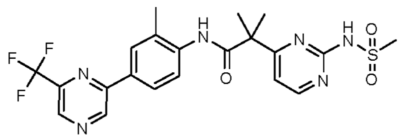 | Method 2 using INTC19 and INTD34; [HPLC Acidic], 495, (2.16) | 11.37 (s, 1H), 9.60 (s, 1H), 9.11 (d, J = 4.4 Hz, 2H), 8.63 (d, J = 5.3 Hz, 1H), 8.07 (d, J = 2.1 Hz, 1H), 8.02 (dd, J = 8.3, 2.2 Hz, 1H), 7.51 (d, J = 8.3 Hz, 1H), 7.23 (d, J = 5.3 Hz, 1H), 3.38 (s, 3H), 2.22 (s, 3H), 1.63 (s, 6H). |
| **P80** | 2-methyl-2-(2-(methylsulfonamido)pyrimidin-4-yl)-N-(4-(6-(trifluoromethyl)pyrazin-2-yl)phenyl) propanamide 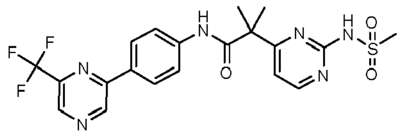 | Method 2, using INTC19 and INTD19, [HPLC Acidic], 481, (2.18) | 11.35 (s, 1H), 9.58 (s, 2H), 9.08 (s, 1H), 8.61 (d, J = 5.3 Hz, 1H), 8.24-8.12 (m, 2H), 7.93 - 7.77 (m, 2H), 7.18 (d, J = 5.3 Hz, 1H), 3.33 (s, 3H), 1.61 (s, 6H). |
| **P81** | N-(4-(6-ethoxypyrazin-2-yl)phenyl)-2-methyl-2-(2-(methylsulfonamido)pyrimidin-4-yl) propanamide 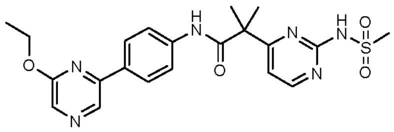 | Method 2, using INTC19 and INTD18, [HPLC Acidic], 457, (2.11) | 11.39 (s, 1H), 9.51 (s, 1H), 8.76 (s, 1H), 8.60 (d, J = 5.3 Hz, 1H), 8.18 (s, 1H), 8.11 - 8.04 (m, 2H), 7.82 - 7.70 (m, 2H), 7.16 (d, J = 5.3 Hz, 1H), 4.48 (q, J = 7.0 Hz, 2H), 3.32 (s, 3H), 1.60 (s, 6H), 1.40 (t, J = 7.1 Hz, 3H). |
| **P82** | 2-(2-((1,1-dimethylethyl)sulfonamido) pyrimidin-4-yl)-N-(4-(6-ethoxypyrazin-2-yl) phenyl)-2-methylpropanamide 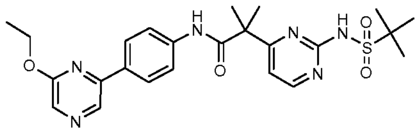 | Method 2, using INTC25 and INTD18, [UPLC Acidic], 499, (1.56) | 10.85 (s, 1H), 9.52 (s, 1H), 8.77 (s, 1H), 8.61 - 8.56 (m, 1H), 8.18 (s, 1H), 8.11 - 8.05 (m, 2H), 7.81 - 7.74 (m, 2H), 7.17 (s, 1H), 4.48 (q, J = 7.0 Hz, 2H), 1.58 (s, 6H), 1.43 - 1.36 (m, 12H). |
| **P83** | 1-(2-(cyclopropanesulfonamido)pyrimidin-4-yl)-N-(4-(6-ethoxypyrazin-2-yl)phenyl) cyclopropanecarboxamide 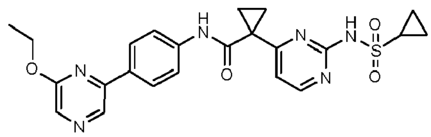 | Method 2, using INTC28 and INTD18, [UPLC acidic], 481, (1.39) | 11.45 (s, 1H), 10.70 (s, 1H), 8.78 (s, 1H), 8.44 (d, J = 5.3 Hz, 1H), 8.19 (s, 1H), 8.13 - 8.07 (m, 2H), 7.87 - 7.80 (m, 2H), 6.92 (s, 1H), 4.48 (q, J = 7.1 Hz, 2H), 3.11 (s, 1H), 1.66 - 1.59 (m, 2H), 1.53 - 1.46 (m, 2H), 1.40 (t, J = 7.1 Hz, 3H), 1.07 - 0.99 (m, 2H), 0.95 - 0.85 (m, 2H). |

(continued)

| P# | Name/Structure (All examples containing chiral centres are racemates unless stated) | Synthesis Method, [LCMS Method], m/z(M+H)+, (RT/Min) | $^1$H NMR Chemical Shift Data (DMSO-d6 unless stated) |
|---|---|---|---|
| **P84** | 2-(2-(cyclopropanesulfonamido)pyrimidin-4-yl)-N-(5'-(trifluoromethyl)-[3,3'-bipyridinl-6-yl) butanamide 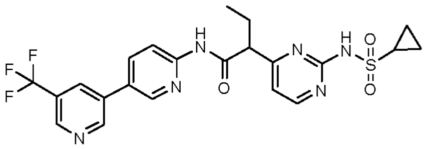 | Method 3 using INTC37 and INTD44, [UPLC Acidic], 507, (1.36) | 11.24 (s, 1H), 11.04 (s, 1H), 9.27 (d, J = 2.2 Hz, 1H), 8.98 (dd, J = 2.2, 1.0 Hz, 1H), 8.86 (dd, J = 2.6, 0.8 Hz, 1H), 8.59 - 8.54 (m, 2H), 8.33 (dd, J = 8.7, 2.6 Hz, 1H), 8.21 (d, J = 8.7 Hz, 1H), 7.21 (d, J = 5.2 Hz, 1H), 4.00 (dd, J = 8.6, 6.3 Hz, 1H), 3.32 - 3.28 (m, 1H), 2.11 - 2.04 (m, 1H), 2.00 - 1.90 (m, 1H), 1.16 - 1.06 (m, 2H), 1.00 - 0.88 (m, 5H). |
| **P85** | 2-(2-(cyclopropanesulfonamido)pyrimidin-4-yl)-N-(5'-(2,2,2-trifluoroethoxy)-[3,3'-bipyridin]-6-yl)butanamide 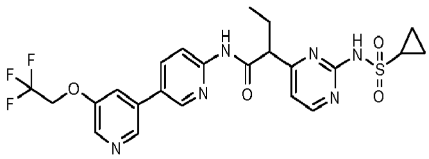 | Method 3 using INTC37 and INTD17, [UPLC Acidic], 537, (1.3) | 11.23 (s, 1H), 11.00 (s, 1H), 8.79 (d, J = 2.5 Hz, 1H), 8.66 (d, J = 1.8 Hz, 1H), 8.56 (d, J = 5.2 Hz, 1H), 8.41 (d, J = 2.8 Hz, 1H), 8.24 (dd, J = 8.7, 2.5 Hz, 1H), 8.19 (d, J = 8.7 Hz, 1H), 7.92 - 7.89 (m, 1H), 7.21 (d, J = 5.2 Hz, 1H), 4.99 (q, J = 8.8 Hz, 2H), 4.00 (dd, J = 7.5 Hz, 1H), 2.12 - 2.04 (m, 1H), 1.98 - 1.90 (m, 1H), 1.12-1.08 (m, 2H), 0.98 - 0.90 (m, 5H), 1 H obscured by $H_2O$. |
| **P86** | N-([3,3'-bipyridin]-6-yl)-2-(2-(cyclopropanesulfonamido)pyrimidin-4-yl) butanamide 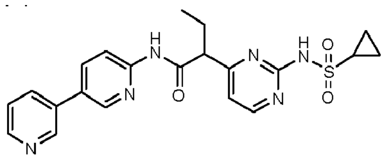 | Method 3 using INTC37 and a commercial aniline; [UPLC Acidic], 439, (0.81) | 11.25 (s, 1H), 10.96 (s, 1H), 8.94 (d, J = 2.4 Hz, 1H), 8.73 (dd, J = 1.7 Hz, 1H), 8.59 (dd, J = 4.7, 1.6 Hz, 1H), 8.56 (d, J = 5.1 Hz, 1H), 8.19 (d, J = 1.7 Hz, 2H), 8.14 (ddd, J = 8.0, 1.8 Hz, 1H), 7.51 (dd, J = 7.9, 4.8 Hz, 1H), 7.21 (d, J = 5.2 Hz, 1H), 4.04 - 3.96 (m, 1H), 3.31 - 3.28 (m, 1H), 2.13 - 2.02 (m, 1H), 1.99 - 1.89 (m, 1H), 1.17 - 1.02 (m, 2H), 0.99 - 0.86 (m, 5H). |
| **P87** | 2-(2-(cyclopropanesulfonamido)pyrimidin-4-yl)-N-(5-(6-(trifluoromethyl)pyrazin-2-yl)pyridin-2-yl)butanamide 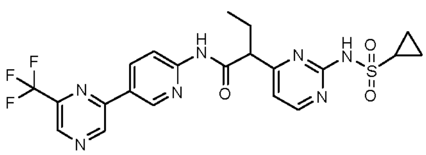 | Method 3 using INTC37 and INTD2, [UPLC Acidic], 508, (1.41) | 11.24 (s, 1H), 11.16 (s, 1H), 9.66 (s, 1H), 9.16 (dd, J = 2.5, 0.8 Hz, 2H), 8.61 - 8.53 (m, 2H), 8.31 - 8.24 (m, 1H), 7.22 (d, J = 5.2 Hz, 1H), 4.06 - 3.97 (m, 1H), 3.32 - 3.26 (m, 1H), 2.15 - 2.02 (m, 1H), 2.01 - 1.88 (m, 1H), 1.17 - 1.03 (m, 2H), 1.03 - 0.85 (m, 5H). |
| **P88** | 2-(2-(cyclopropanesulfonamido)pyrimidin-4-yl)-N-(5-(6-ethoxypyrazin-2-yl)pyridin-2-yl) butanamide 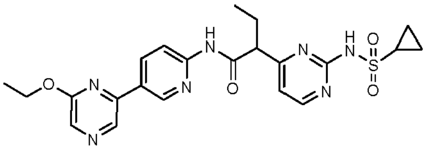 | Method 3 using INTC37 and INTD33, [UPLC Acidic], 484, (1.39) | 11.24 (s, 1H), 11.04 (s, 1H), 9.11 - 9.02 (m, 1H), 8.84 (s, 1H), 8.56 (d, J = 5.2 Hz, 1H), 8.53 - 8.46 (m, 1H), 8.25 (s, 1H), 8.21 (d, J = 8.8 Hz, 1H), 7.21 (d, J = 5.2 Hz, 1H), 4.48 (q, J = 7.0 Hz, 2H), 4.06 - 3.96 (m, 1H), 3.32 - 3.26 (m, 1H), 2.15 - 2.01 (m, 1H), 2.00 - 1.88 (m, 1H), 1.40 (t, J = 7.0 Hz, 3H), 1.17 - 1.03 (m, 2H), 1.03 - 0.87 (m, 5H). |

(continued)

| P# | Name/Structure (All examples containing chiral centres are racemates unless stated) | Synthesis Method, [LCMS Method], m/z(M+H)+, (RT/Min) | $^1H$ NMR Chemical Shift Data (DMSO-d6 unless stated) |
|---|---|---|---|
| **P89** | 2-(2-(cyclopropanesulfonamido)pyrimidin-4-yl)-N-(5-(6-isopropoxypyrazin-2-yl)pyridin-2-yl) butanamide 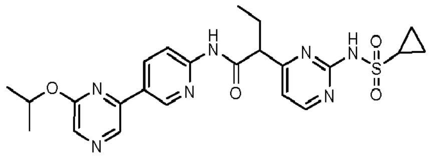 | Method 3 using INTC37 and INTD32, [UPLC Acidic], 498, (1.48) | 11.23 (s, 1H), 11.03 (s, 1H), 9.05 (dd, J = 2.5, 0.8 Hz, 1H), 8.82 (s, 1H), 8.56 (d, J = 5.2 Hz, 1H), 8.48 (dd, J = 8.8, 2.5 Hz, 1H), 8.25 - 8.17 (m, 2H), 7.21 (d, J = 5.2 Hz, 1H), 5.41 (hept, J = 6.1 Hz, 1H), 4.01 (dd, J = 8.6, 6.4 Hz, 1H), 3.32 - 3.28 (m, 1H), 2.13 - 2.03 (m, 1H), 2.01 - 1.89 (m, 1H), 1.38 (d, J = 6.2 Hz, 6H), 1.15 -1.05 (m, 2H), 1.01 - 0.88 (m, 5H). |
| **P90** | N-(4-(5-chloropyridin-3-yl)-2-fluorophenyl)-2-(2-(cyclopropanesulfonamido)pyrimidin-4-yl) butanamide 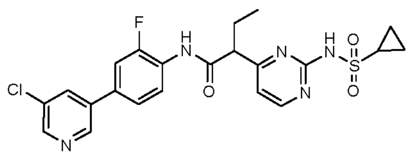 | Method 3 using INTC37 and INTD13, [UPLC Acidic], 490, (1.37) | 11.27 (s, 1H), 10.21 (s, 1H), 8.91 (d, J = 2.0 Hz, 1H), 8.63 (d, J = 2.3 Hz, 1H), 8.56 (d, J = 5.2 Hz, 1H), 8.31 (dd, J = 2.3 Hz, 1H), 8.00 (dd, J = 8.3 Hz, 1H), 7.81 (dd, J = 12.2, 2.1 Hz, 1H), 7.65 (dd, J = 8.4, 2.1 Hz, 1H), 7.19 (d, J = 5.2 Hz, 1H), 3.97 (dd, J = 8.7, 6.3 Hz, 1H), 3.31 - 3.27 (m, 1H), 2.11 - 2.01 (m, 1H), 1.99 - 1.91 (m, 1H), 1.16 - 1.08 (m, 2H), 1.05 - 0.91 (m, 5H). |
| **P91** | 2-(2-(cyclopropanesulfonamido)pyrimidin-4-yl)-N-(2-fluoro-4-(5-(2,2,2-trifluoroethoxy) pyridin-3-yl)phenyl)butanamide 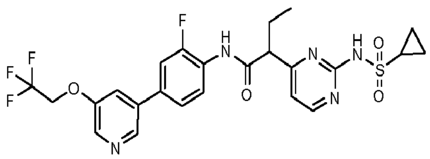 | Method 3 using INTC37 and INTD14; [UPLC Acidic], 554, (1.38) | 11.27 (s, 1H), 10.20 (s, 1H), 8.65 (d, J = 1.8 Hz, 1H), 8.57 (d, J = 5.2 Hz, 1H), 8.39 (d, J = 2.8 Hz, 1H), 7.99 (dd, J = 8.3 Hz, 1H), 7.87 (dd, J = 2.3 Hz, 1H), 7.80 (dd, J = 12.2, 2.1 Hz, 1H), 7.64 (dd, J = 8.5, 2.0 Hz, 1H), 7.20 (d, J = 5.2 Hz, 1H), 4.99 (q, J = 8.9 Hz, 2H), 3.97 (dd, J = 8.8, 6.2 Hz, 1H), 2.10 - 2.01 (m, 1H), 1.99 - 1.89 (m, 1H), 1.15 - 1.08 (m, 2H), 1.03 - 0.92 (m, 5H), 1H obscured by $H_2O$. |
| **P92** | 2-(2-(cyclopropanesulfonamido)pyrimidin-4-yl)-N-(2-fluoro-4-(5-isopropoxypyridin-3-yl) phenyl)butanamide 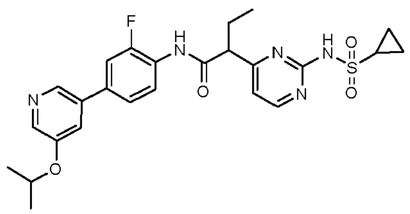 | Method 3 using INTC37 and INTD12, [UPLC Acidic], 514, (1.22) | 11.25 (s, 1H), 10.17 (s, 1H), 8.57 (d, J = 5.1 Hz, 1H), 8.49 (d, J = 1.9 Hz, 1H), 8.25 (d, J = 2.7 Hz, 1H), 7.96 (dd, J = 8.3 Hz, 1H), 7.74 (dd, J = 12.2, 2.1 Hz, 1H), 7.69 - 7.62 (m, 1H), 7.59 (dd, J = 8.4, 2.0 Hz, 1H), 7.20 (s, 1H), 4.92 - 4.82 (m, 1H), 4.01 - 3.93 (m, 1H), 3.31 - 3.27 (m, 1H), 2.13 - 2.01 (m, 1H), 1.99- 1.90 (m, 1H), 1.32 (d, J = 6.0 Hz, 6H), 1.17 - 1.09 (m, 2H), 1.05-0.92 (m, 5H). |

(continued)

| P# | Name/Structure (All examples containing chiral centres are racemates unless stated) | Synthesis Method, [LCMS Method], m/z(M+H)+, (RT/Min) | $^1$H NMR Chemical Shift Data (DMSO-d6 unless stated) |
|---|---|---|---|
| P93 | 2-(2-(cyclopropanesulfonamido)pyrimidin-4-yl)-N-(2-fluoro-4-(pyridin-3-yl)phenyl) butanamide 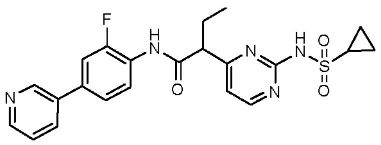 | Method 3 using INTC37 and INTD50; [UPLC Acidic], 456, (0.85) | 11.27 (s, 1H), 10.17 (s, 1H), 8.93 (d, J = 2.4 Hz, 1H), 8.62 - 8.54 (m, 2H), 8.11 (dt, J = 8.1, 1.9 Hz, 1H), 7.98 (dd, J = 8.3 Hz, 1H), 7.72 (dd, J = 12.2, 2.1 Hz, 1H), 7.58 (dd, J = 8.4, 2.1 Hz, 1H), 7.52 - 7.45 (m, 1H), 7.20 (d, J = 5.2 Hz, 1H), 4.00 - 3.93 (m, 1H), 2.12 - 2.01 (m, 1H), 2.00 - 1.91 (m, 1H), 1.16- 1.08 (m, 2H), 1.03 - 0.91 (m, 5H), 1H obscured by $H_2O$. |
| P94 | 2-(2-(cyclopropanesulfonamido)pyrimidin-4-yl)-N-(2-fluoro-4-(6-(trifluoromethyl)pyrazin-2-yl)phenyl)butanamide 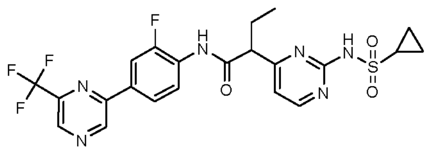 | Method 3 using INTC37 and INTD25, [UPLC Acidic], 525, (1.49) | 11.25 (s, 1H), 10.32 (s, 1H), 9.64 (s, 1H), 9.15 (s, 1H), 8.57 (d, J = 5.2 Hz, 1H), 8.21 - 8.09 (m, 2H), 8.06 (dd, J = 8.6, 2.0 Hz, 1H), 7.21 (d, J = 5.3 Hz, 1H), 4.02 (t, J = 7.5 Hz, 1H), 3.32 - 3.26 (m, 1H), 2.12 - 2.02 (m, 1H), 2.01 - 1.88 (m, 1H), 1.17 - 1.05 (m, 2H), 1.05 - 0.92 (m, 5H). |
| P95 | 2-(2-(cyclopropanesulfonamido)pyrimidin-4-yl)-N-(2-fluoro-4-(6-methoxypyrazin-2-yl) phenyl)butanamide 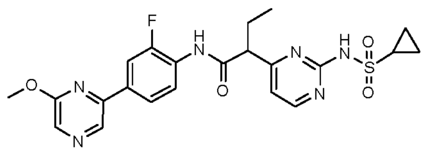 | Method 3 using INTC37 and INTD46, [UPLC Acidic], 487, (1.35) | 11.25 (s, 1H), 10.24 (s, 1H), 8.85 (s, 1H), 8.57 (d, J = 5.2 Hz, 1H), 8.28 (s, 1H), 8.09 - 8.03 (m, 2H), 8.00 - 7.97 (m, 1H), 7.20 (d, J = 4.7 Hz, 1H), 4.03 (s, 3H), 3.99 (dd, J = 10.2, 5.0 Hz, 1H), 3.31 - 3.27 (m, 1H), 2.12-2.02 (m, 1H), 2.00 - 1.91 (m, 1H), 1.16 - 1.08 (m, 2H), 1.02 - 0.93 (m, 5H). |
| P96 | 2-(2-(cyclopropanesulfonamido)pyrimidin-4-yl)-N-(4-(6-ethoxypyrazin-2-yl)-2-fluorophenyl) butanamide 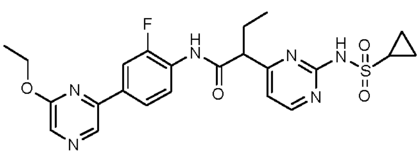 | Method 3 using INTC37 and INTD24, [UPLC acidic], 501, (1.45) | 11.27 (s, 1H), 10.24 (s, 1H), 8.83 (s, 1H), 8.56 (d, J = 5.2 Hz, 1H), 8.24 (s, 1H), 8.09 - 8.00 (m, 2H), 7.99 - 7.94 (m, 1H), 7.19 (d, J = 5.2 Hz, 1H), 4.49 (q, J = 7.1 Hz, 2H), 4.05 - 3.92 (m, 1H), 3.33 - 3.28 (m, 1H), 2.12 - 2.01 (m, 1H), 2.00 - 1.89 (m, 1H), 1.40 (t, J = 7.0 Hz, 3H), 1.17 -1.06 (m, 2H), 1.05 - 0.89 (m, 5H). |
| P97 | 2-(2-(cyclopropanesulfonamido)pyrimidin-4-yl)-N-(2-fluoro-4-(6-isopropoxypyrazin-2-yl) phenyl)butanamide 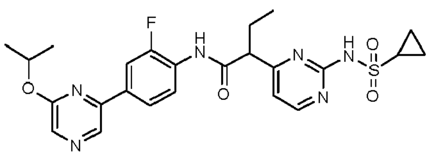 | Method 3 using INTC37 and INTD35, [UPLC Acidic], 515, (1.55) | 11.25 (s, 1H), 10.24 (s, 1H), 8.81 (s, 1H), 8.57 (d, J = 5.2 Hz, 1H), 8.19 (s, 1H), 8.06 (d, J = 8.3 Hz, 1H), 8.00 (dd, J = 12.2, 1.9 Hz, 1H), 7.95 (dd, J = 8.5, 2.0 Hz, 1H), 7.20 (d, J = 5.1 Hz, 1H), 5.45 - 5.39 (m, 1H), 4.00 (t, J = 7.7 Hz, 1H), 3.31 - 3.27 (m, 1H), 2.13 - 2.02 (m, 1H), 1.99 - 1.90 (m, 1H), 1.39 (d, J = 6.2 Hz, 6H), 1.16-1.08 (m, 2H), 1.03 - 0.93 (m, 5H). |

(continued)

| P# | Name/Structure (All examples containing chiral centres are racemates unless stated) | Synthesis Method, [LCMS Method], m/z (M+H)+, (RT/Min) | $^1$H NMR Chemical Shift Data (DMSO-d6 unless stated) |
|---|---|---|---|
| **P98** | 2-(2-(cyclopropanesulfonamido)pyrimidin-4-yl)-N-(2-fluoro-4-(6-(2,2,2-trifluoroethoxy) pyrazin-2-yl)phenyl)butanamide 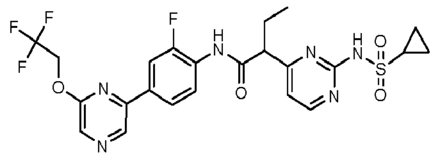 | Method 3 using INTC37 and INTD28, [UPLC Acidic], 555, (1.49) | 11.27 (s, 1H), 10.28 (s, 1H), 9.00 (s, 1H), 8.57 (d, J = 5.2 Hz, 1H), 8.44 (s, 1H), 8.15 (dd, J = 12.3, 1.9 Hz, 1H), 8.10 (dd, J = 8.1 Hz, 1H), 8.04 (dd, J = 8.5, 1.9 Hz, 1H), 7.21 (d, J = 5.2 Hz, 1H), 5.22 (q, J = 9.0 Hz, 2H), 4.01 (dd, J = 8.6, 6.2 Hz, 1H), 2.12 - 2.02 (m, 1H), 1.99 - 1.90 (m, 1H), 1.17 - 1.06 (m, 2H), 1.03 - 0.90 (m, 5H), 1H obscured by $H_2O$. |
| **P99** | N-(4-(5-cyanopyridin-3-yl)phenyl)-2-(2-(cyclopropanesulfonamido)pyrimidin-4-yl) butanamide 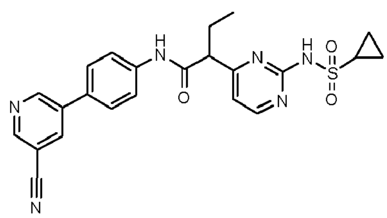 | Method 3 using INTC37 and INTD5, [UPLC Acidic], 463, (1.23) | 11.24 (s, 1H), 10.41 (s, 1H), 9.18 (d, J = 2.3 Hz, 1H), 8.97 (d, J = 1.9 Hz, 1H), 8.63 (dd, J = 2.1 Hz, 1H), 8.57 (d, J = 5.2 Hz, 1H), 7.83 - 7.80 (m, 2H), 7.78 - 7.75 (m, 2H), 7.21 (d, J = 5.2 Hz, 1H), 3.77 (dd, J = 8.7, 6.3 Hz, 1H), 3.31 - 3.26 (m, 1H), 2.13 - 2.02 (m, 1H), 1.98 - 1.89 (m, 1H), 1.13 - 1.07 (m, 2H), 1.00 - 0.90 (m, 5H). |
| **P100** | 2-(2-(cyclopropanesulfonamido)pyrimidin-4-yl)-N-(4-(5-(2,2,2-trifluoroethoxy)pyridin-3-yl) phenyl)butanamide 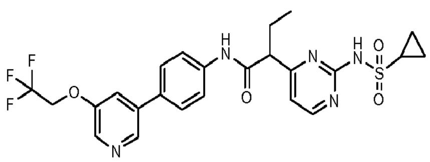 | Method 3 using INTC37 and INTD15, [UPLC Acidic], 536, (1.32) | 11.27 (s, 1H), 10.39 (s, 1H), 8.61 - 8.53 (m, 2H), 8.36 (d, J = 2.8 Hz, 1H), 7.82 - 7.70 (m, 5H), 7.21 (d, J = 5.2 Hz, 1H), 4.98 (q, J = 8.8 Hz, 2H), 3.76 (dd, J = 8.7, 6.3 Hz, 1H), 3.31 - 3.26 (m, 1H), 2.11 - 2.02 (m, 1H), 1.98 - 1.90 (m, 1H), 1.13 - 1.05 (m, 2H), 1.00 - 0.88 (m, 5H). |
| **P101** | 2-(2-(cyclopropanesulfonamido)pyrimidin-4-yl)-N-(4-(5-isopropoxypyridin-3-yl)phenyl) butanamide 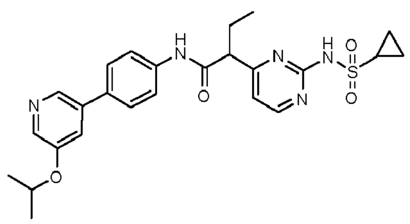 | Method 3 using INTC37 and INTD11, [UPLC Acidic], 496, (1.13) | 11.26 (s, 1H), 10.36 (s, 1H), 8.57 (d, J = 5.2 Hz, 1H), 8.44 (d, J = 1.9 Hz, 1H), 8.21 (d, J = 2.7 Hz, 1H), 7.75 - 7.69 (m, 4H), 7.58 - 7.55 (m, 1H), 7.20 (d, J = 5.2 Hz, 1H), 4.84 (hept, J = 6.0 Hz, 1H), 3.76 (dd, J = 8.8, 6.3 Hz, 1H), 3.33 - 3.27 (m, 1H), 2.13 - 2.02 (m, 1H), 1.99 - 1.90 (m, 1H), 1.32 (d, J = 6.0 Hz, 6H), 1.14 - 1.05 (m, 2H), 1.01 - 0.88 (m, 5H). |
| **P102** | 2-(2-(cyclopropanesulfonamido)pyrimidin-4-yl)-N-(4-(pyridin-3-yl)phenyl)butanamide 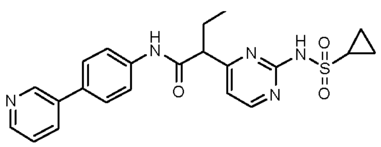 | Method 2, using INTC35 and a commercial aniline, [HPLC acidic], 438, (1.31) | 11.27 (s, 1H), 10.36 (s, 1H), 8.94 - 8.80 (m, 1H), 8.61 - 8.47 (m, 2H), 8.09 - 8.01 (m, 1H), 7.78 - 7.61 (m, 4H), 7.50 - 7.42 (m, 1H), 7.20 (d, J = 5.2 Hz, 1H), 3.79 - 3.72 (m, 1H), 3.31 - 3.25 (m, 1H), 2.14 - 2.01 (m, 1H), 2.00 - 1.87 (m, 1H), 1.15 - 1.04 (m, 2H), 1.02 - 0.86 (m, 5H). |

(continued)

| P# | Name/Structure (All examples containing chiral centres are racemates unless stated) | Synthesis Method, [LCMS Method], m/z(M+H)+, (RT/Min) | $^1$H NMR Chemical Shift Data (DMSO-d6 unless stated) |
|---|---|---|---|
| **P103** | 2-(2-(cyclopropanesulfonamido)pyrimidin-4-yl)-N-(4-(6-(trifluoromethyl)pyrazin-2-yl)phenyl) butanamide 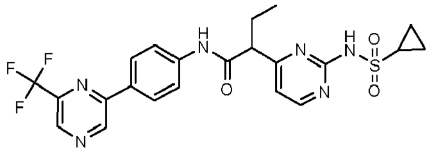 | Method 1 using INTC37 and INTD19, [UPLC Acidic], 507, (1.47) | 11.27 (s, 1H), 10.52 (s, 1H), 9.58 (s, 1H), 9.08 (s, 1H), 8.57 (d, J = 5.2 Hz, 1H), 8.23 - 8.15 (m, 2H), 7.86 - 7.78 (m, 2H), 7.20 (d, J = 5.2 Hz, 1H), 3.85 - 3.72 (m, 1H), 3.32 - 3.24 (m, 1H), 2.14 - 2.01 (m, 1H), 2.00 - 1.90 (m, 1H), 1.14 - 1.03 (m, 2H), 1.02 - 0.85 (m, 5H). |
| **P104** | N-(4-(6-chloropyrazin-2-yl)phenyl)-2-(2-(cyclopropanesulfonamido)pyrimidin-4-yl) butanamide 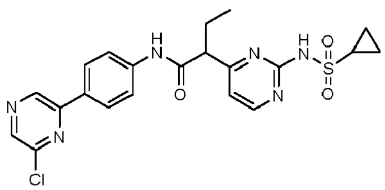 | Method 3 using INTC37 and INTD22, [UPLC Acidic], 473, (1.4) | 11.25 (s, 1H), 10.49 (s, 1H), 9.24 (s, 1H), 8.69 (s, 1H), 8.57 (d, J = 5.2 Hz, 1H), 8.15 - 8.09 (m, 2H), 7.83 - 7.75 (m, 2H), 7.20 (d, J = 5.2 Hz, 1H), 3.78 (dd, J = 8.6, 6.3 Hz, 1H), 3.30 - 3.26 (m, 1H), 2.12 - 2.03 (m, 1H), 1.96 - 1.92 (m, 1H), 1.13-1.05 (m, 2H), 1.01 - 0.88 (m, 5H). |
| **P105** | 2-(2-(cyclopropanesulfonamido)pyrimidin-4-yl)-N-(4-(6-ethoxypyrazin-2-yl)phenyl) butanamide 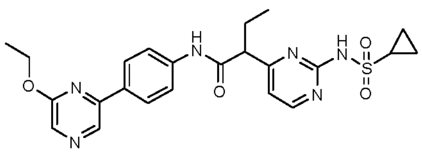 | Method 1 using INTC37 and INTD18, [UPLC acidic], 483, (1.43) | 11.28 (s, 1H), 10.43 (s, 1H), 8.76 (s, 1H), 8.56 (d, J = 5.2 Hz, 1H), 8.18 (s, 1H), 8.15 - 8.04 (m, 2H), 7.80 - 7.71 (m, 2H), 7.19 (d, J = 5.2 Hz, 1H), 4.48 (q, J = 7.0 Hz, 2H), 3.81 - 3.73 (m, 1H), 3.32 - 3.26 (m, 1H), 2.13 - 2.01 (m, 1H), 2.01 - 1.87 (m, 1H), 1.40 (t, J = 7.0 Hz, 3H), 1.13- 1.04 (m, 2H), 1.03 - 0.85 (m, 5H). |
| **P106** | 2-(2-(cyclopropanesulfonamido)pyrimidin-4-yl)-N-(4-(6-methoxypyrazin-2-yl)phenyl) butanamide 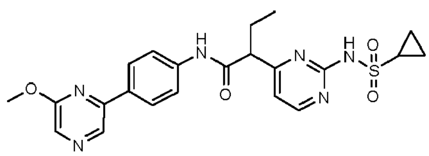 | Method 3 using INTC37 and INTD1, [UPLC Acidic], 469, (1.33) | 11.25 (s, 1H), 10.44 (s, 1H), 8.78 (s, 1H), 8.57 (d, J = 5.2 Hz, 1H), 8.22 (s, 1H), 8.15 - 8.10 (m, 2H), 7.80 - 7.73 (m, 2H), 7.21 (d, J = 5.3 Hz, 1H), 4.02 (s, 3H), 3.78 (dd, J = 8.7, 6.3 Hz, 1H), 3.32 - 3.27 (m, 1H), 2.13 - 2.03 (m, 1H), 1.99 - 1.90 (m, 1H), 1.14 - 1.06 (m, 2H), 1.00 - 0.89 (m, 5H). |
| **P107** | 2-(2-(cyclopropanesulfonamido)pyrimidin-4-yl)-N-(4-(6-isopropoxypyrazin-2-yl)phenyl) butanamide 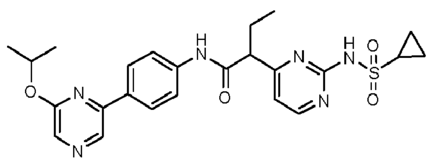 | Method 3 using INTC37 and INTD20, [UPLC Acidic], 497, (1.53) | 11.25 (s, 1H), 10.44 (s, 1H), 8.74 (s, 1H), 8.57 (d, J = 5.2 Hz, 1H), 8.13 (s, 1H), 8.11 - 8.05 (m, 2H), 7.79 - 7.73 (m, 2H), 7.21 (d, J = 5.2 Hz, 1H), 5.41 (hept, J = 6.1 Hz, 1H), 3.78 (dd, J = 8.7, 6.4 Hz, 1H), 3.32 - 3.26 (m, 1H), 2.12 - 2.03 (m, 1H), 1.99-1.90 (m, 1H), 1.38 (d, J = 6.2 Hz, 6H), 1.14 - 1.06 (m, 2H), 1.00 - 0.87 (m, 5H). |

(continued)

| P# | Name/Structure (All examples containing chiral centres are racemates unless stated) | Synthesis Method, [LCMS Method], m/z(M+H)$^+$, (RT/Min) | $^1$H NMR Chemical Shift Data (DMSO-d6 unless stated) |
|---|---|---|---|
| **P108** | 2-(2-(cyclopropanesulfonamido)pyrimidin-4-yl)-N-(4-(6-(2,2,2-trifluoroethoxy)pyrazin-2-yl) phenyl)butanamide 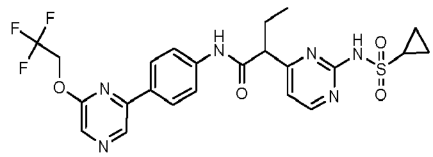 | Method 3 using INTC37 and INTD29, [UPLC Acidic], 537, (1.49) | 11.25 (s, 1H), 10.46 (s, 1H), 8.93 (s, 1H), 8.57 (d, J = 5.2 Hz, 1H), 8.38 (s, 1H), 8.20 - 8.14 (m, 2H), 7.81 - 7.76 (m, 2H), 7.21 (d, J = 5.3 Hz, 1H), 5.19 (q, J = 9.0 Hz, 2H), 3.78 (dd, J = 8.5, 6.4 Hz, 1H), 2.13 - 2.03 (m, 1H), 2.01 - 1.90 (m, 1H), 1.14 - 1.07 (m, 2H), 0.99 - 0.89 (m, 5H), 1H obscured by $H_2O$. |
| **P109** | 2-(2-(cyclopropanesulfonamido)pyrimidin-4-yl)-N-(4-(pyrazin-2-yl)phenyl)butanamide 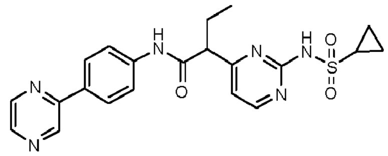 | Method 3 using INTC37 and INTD43, [UPLC Basic], 439, (0.87) | 11.25 (s, 1H), 10.44 (s, 1H), 9.22 (d, J = 1.6 Hz, 1H), 8.68 (dd, J = 2.5, 1.5 Hz, 1H), 8.60 - 8.53 (m, 2H), 8.15 - 8.10 (m, 2H), 7.82 - 7.75 (m, 2H), 7.20 (d, J = 5.2 Hz, 1H), 3.78 (dd, J = 8.7, 6.3 Hz, 1H), 3.31 - 3.26 (m, 1H), 2.13 - 2.02 (m, 1H), 2.00 - 1.89 (m, 1H), 1.12 - 1.04 (m, 2H), 1.01 - 0.88 (m, 5H). |
| **P110** | 2-(2-(cyclopropanesulfonamido)pyrimidin-4-yl)-N-(4-(6-ethoxypyrazin-2-yl)phenyl)-4-methoxybutanamide 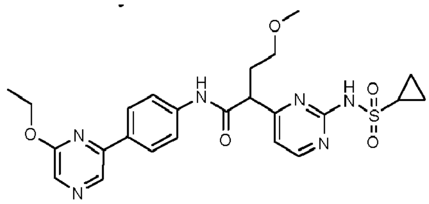 | Method 3 using INTC38 and INTD18, [UPLC Acidic], 513, (1.34) | 11.28 (s, 1H), 10.47 (s, 1H), 8.76 (s, 1H), 8.56 (d, J = 5.2 Hz, 1H), 8.18 (s, 1H), 8.11 - 8.06 (m, 2H), 7.78 - 7.67 (m, 2H), 7.20 (d, J = 5.2 Hz, 1H), 4.47 (q, J = 7.1 Hz, 2H), 4.06 - 3.95 (m, 1H), 3.41 - 3.35 (m, 3H), 3.23 (s, 3H), 2.33 - 2.25 (m, 1H), 2.20 - 2.11 (m, 1H), 1.40 (t, J = 7.0 Hz, 3H), 1.18 - 1.03 (m, 2H), 1.01 - 0.88 (m, 2H). |
| **P111** | 2-(2-(cyclopropanesulfonamido)pyrimidin-4-yl)-N-(4-(pyridin-3-yl)phenyl)propanamide 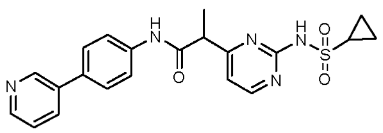 | Method 2, using INTC18 and a commercial aniline, [UPLC acidic], 424, (0.70) | (Methanol-d4) 8.81 (s, 1H), 8.59 - 8.46 (m, 2H), 8.21 - 8.02 (m, 2H), 7.81 - 7.73 (m, 2H), 7.70 - 7.61 (m, 2H), 7.56 - 7.48 (m, 1H), 7.17 (d, J = 5.2 Hz, 1H), 4.08 - 3.92 (m, 1H), 3.32 - 3.25 (m, 1H), 1.63 (d, J = 6.9 Hz, 3H), 1.29 - 1.18 (m, 2H), 1.03 - 0.88 (m, 2H), 1 exchangeable proton not observed. |

## Biological Examples

### Biological Example 1 - Human CTPS1 Enzyme Inhibition

The enzyme inhibitory activities of compounds invented against the target of interest were determined using the ADP-Glo™ Max assay (Promega, UK). Assays for human CTPS1 were performed in 1x assay buffer containing 50mM Tris, 10mM $MgCl_2$, 0.01% Tween-20, pH to 8.0 accordingly. Finally, immediately before use, L-cysteine was added to the 1x assay buffer to a final concentration of 2mM. All reagents are from Sigma-Aldrich unless specified otherwise. Human full length active C-terminal FLAG-Hiss-tag CTPS1 (UniProtKB - P17812, CTPS[1-591]-GGDYKDDDDKGGHHHHHHHH) was obtained from Proteros biostructures GmbH.

Assay Procedure.

3x human CTPS1 protein was prepared in 1x assay buffer to the final working protein concentration required for the reaction. A 2uL volume per well of 3x human CTPS1 protein was mixed with 2uL per well of 3x test compound (compound prepared in 1x assay buffer to an appropriate final 3x compound concentration respective to the concentration response curve designed for the compounds under test) for 10 minutes at 25°C. The enzymatic reaction was then initiated by addition of a 2uL per well volume of a pre-mixed substrate mix (UltraPure ATP from ADP-Glo™ Max kit (0.31mM), GTP (0.034mM), UTP (0.48mM) and L-glutamine (0.186mM)) and the mixture was incubated for an appropriate amount of time within the determined linear phase of the reaction at 25°C under sealed plate conditions with constant agitation at 500 revolutions per minute (rpm). ADP-Glo™ Max reagent was added for 60 minutes (6μL per well) and subsequently ADP-Glo™ Max development reagent was added for 60 minutes (12uL per well) prior to signal detection in a microplate reader (EnVision® Multilabel Reader, Perkin Elmer). Following each reagent addition over the course of the assay, assay plates were pulse centrifuged for 30 seconds at 500rpm.

In all cases, the enzyme converts ATP to ADP and the ADP-Glo™ Max reagent subsequently depletes any remaining endogenous ATP in the reaction system. The ADP-Glo™ Max detection reagent converts the ADP that has been enzymatically produced back into ATP and using ATP as a substrate together with luciferin for the enzyme luciferase, light is generated which produces a detectable luminescence. The luminescent signal measured is directly proportional to the amount of ADP produced by the enzyme reaction and a reduction in this signal upon compound treatment demonstrates enzyme inhibition. The percentage inhibition produced by each concentration of compound was calculated using the equation shown below:

$$\%\,Inhibition = 1 - \frac{(Mean_{Min} - Mean_{Inh})}{(Mean_{Min} - \ Mean_{Max})}\ x\ 100$$

Percentage inhibition was then plotted against compound concentration, and the 50% inhibitory concentration ($IC_{50}$) was determined from the resultant concentration-response curve.

**Table 7:** *Human CTPS1 Enzyme Inhibition data grouped by potency range* (± indicates $IC_{50}$ in the range of >10 to 20 micromolar, + indicates $IC_{50}$ in the range >1 to 10 micromolar, ++ indicates $IC_{50}$ in the range >0.1 to 1 micromolar, +++ indicates $IC_{50}$ of ≤0.1 micromolar)

| P | CTPS1 | | P | CTPS1 | | P | CTPS1 | | P | CTPS1 |
|---|---|---|---|---|---|---|---|---|---|---|
| P1 | ++ | | P29 | + | | P57 | ++ | | P85 | ++ |
| P2 | +++ | | P30 | +++ | | P58 | ++ | | P86 | + |
| P3 | +++ | | P31 | +++ | | P59 | + | | P87 | +++ |
| P4 | ++ | | P32 | +++ | | P60 | + | | P88 | +++ |
| P5 | + | | P33 | +++ | | P61 | + | | P89 | +++ |
| P6 | ++ | | P34 | +++ | | P62 | + | | P90 | ++ |
| P7 | ++ | | P35 | ++ | | P63 | ± | | P91 | ++ |
| P8 | +++ | | P36 | + | | P64 | +++ | | P92 | ++ |
| P9 | +++ | | P37 | +++ | | P65 | +++ | | P93 | + |
| P10 | +++ | | P38 | +++ | | P66 | ++ | | P94 | +++ |
| P11 | +++ | | P39 | +++ | | P67 | +++ | | P95 | +++ |
| P12 | +++ | | P40 | ++ | | P68 | ++ | | P96 | +++ |
| P13 | ++ | | P41 | +++ | | P69 | ++ | | P97 | +++ |
| P14 | ++ | | P42 | + | | P70 | + | | P98 | +++ |
| P15 | + | | P43 | ++ | | P71 | + | | P99 | ++ |
| P16 | ++ | | P44 | ++ | | P72 | ++ | | P100 | ++ |
| P17 | + | | P45 | ++ | | P73 | ++ | | P101 | ++ |

(continued)

| P | CTPS1 | | P | CTPS1 | | P | CTPS1 | | P | CTPS1 |
|---|---|---|---|---|---|---|---|---|---|---|
| P18 | +++ | | P46 | ++ | | P74 | ++ | | P102 | ++ |
| P19 | +++ | | P47 | +++ | | P75 | ++ | | P103 | ++ |
| P20 | +++ | | P48 | ++ | | P76 | ++ | | P104 | ++ |
| P21 | +++ | | P49 | +++ | | P77 | + | | P105 | +++ |
| P22 | ++ | | P50 | + | | P78 | ++ | | P106 | +++ |
| P23 | ++ | | P51 | ++ | | P79 | ++ | | P107 | +++ |
| P24 | ++ | | P52 | ++ | | P80 | ++ | | P108 | +++ |
| P25 | ++ | | P53 | ++ | | P81 | +++ | | P109 | ++ |
| P26 | ++ | | P54 | ++ | | P82 | + | | P110 | +++ |
| P27 | +++ | | P55 | +++ | | P83 | +++ | | P111 | ++ |
| P28 | ++ | | P56 | ++ | | P84 | ++ | | | |

All compounds of the invention which have been tested were found to demonstrate inhibition of CTPS1 enzyme in this assay. Consequently, these compounds may be expected to have utility in the inhibition of CTPS1.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

The application of which this description and claims forms part may be used as a basis for priority in respect of any subsequent application. The claims of such subsequent application may be directed to any feature or combination of features described herein. They may take the form of product, composition, process, or use claims and may include, by way of example and without limitation, the claims which follow.

All publications, including but not limited to patents and patent applications, cited in this specification are herein incorporated by reference as if each individual publication were specifically and individually indicated to be incorporated by reference herein as though fully set forth.

**Clauses of the invention:**

Clause 1. A compound of formula (I):

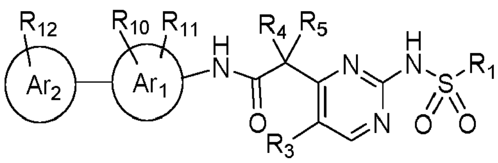

(I)

wherein

$R_1$ is $C_{1-5}$alkyl or $C_{0-2}$alkylene$C_{3-5}$cycloalkyl which cycloalkyl is optionally substituted by $CH_3$;

$R_3$ is H, halo or $CH_3$;

$R_4$ and $R_5$ are each independently H, $C_{1-6}$alkyl, $C_{1-3}$alkyleneO$C_{1-3}$alkyl, or $R_4$ and $R_5$ together with the carbon atom to which they are attached form a $C_{3-6}$spirocycloalkyl;

Ar1 is a 6-membered aryl or heteroaryl;

Ar2 is a 6-membered aryl or heteroaryl and is attached to Ar1 in the para position relative to the amide;

$R_{10}$ is H, halo, $C_{1-2}$alkyl, $OC_{1-2}$alkyl, $OC_{1-2}$haloalkyl or CN;

$R_{11}$ is H, F, $CH_3$ or $OCH_3$; and

$R_{12}$ is attached to Ar2 in the ortho or meta position relative to Ar1 and $R_{12}$ is H, halo, $C_{1-4}$alkyl, $OC_{1-4}$alkyl, $OC_{0-2}$alkylene$C_{3-5}$cycloalkyl, CN, $C_{1-4}$haloalkyl, $OC_{1-4}$haloalkyl;

or a salt and/or solvate thereof and/or derivative thereof.

Clause 2. The compound according to clause 1 wherein $R_1$ is $C_{1-5}$alkyl.

Clause 3. The compound according to clause 1 wherein $R_1$ is $C_{0-2}$alkylene$C_{3-5}$cycloalkyl which cycloalkyl is optionally substituted by $CH_3$.

Clause 4. The compound according to clause 3 wherein $R_1$ is $C_{0-2}$alkylene$C_{3-5}$cycloalkyl.

Clause 5. The compound according to clause 3 wherein $R_1$ is $C_{0-2}$alkylene$C_{3-5}$cycloalkyl which cycloalkyl is substituted by $CH_3$.

Clause 6. The compound according to any one of clauses 3 to 5 wherein $R_1$ is $C_{3-5}$cycloalkyl optionally substituted by $CH_3$.

Clause 7. The compound according to any one of clauses 3 to 5 wherein $R_1$ is $C_1$alkylene$C_{3-5}$cycloalkyl optionally substituted by $CH_3$.

Clause 8. The compound according to any one of clauses 3 to 5 wherein $R_1$ is $C_2$alkylene$C_{3-5}$cycloalkyl optionally substituted by $CH_3$.

Clause 9. The compound according to any one of clauses 1 to 8 wherein $R_1$ is cyclopropyl, cyclopropyl substituted by $CH_3$ at the point of attachment, cyclobutyl, methyl or ethyl.

Clause 10. The compound according to clause 9 wherein $R_1$ is cyclopropyl, methyl or ethyl.

Clause 11. The compound according to clause 10 wherein $R_1$ is cyclopropyl.

Clause 12. The compound according to any one of clauses 1 to 11 wherein $R_3$ is H.

Clause 13. The compound according to any one of clauses 1 to 11 wherein $R_3$ is halo.

Clause 14. The compound according to clause 13 wherein $R_3$ is fluoro.

Clause 15. The compound according to any one of clauses 1 to 11 wherein $R_3$ is $CH_3$.

Clause 16. The compound according to any one of clauses 1 to 15 wherein $R_4$ is H.

Clause 17. The compound according to any one of clauses 1 to 15 wherein $R_4$ is $C_{1-6}$alkyl.

Clause 18. The compound according to clause 17 wherein $R_4$ is methyl or ethyl.

Clause 19. The compound according to any one of clauses 1 to 15 wherein $R_4$ is $C_{1-3}$alkylene$OC_{1-3}$alkyl.

Clause 20. The compound according to clause 19 wherein $R_4$ is $C_2$alkylene$OC_{1-3}$alkyl.

Clause 21. The compound according to clause 20 wherein $R_4$ is $CH_2CH_2OCH_3$.

Clause 22. The compound according to any one of clauses 1 to 21 wherein $R_5$ is H.

Clause 23. The compound according to any one of clauses 1 to 21 wherein $R_5$ is $C_{1-6}$alkyl.

Clause 24. The compound according to clause 23 wherein $R_5$ is methyl or ethyl.

Clause 25. The compound according to any one of clauses 1 to 18 wherein $R_5$ is $C_{1-3}$alkyleneO$C_{1-3}$alkyl, such as $C_2$alkyleneO$C_{1-3}$alkyl e.g. $CH_2CH_2OCH_3$.

Clause 26. The compound according to any one of clauses 1 to 25 wherein $R_4$ and $R_5$ are both H.

Clause 27. The compound according to any one of clauses 1 to 25 wherein $R_4$ and $R_5$ are both methyl.

Clause 28. The compound according to any one of clauses 1 to 25 wherein $R_4$ and $R_5$ are both ethyl.

Clause 29. The compound according to any one of clauses 1 to 25 wherein $R_4$ is ethyl and $R_5$ is H.

Clause 30. The compound according to clause 29 wherein $R_4$ and $R_5$ are arranged in an S configuration.

Clause 31. The compound according to any one of clauses 1 to 15 wherein $R_4$ and $R_5$ together with the carbon atom to which they are attached form a $C_{3-6}$spirocycloalkyl.

Clause 32. The compound according to clause 31 wherein $R_4$ and $R_5$ together with the carbon atom to which they are attached form a spirocyclopropyl ring or a spirocyclopentyl ring.

Clause 33. The compound according to any one of clauses 1 to 32 wherein Ar1 is phenyl.

Clause 34. The compound according to any one of clauses 1 to 32 wherein Ar1 is 2-pyridyl.

Clause 35. The compound according to any one of clauses 1 to 34 wherein Ar2 is 3-pyridyl.

Clause 36. The compound according to any one of clauses 1 to 34 wherein Ar2 is 2,5-pyrazinyl.

Clause 37. The compound according to any one of clauses 1 to 36 wherein $R_{10}$ is H.

Clause 38. The compound according to any one of clauses 1 to 36 wherein $R_{10}$ is halo such as fluoro or chloro.

Clause 39. The compound according to any one of clauses 1 to 36 wherein $R_{10}$ is $C_{1-2}$alkyl such as $CH_3$.

Clause 40. The compound according to any one of clauses 1 to 36 wherein $R_{10}$ is O$C_{1-2}$alkyl such as $OCH_3$.

Clause 41. The compound according to any one of clauses 1 to 36 wherein $R_{10}$ is O$C_{1-2}$haloalkyl such as $OCF_3$.

Clause 42. The compound according to any one of clauses 1 to 36 wherein $R_{10}$ is CN.

Clause 43. The compound according to any one of clauses 1 to 42 wherein $R_{11}$ is H.

Clause 44. The compound according to any one of clauses 1 to 42 wherein $R_{11}$ is F.

Clause 45. The compound according to any one of clauses 1 to 42 wherein $R_{11}$ is $CH_3$.

Clause 46. The compound according to any one of clauses 1 to 42 wherein $R_{11}$ is $OCH_3$.

Clause 47. The compound according to any one of clauses 1 to 46 wherein $R_{12}$ is H.

Clause 48. The compound according to any one of clauses 1 to 46 wherein $R_{12}$ is halo such as fluoro or chloro.

Clause 49. The compound according to any one of clauses 1 to 46 wherein $R_{12}$ is $C_{1-4}$alkyl such as $CH_3$.

Clause 50. The compound according to any one of clauses 1 to 46 wherein $R_{12}$ is O$C_{1-4}$alkyl such as methoxy,

ethoxy or isopropoxy.

Clause 51. The compound according to any one of clauses 1 to 46 wherein $R_{12}$ is $OC_{0-2}$alkylene$C_{3-5}$cycloalkyl such as $OC_0$alkylene$C_3$cycloalkyl.

Clause 52. The compound according to any one of clauses 1 to 46 wherein $R_{12}$ is CN.

Clause 53. The compound according to any one of clauses 1 to 46 wherein $R_{12}$ is $C_{1-4}$haloalkyl such as $CF_3$.

Clause 54. The compound according to any one of clauses 1 to 46 wherein $R_{12}$ is $OC_{1-4}$haloalkyl such as $OCH_2CF_3$ or $OCHF_2$.

Clause 55. A compound of the examples P1 to P111.

Clause 56. A compound of the formula (II):

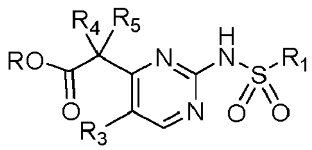

wherein $R_1$, $R_3$, $R_4$ and $R_5$ are as defined in any preceding clause and R is H, $C_{1-6}$alkyl (e.g. methyl and ethyl) or benzyl.

Clause 57. A compound of formula (III):

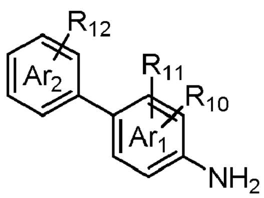

wherein Ar1, Ar2, $R_{10}$, $R_{11}$ and $R_{12}$ are as defined in any preceding clause.

Clause 58. A compound of formula (XX):

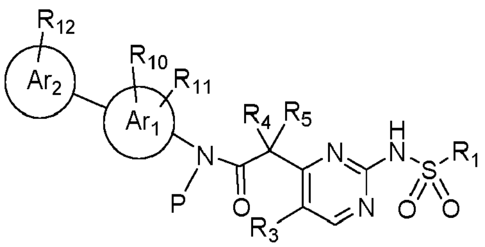

(XX);

wherein Ar1, Ar2, $R_1$, $R_3$, $R_4$, $R_5$, $R_{10}$, $R_{11}$ and $R_{12}$ are as defined in any preceding clause and P is a nitrogen protecting group such as para-methoxybenzyl.

Clause 59. A compound according to any one of clauses 1 to 55, for use as a medicament.

Clause 60. The compound according to clause 59, for use in the inhibition of CTPS1 in a subject.

Clause 61. The compound according to clause 59, for use in the reduction of T-cell and/or B-cell proliferation in a subject.

Clause 62. The compound according to clause 59, for use in the treatment or prophylaxis of: inflammatory skin diseases such as psoriasis or lichen planus; acute and/or chronic GVHD such as steroid resistant acute GVHD; acute lymphoproliferative syndrome (ALPS); systemic lupus erythematosus, lupus nephritis or cutaneous lupus; or transplantation.

Clause 63. A method for the inhibition of CTPS1 in a subject, which comprises administering to the subject an effective amount of a compound according to any one of clauses 1 to 59.

Clause 64. Use of a compound according to any one of clauses 1 to 59, in the manufacture of a medicament for the inhibition of CTPS1 in a subject.

Clause 65. A pharmaceutical composition comprising a compound according to any one of clauses 1 to 59.

Clause 66. The compound, method or use according to any one of clauses 59 to 64, for administration to a human subject.

Clause 67. The compound, method, use or composition according to any one of clauses 59 to 65, for administration in conjunction with a further pharmaceutically acceptable active ingredient or ingredients.

Clause 68. The compound according to any one of clauses 1 to 59, which is in natural isotopic form.

REFERENCES

Cheng, D. et al. Discovery of Pyridinyl Acetamide Derivatives as Potent, Selective, and Orally Bioavailable Porcupine Inhibitors. Medicinal Chemistry Letters, 7(7), 676-680; 2016

Evans, D. R. & Guy, H. I. Mammalian pyrimidine biosynthesis: fresh insights into an ancient pathway. J. Biol. Chem. 279, 33035-33038 (2004).

Fairbanks, L. D., Bofill, M., Ruckemann, K. & Simmonds, H. A. Importance of ribonucleotide availability to proliferating T-lymphocytes from healthy humans. Disproportionate expansion of pyrimidine pools and contrasting effects of de novo synthesis inhibitors. J. Biol. Chem. 270, 29682-29689 (1995).

Higgins, M. J., Graves, P. R. & Graves, L. M. Regulation of human cytidine triphosphate synthetase 1 by glycogen synthase kinase 3. J. Biol. Chem. 282, 29493-29503 (2007).

Kursula, P., Flodin, S., Ehn, M., Hammarström, M., Schuler, H., Nordlund, P. and Stenmarka, P. Structure of the synthetase domain of human CTP synthetase, a target for anticancer therapy. Acta Crystallogr Sect F Struct Biol Cryst Commun. 62 (Pt7): 613-617 (2006). Lieberman I. Enzymatic amination of uridine triphosphate to cytidine triphosphate. The J. Biol. Chem. 222 (2): 765-75 (1956).

Lübbers, T et al. Aminothiazoles as γ-secretase modulators. Bioorganic & Medicinal Chemistry Letters, 21(21), 6554-6558; 2011

Martin E. et al. ; CTP synthase 1 deficiency in humans reveals its central role in lymphocytes proliferation. Nature. Jun 12; 510(7504):288-92 (2014). Erratum in: Nature. Jul 17; 511(7509):370 (2014).

Ostrander, D. B., O'Brien, D. J., Gorman, J. A. & Carman, G. M. Effect of CTP synthetase regulation by CTP on phospholipid synthesis in Saccharomyces cerevisiae. J. Biol. Chem. 273, 18992-19001 (1998).

Sakamoto K, Ishibashi Y, Adachi R, et al. Identification of cytidine-5-triphosphate synthase1-selective inhibitory peptide from random peptide library displayed on T7 phage. Peptides. 2017; 94:56-63 (2017).

van den Berg, A. A. et al. Cytidine triphosphate (CTP) synthetase activity during cell cycle progression in normal and malignant T-lymphocytic cells. Eur. J. Cancer 31, 108-112 (1995).

van Kuilenburg, A.B.P, Meinsma, R., Vreken, P., Waterham, H.R., van Gennip, A.H. Identification of a cDNA encoding an isoform of human CTP synthetase. Biochimica et Biophysica Acta 1492548-552 (2000).

Xing-Li F. et al. Efficient Diphosphane-Based Catalyst for the Palladium-Catalyzed Suzuki Cross-Coupling Reaction of 3-Pyridylboronic Acids. European Journal of Organic Chemistry, (13), 2051-2054; 2009

## Claims

**1.** A compound of formula (I):

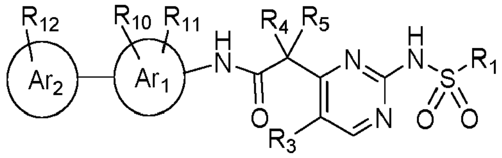

(I)

wherein

$R_1$ is $C_{1-5}$alkyl or $C_{0-2}$alkylene$C_{3-5}$cycloalkyl which cycloalkyl is optionally substituted by $CH_3$;
$R_3$ is H, halo or $CH_3$;
$R_4$ and $R_5$ are each independently H, $C_{1-6}$alkyl, $C_{1-3}$alkylene$OC_{1-3}$alkyl, or $R_4$ and $R_5$ together with the carbon atom to which they are attached form a $C_{3-6}$spirocycloalkyl;
Ar1 is a 6-membered aryl or heteroaryl;
Ar2 is a 6-membered aryl or heteroaryl and is attached to Ar1 in the para position relative to the amide;
$R_{10}$ is H, halo, $C_{1-2}$alkyl, $OC_{1-2}$alkyl, $OC_{1-2}$haloalkyl or CN;
$R_{11}$ is H, F, $CH_3$ or $OCH_3$; and
$R_{12}$ is attached to Ar2 in the ortho or meta position relative to Ar1 and $R_{12}$ is H, halo, $C_{1-4}$alkyl, $OC_{1-4}$alkyl, $OC_{0-2}$alkylene$C_{3-5}$cycloalkyl, CN, $C_{1-4}$haloalkyl, $OC_{1-4}$haloalkyl;

or a salt and/or solvate thereof and/or derivative thereof.

**2.** The compound according to claim 1 wherein $R_1$ is $C_{3-5}$cycloalkyl optionally substituted by $CH_3$.

**3.** The compound according to claim 1 or claim 2 wherein $R_4$ is $C_{1-6}$alkyl, particularly methyl or ethyl.

**4.** The compound according to any one of claims 1 to 3 wherein $R_5$ is H, methyl or ethyl.

**5.** The compound according to any one of claims 1 to 3 wherein $R_4$ and $R_5$ together with the carbon atom to which they are attached form a spirocyclopropyl ring or a spirocyclopentyl ring.

**6.** The compound according to any one of claims 1 to 5 wherein Ar1 is phenyl or 2-pyridyl.

**7.** The compound according to any one of claims 1 to 6 wherein Ar2 is 3-pyridyl or 2,5-pyrazinyl.

**8.** The compound according to any one of claims 1 to 7 wherein $R_{10}$ is H, F, Cl, $CH_3$, O $CH_3$, $OCF_3$ or CN e.g. H or F.

**9.** The compound according to any one of claims 1 to 8 wherein $R_{11}$ is H or F e.g. H.

**10.** The compound according to any one of claims 1 to 9 wherein $R_{12}$ is H, F, Cl, $CH_3$, methoxy, ethoxy, isopropoxy, $OC_0$alkylene$C_3$cycloalkyl, CN, $CF_3$, $OCHF_2$ or $OCH_2CF_3$ e.g. methoxy, ethoxy, isopropoxy, $OC_0$alkylene$C_3$cycloalkyl, $CF_3$, $OCHF_2$ or $OCH_2CF_3$.

**11.** The compound according to any one of claims 1 to 10 for use as a medicament.

**12.** The compound according to claim 11, for use in the reduction of T-cell and/or B-cell proliferation in a subject.

**13.** The compound according to claim 11, for use in the treatment or prophylaxis of: inflammatory skin diseases such as psoriasis or lichen planus; acute and/or chronic GVHD such as steroid resistant acute GVHD; acute lymphoproliferative syndrome (ALPS); systemic lupus erythematosus, lupus nephritis or cutaneous lupus; or transplantation.

**14.** A pharmaceutical composition comprising a compound according to any one of claims 1 to 10.

**15.** A compound of the formula (II):

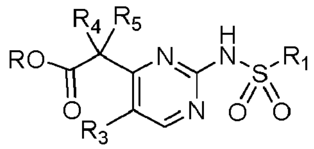

wherein $R_1$, $R_3$, $R_4$ and $R_5$ are as defined in any preceding claim and R is H, $C_{1-6}$alkyl (e.g. methyl and ethyl) or benzyl.

Europäisches Patentamt
European Patent Office
Office européen des brevets

# EUROPEAN SEARCH REPORT

Application Number
EP 18 16 3772

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SAKAMOTO KOTARO ET AL: "Identification of cytidine-5-triphosphate synthase1-selective inhibitory peptide from random peptide library displayed on T7 phage", PEPTIDES, ELSEVIER, AMSTERDAM, NL, vol. 94, 1 July 2017 (2017-07-01), pages 56-63, XP085130857, ISSN: 0196-9781, DOI: 10.1016/J.PEPTIDES.2017.06.007 * abstract * ----- | 1-15 | INV. C07D403/12 C07D401/14 C07D401/12 A61P35/00 A61K31/506 |
| A | WO 2014/170435 A2 (INSERM INST NAT DE LA SANTÉ ET DE LA RECH MÉDICALE [FR]; FOND IMAGINE) 23 October 2014 (2014-10-23) * pages 1-2; claims 13-22 * ----- | 1-15 | |
| A | WO 2009/075874 A1 (AMGEN INC [US]; HITCHCOCK STEPHEN [US]; CHEN JIAN J [US]; NCUBE MQHELE) 18 June 2009 (2009-06-18) * pages 1-3; examples * ----- | 1-15 | |

TECHNICAL FIELDS SEARCHED (IPC)

C07D
A61P

The present search report has been drawn up for all claims

1

| Place of search | Date of completion of the search | Examiner |
|---|---|---|
| Munich | 3 May 2018 | Lauro, Paola |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 18 16 3772

This annex lists the patent family members relating to the patent documents cited in the above-mentioned European search report. The members are as contained in the European Patent Office EDP file on
The European Patent Office is in no way liable for these particulars which are merely given for the purpose of information.

03-05-2018

| Patent document cited in search report | Publication date | Patent family member(s) | Publication date |
|---|---|---|---|
| WO 2014170435 A2 | 23-10-2014 | CA 2909434 A1 | 23-10-2014 |
| | | CN 105473136 A | 06-04-2016 |
| | | EP 2986287 A2 | 24-02-2016 |
| | | JP 2016523818 A | 12-08-2016 |
| | | US 2016051674 A1 | 25-02-2016 |
| | | WO 2014170435 A2 | 23-10-2014 |
| WO 2009075874 A1 | 18-06-2009 | AU 2008335709 A1 | 18-06-2009 |
| | | CA 2707492 A1 | 18-06-2009 |
| | | CN 101896461 A | 24-11-2010 |
| | | EP 2231602 A1 | 29-09-2010 |
| | | JP 2011506445 A | 03-03-2011 |
| | | US 2011166132 A1 | 07-07-2011 |
| | | WO 2009075874 A1 | 18-06-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CHENG, D. et al.** Discovery of Pyridinyl Acetamide Derivatives as Potent, Selective, and Orally Bioavailable Porcupine Inhibitors. *Medicinal Chemistry Letters,* 2016, vol. 7 (7), 676-680
- **EVANS, D. R. ; GUY, H. I.** Mammalian pyrimidine biosynthesis: fresh insights into an ancient pathway. *J. Biol. Chem.,* 2004, vol. 279, 33035-33038
- **FAIRBANKS, L. D. ; BOFILL, M. ; RUCKEMANN, K. ; SIMMONDS, H. A.** Importance of ribonucleotide availability to proliferating T-lymphocytes from healthy humans. Disproportionate expansion of pyrimidine pools and contrasting effects of de novo synthesis inhibitors. *J. Biol. Chem.,* 1995, vol. 270, 29682-29689
- **HIGGINS, M. J. ; GRAVES, P. R. ; GRAVES, L. M.** Regulation of human cytidine triphosphate synthetase 1 by glycogen synthase kinase 3. *J. Biol. Chem.,* 2007, vol. 282, 29493-29503
- **KURSULA, P. ; FLODIN, S. ; EHN, M. ; HAMMARSTRÖM, M. ; SCHULER, H. ; NORDLUND, P. ; STENMARKA, P.** Structure of the synthetase domain of human CTP synthetase, a target for anticancer therapy. *Acta Crystallogr Sect F Struct Biol Cryst Commun,* 2006, vol. 62, 613-617
- **LIEBERMAN I.** Enzymatic amination of uridine triphosphate to cytidine triphosphate. *The J. Biol. Chem.,* 1956, vol. 222 (2), 765-75
- **LÜBBERS, T et al.** Aminothiazoles as $\gamma$-secretase modulators. *Bioorganic & Medicinal Chemistry Letters,* 2011, vol. 21 (21), 6554-6558
- **MARTIN E. et al.** CTP synthase 1 deficiency in humans reveals its central role in lymphocytes proliferation. *Nature,* 12 June 2014, vol. 510 (7504), 288-92
- **ERRATUM.** *Nature,* 2014, vol. 511 (7509), 370
- **OSTRANDER, D. B. ; O'BRIEN, D. J. ; GORMAN, J. A. ; CARMAN, G. M.** Effect of CTP synthetase regulation by CTP on phospholipid synthesis in Saccharomyces cerevisiae. *J. Biol. Chem.,* 1998, vol. 273, 18992-19001
- **SAKAMOTO K ; ISHIBASHI Y ; ADACHI R et al.** Identification of cytidine-5-triphosphate synthase1-selective inhibitory peptide from random peptide library displayed on T7 phage. *Peptides,* 2017, vol. 94, 56-63
- **VAN DEN BERG, A. A. et al.** Cytidine triphosphate (CTP) synthetase activity during cell cycle progression in normal and malignant T-lymphocytic cells. *Eur. J. Cancer,* 1995, vol. 31, 108-112
- **VAN KUILENBURG, A.B.P ; MEINSMA, R. ; VREKEN, P. ; WATERHAM, H.R. ; VAN GENNIP, A.H.** Identification of a cDNA encoding an isoform of human CTP synthetase. *Biochimica et Biophysica Acta,* 2000, 1492548-552
- **XING-LI F. et al.** Efficient Diphosphane-Based Catalyst for the Palladium-Catalyzed Suzuki Cross-Coupling Reaction of 3-Pyridylboronic Acids. *European Journal of Organic Chemistry,* 2009, vol. 13, 2051-2054